# EUROPEAN PATENT APPLICATION

(11) **EP 4 509 155 A1**
(43) Date of publication of application: **19.02.2025**
(21) Application number: 23818967.4
(22) Date of filing: 29.05.2023
(51) Int. Cl.: A61M 11/00, A61M 15/00

(54) **MOUNTING AND DISMOUNTING METHOD FOR NEBULIZATION ASSEMBLY, AND NEBULIZATION THERAPEUTIC DEVICE**

(30) Priority: 06.06.2022 CN 202221420322 U; 08.09.2022 CN 202222409381 U; 09.09.2022 CN 202222413845 U; 01.11.2022 CN 202222931573 U; 29.12.2022 CN 202223598482 U; 29.12.2022 CN 202211714344; 30.12.2022 CN 202211742940
(71) Applicant: BMC Medical Co., Ltd., Beijing 100142 (CN)
(72) Inventor: YANG, Junda, Tianjin 301700 (CN); HE, Wei, Beijing 100041 (CN); NIU, Shiteng, Tianjin 301700 (CN); ZHUANG, Zhi, Beijing 100041 (CN)
(74) Representative: Grünecker Patent- und Rechtsanwälte PartG mbB
(86) International application number: PCT/CN2023/096853
(87) International publication number: WO 2023/236802

(57) **Abstract**

A mounting and dismounting method for a nebulization assembly, and a nebulization therapeutic device, which belong to the technical field of nebulization. When performing dismounting of a nebulizer assembly, a whole formed by a nebulization assembly (30) and a liquid storage apparatus (20) may be removed from a main unit (10) when the nebulization assembly (30) and the liquid storage apparatus (20) are in a first assembly state; because the nebulization assembly (30) and the liquid storage apparatus (20) are fixed to one another when in the first assembly state, the problem of liquid medicine leakage is prevented, and the risk of a short circuit between detection pins of the main unit (10) is reduced. After the whole formed by the nebulization assembly (30) and the liquid storage apparatus (20) is removed from the main unit (10), the nebulization assembly (30) and the liquid storage apparatus (20) are switched to a second assembly state; at this point the nebulization assembly (30) and the liquid storage apparatus (20) are in a separable state, and the nebulization assembly (30) may be smoothly taken off of the liquid storage apparatus (20); even if liquid medicine leakage occurs during this process, the main unit (10) is unaffected, thereby improving use safety.

## Description

### CROSS REFERENCE TO RELATED APPLICATIONS

The present disclosure claims the priority to seven Chinese patent applications, namely, Chinese Patent Application No. 202221420322.8 filed on Jun. 6, 2022 and titled as "Medicine Cup Assembly and Mesh-Type Nebulizer", Chinese Patent Application No. 202222409381.1 filed on Sep. 8, 2022 and titled as "Device Having Nebulization Function", Chinese Patent Application No. 202222413845.6 filed on Sep. 9, 2022 and titled as "Assembly Having Nebulization Function and Nebulization Device", Chinese Patent Application No. 202222931573.9 filed on Nov. 1, 2022 and titled as "Power Management System and Nebulization Therapeutic Device", Chinese Patent Application No. 202223598482.4 filed on Dec. 29, 2022 and titled as "Nebulizer", Chinese Patent Application No. 202211714344.X filed on Dec. 29, 2022 and titled as "Power Supply Mutual Exclusion Structure, Power Supply Mutual Exclusion Method and Electronic Product", and Chinese Patent Application No. 202211742940.9 filed on Dec. 30, 2022 and titled as "Dismounting and Mounting Method for Nebulization Assembly and Nebulization Therapeutic Device", the entire contents of which are incorporated herein by reference.

### FIELD

The present disclosure belongs to the technical field of nebulization, in particular to a dismounting and mounting method for a nebulization assembly and a nebulization therapeutic device.

### BACKGROUND

Nebulization therapeutic devices (also referred to as nebulizers) are widely applied in clinical treatment and daily life. A nebulization therapeutic device can be used to nebulize a liquid medicine into tiny particles, and then the medicine can be introduced into the respiratory tract and lungs through respiratory inhalation, so as to achieve a purpose of painless, rapid and effective treatment.

At present, a nebulization therapeutic device generally comprises a main unit, a liquid storage apparatus and a nebulization assembly. The liquid in the liquid storage apparatus is introduced into the nebulization assembly through a liquid outlet and then is driven to pass through a nebulization plate to form nebulized particles.

When the nebulization assembly is to be dismounted, the nebulization assembly is usually removed from the liquid storage apparatus because the liquid storage apparatus is fixed to the main unit. In that process, the liquid medicine tends to leak through the liquid outlet of the liquid storage apparatus, and the leaking liquid may cause a short circuit between probes on the main unit, consequently leading to a safety accident.

### SUMMARY

An object of the embodiments of the present disclosure is to provide a dismounting and mounting method for a nebulization assembly and a nebulization therapeutic device, which can solve the problem that the liquid leaking through a liquid outlet of the liquid storage apparatus may easily cause a short circuit between the probes on the main unit and cause a safety accident consequently when the nebulization assembly is dismounted in the prior art.

In order to solve the above technical problem, the present disclosure is implemented as follows:
In a first aspect, an embodiment of the present disclosure provides a method for dismounting a nebulization assembly, which comprises:
removing an entire assembly formed by a nebulization assembly and a liquid storage apparatus from a main unit when the nebulization assembly and the liquid storage apparatus are in a first assembly state, wherein the first assembly state is a state in which the nebulization assembly and the liquid storage apparatus are fixed in relation to each other;
controlling the nebulization assembly and the liquid storage apparatus to switch to a second assembly state, wherein the second assembly state is a state in which the nebulization assembly and the liquid storage apparatus are separatable; and
separating the nebulization assembly from the liquid storage apparatus.

Optionally, removing the entire assembly formed by the nebulization assembly and the liquid storage apparatus from the main unit when the nebulization assembly and the liquid storage apparatus are in the first assembly state comprises:
controlling the liquid storage apparatus and the main unit to switch from a locked state to an unlocked state; and
sliding the liquid storage apparatus along a slide channel on the main unit, and separating a first limiting structure on the nebulization assembly from a second limiting structure on the main unit at the same time, so as to remove the entire assembly formed by the nebulization assembly and the liquid storage apparatus from the main unit;
wherein the first limiting structure is snap-fitted with the second limiting structure when the liquid storage apparatus and the main unit are in the locked state.

Optionally, controlling the liquid storage apparatus and the main unit to switch from the locked state to the unlocked state comprises:
switching at least one snap-fitting clip on the main unit and at least one snap-fitting groove in the liquid storage apparatus from a snap-fitted state to a disengaged state; and/or switching at least one snap-fitting groove in the main unit and at least one snap-fitting clip on the liquid storage apparatus from a snap-fitted state to a disengaged state;
wherein the liquid storage apparatus and the main unit are in the locked state when the snap-fitting clips and the snap-fitting grooves are in the snap-fitted state; and the liquid storage apparatus and the main unit are in the unlocked state when the snap-fitting clips and the snap-fitting grooves are in the disengaged state.

Optionally, controlling the liquid storage apparatus and the main unit to switch from the locked state to the unlocked state comprises:
switching a key on the main unit from a first state to a second state;
wherein the liquid storage apparatus and the main unit are in the locked state when the key is in the first state; and the liquid storage apparatus and the main unit are in the unlocked state when the key is in the second state.

Optionally, controlling the nebulization assembly and the liquid storage apparatus to switch to the second assembly state comprises:
rotating a locking element on the liquid storage apparatus to switch the locking element and the liquid storage apparatus from a connected state to a separated state;
wherein the locking element is detachably connected to the liquid storage apparatus by rotation, and the nebulization assembly is positioned between the locking element and the liquid storage apparatus;
the nebulization assembly and the liquid storage apparatus are in the first assembly state when the locking element and the liquid storage apparatus are in the connected state; and the nebulization assembly and the liquid storage apparatus are in the second assembly state when the locking element and the liquid storage apparatus are in the separated state.

Optionally, controlling the nebulization assembly and the liquid storage apparatus to switch to the second assembly state comprises:
controlling the nebulization assembly and a mounting base to rotate from a first relative position to a second relative position;
wherein the liquid storage apparatus is provided with the mounting base, and the nebulization assembly is at least partially embedded in the mounting base and rotatably connected to the mounting base;
wherein the nebulization assembly and the mounting base are fixed in relation to each other when the nebulization assembly and the mounting base are in the first relative position; and the nebulization assembly and the mounting base can be separated from each other when the nebulization assembly and the mounting base are in the second relative position.

Optionally, controlling the nebulization assembly and the mounting base to rotate from the first relative position to the second relative position comprises:
fitting a first snap-fitting part on the nebulization assembly with a second snap-fitting part on the mounting base by snap-fitting, so that the nebulization assembly and the mounting base are in the first relative position; and
causing a snap-fitting function of the first snap-fitting part and the second snap-fitting part to fail when the nebulization assembly and the mounting base are controlled to rotate to the second relative position.

Optionally, controlling the nebulization assembly and the mounting base to rotate from the first relative position to the second relative position comprises:
controlling the nebulization assembly and the mounting base to rotate in relation to each other, so as to drive the first limiting structure to slide from a locking end of a third limiting structure to an unlocking end of the third limiting structure;
wherein the first limiting structure is arranged on the nebulization assembly, the third limiting structure is arranged at the bottom of the mounting base, and the third limiting structure is used for limiting a rotation range of the nebulization assembly and the mounting base;
the nebulization assembly and the mounting base are in the first relative position when the first limiting structure is at the locking end; and the nebulization assembly and the mounting base are in the second relative position when the first limiting structure is at the unlocking end.

In a second aspect, an embodiment of the present disclosure provides a method for mounting a nebulization assembly, which comprises: plugging a nebulization assembly into a mounting base of a liquid storage apparatus when the liquid storage apparatus is separated from a main unit, so that the nebulization assembly and the liquid storage apparatus are in a second assembly state, wherein the second assembly state is a state in which the nebulization assembly and the liquid storage apparatus are separatable;
controlling the nebulization assembly and the liquid storage apparatus to switch to a first assembly state, wherein the first assembly state is a state in which the nebulization assembly and the liquid storage apparatus are fixed in relation to each other; and
plugging the entire assembly formed by the nebulization assembly and the liquid storage apparatus into the main unit.

In a third aspect, an embodiment of the present disclosure provides a nebulization therapeutic device, which comprises: a main unit, a liquid storage apparatus and a nebulization assembly, wherein the liquid storage apparatus is detachably mounted to the main unit in a plug-in manner;
the nebulization assembly is connected to the liquid storage apparatus;
the liquid storage apparatus and the nebulization assembly are fixed in relation to each other when an entire assembly formed by the liquid storage apparatus and the nebulization assembly is assembled to the main unit; and
the liquid storage apparatus and the nebulization assembly are dismountable when the entire assembly formed by the liquid storage apparatus and the nebulization assembly is separated from the main unit.

In a fourth aspect, an embodiment of the present disclosure provides a nebulization therapeutic device, which comprises a nebulization assembly, wherein the nebulization assembly can be dismounted with the above-mentioned dismounting method.

In the embodiments of the present disclosure, when performing dismounting of the nebulization assembly, an entire assembly formed by the nebulization assembly and a liquid storage apparatus may be removed from a main unit when the nebulization assembly and the liquid storage apparatus are in a first assembly state; because the nebulization assembly and the liquid storage apparatus are fixed in relation to each another in the first assembly state, a problem of liquid medicine leakage is prevented, and a risk of a short circuit between probes on the main unit is reduced. After the entire assembly formed by the nebulization assembly and the liquid storage apparatus is removed from the main unit, the nebulization assembly and the liquid storage apparatus are switched to a second assembly state; at that point, the nebulization assembly and the liquid storage apparatus are in a separable state, and the nebulization assembly may be smoothly taken off of the liquid storage apparatus; even if liquid medicine leakage occurs in this process, the main unit is unaffected, thereby the safety of use is greatly improved.

Another object of the embodiments of the present disclosure is to provide a medicine cup assembly and a mesh-type nebulizer, which can solve a problem that a defective connection between the nebulization device and the medicine cup device in the prior art affects the sealing performance of the medicine cup assembly.

In order to solve the above technical problem, the present disclosure is implemented as follows:
In a first aspect, an embodiment of the present disclosure provides a medicine cup assembly, which comprises a nebulization device, a medicine cup device and a locking element, wherein the nebulization device is detachably connected to the medicine cup device, and the nebulization device is at least partially positioned inside the medicine cup device; the locking element is detachably connected to the medicine cup device by rotation, and the nebulization device is positioned between the locking element and the medicine cup device; the locking element is at least partially positioned inside the medicine cup device when the locking element is connected to the medicine cup device and the locking element is used for locking the nebulization device to the medicine cup device.

In the embodiment of the present disclosure, the medicine cup device is configured for storing a liquid medicine, the nebulization device is configured for nebulizing the liquid medicine in the medicine cup into nebulized particles, and the nebulization device is further used for connecting an external inhalation device, so that a user can inhale the nebulized particles through the inhalation device. The nebulization device is at least partially positioned inside the medicine cup device, and the nebulization device is detachably connected to the medicine cup device; when the locking element is connected to the medicine cup device, the nebulization device is positioned between the locking element and the medicine cup device; when the nebulization device doesn't take effect anymore, the user may separate the locking element from the medicine cup device, so that the nebulization device can be smoothly pulled out of the medicine cup device, and then a new nebulization device can be smoothly plugged into the medicine cup device. The locking element is configured to lock the nebulization device to the medicine cup device, and the locking element is at least partially positioned in the medicine cup device; when the nebulization device is connected to the medicine cup device, the locking element is detachably connected to the medicine cup device by rotation; under the locking action of the locking element, the nebulization device will not rotate or get loose unless it is dismounted intentionally, thereby the sealing performance of the connection between the nebulization device and the medicine cup device is enhanced, and a beneficial effect of preventing the liquid medicine in the medicine cup assembly from leaking due to poor sealing performance is achieved.

Optionally, the locking element and the nebulization assembly are configured integrally, and the nebulization device abuts against the medicine cup device when the locking element is connected to the medicine cup device.

Optionally, the locking element and the nebulization device are separate structures, and the locking element is fitted around the periphery of the nebulization device.

Optionally, the locking element is of an annular structure, and is provided with a raised rib on a side connected to the medicine cup device, and the medicine cup device is provided with a slide channel at a position where the medicine cup device is connected to the locking element; and the raised rib matches the slide channel and is fitted with the slide channel, so that the medicine cup device is rotatably connected to the locking element.

Optionally, the slide channel comprises a limiting groove, which matches the raised rib and is used for limiting the position of the raised rib.

Optionally, either of the raised rib and the limiting groove is provided with a first recess, and the other of the raised rib and the limiting groove is provided with a first bump, and the first recess is snap-fitted with the first bump.

Optionally, the medicine cup device comprises a medicine cup body, which is provided with an accommodating cavity on a side for at least partially accommodating the nebulization device.

Optionally, a boss is arranged in the accommodating cavity, and a sidewall of the boss and an inner wall of the accommodating cavity enclose to form a second recess.

Optionally, the medicine cup device further comprises a medicine cup cap fastener and a medicine cup cap, wherein the medicine cup cap fastener is movably connected to the medicine cup cap and the medicine cup body respectively.

Optionally, the medicine cup device further comprises a first sealing element, which is arranged between the medicine cup cap and the medicine cup body.

Optionally, the nebulization device comprises a first housing, a second sealing element, a nebulization plate, and a second housing fixedly connected to the first housing, which are arranged sequentially, wherein the first housing is detachably connected to the medicine cup device, and the second sealing element and the nebulization plate are sandwiched between the first housing and the second housing.

Optionally, the first housing is provided with a protrusion, which is used to fit with the second recess to realize a detachable connection between the medicine cup device and the nebulization device in a plug-in manner; the first housing is further provided with metal contacts, which are used for receiving electric energy and electrical signals.

In a second aspect, an embodiment of the present disclosure further provides a mesh-type nebulizer, which comprises the medicine cup assembly as described above, and further comprises a main unit assembly, wherein the medicine cup assembly is provided with a first sliding connection structure, and the main unit assembly is provided with a second sliding connection structure; the first sliding connection structure and the second sliding connection structure are fitted with each other, and are used for detachably connecting the medicine cup assembly to the main unit assembly.

According to the present disclosure, the medicine cup assembly as described above is connected to the main unit assembly; when the medicine cup assembly is connected to the main unit assembly, the nebulization device and the medicine cup device that are detachably connected in a plug-in manner and the locking element that locks the nebulization device to the medicine cup device prevent the nebulization device from rotating with an inhalation device when the user mounts the inhalation device by rotation; besides, the metal contacts arranged on the nebulization device are always in contact with metal probes on the main unit assembly, so that beneficial effects of ensuring the electrical connection between the medicine cup assembly and the main unit assembly and reducing the risk of damage of the metal probes are achieved.

Optionally, the main unit assembly is provided with metal probes, which abut against the metal contacts to realize an electrical connection between the main unit assembly and the medicine cup assembly, and the main unit assembly is used to supply power and transmit electrical signals to the medicine cup assembly.

Another object of the embodiments of the present disclosure is to provide an assembly having a nebulization function and a nebulization device, which can solve a problem that the liquid stored in the nebulization device becomes an accessible charged body and brings an electric shock risk in some special circumstances because the liquid is electrically communicated with a liquid level detection device in the prior art.

In order to solve the above technical problem, the present disclosure is implemented as follows:
In a first aspect, an embodiment of the present disclosure provides an assembly having a nebulization function, which comprises: a housing, a nebulization module and a detection electrode, wherein
the housing is provided with a liquid storage cavity and a liquid outlet in communication with the liquid storage cavity;
the nebulization module is detachably connected to the housing, and the nebulization module is in communication with the liquid outlet;
the detection electrode is arranged on an outer wall of the housing at the bottom of the housing, and the outer wall of the housing separates the detection electrode from the liquid storage cavity; and the detection electrode is used for detecting whether the liquid in the liquid storage cavity is in shortage.

Optionally, the assembly having a nebulization function further comprises: a detection circuit and a control module, wherein
the detection circuit is electrically connected to the detection electrode and the control module respectively, and is used for acquiring a potential signal of the detection electrode and transmitting the potential signal to the control module; and
the control module is used for judging whether the liquid storage cavity is short of liquid according to the potential signal.

Optionally, the outer wall of the housing is provided with an accommodating slot at the bottom of the housing, and the detection electrode is at least partially embedded in the accommodating slot.

Optionally, the accommodating slot is provided with a first snap-fitting part therein, the detection electrode is provided with a second snap-fitting part, and the first snap-fitting part is snap-fitted with the second snap-fitting part.

Optionally, the thickness of the bottom of the accommodating slot is 0.1 mm - 3mm.

Optionally, the thickness of the wall of the accommodating slot near the nebulization module is 0.1 mm - 3 mm.

Optionally, the nebulization module comprises a nebulization plate, which is arranged opposite to the liquid outlet; and
the distance between the nebulization plate and the detection electrode is 0.1 mm - 20 mm in the thickness direction of the nebulization plate.

Optionally, the distance between an upper edge of the detection electrode and a lower edge of the nebulization plate is 0 mm - 20 mm in the height direction of the nebulization plate.

Optionally, the dimension of the detection electrode is 1 mm - 10 mm in the thickness direction of the nebulization plate; and
the dimension of the detection electrode is 1 mm - 10 mm in the height direction of the nebulization plate.

In a second aspect, an embodiment of the present disclosure provides a nebulization device, which comprises the above-mentioned assembly having a nebulization function.

In the embodiment of the present disclosure, the assembly having a nebulization function comprises: a housing, a nebulization module and a detection electrode; the housing is provided with a liquid storage cavity and a liquid outlet in communication with the liquid storage cavity; the nebulization module is detachably connected to the housing and is in communication with the liquid outlet; the detection electrode is arranged on an outer wall of the housing at the bottom of the housing, and the outer wall of the housing separates the detection electrode from the liquid storage cavity; the detection electrode is used for detecting whether the liquid in the liquid storage cavity is in shortage. By arranging the detection electrode on the outer wall of the housing, a liquid shortage detection function can also be realized under a condition that the detection electrode is not in direct contact with the liquid in the liquid storage cavity, so that the liquid is prevented from becoming an accessible charged body, a risk of electric shock is reduced, and the safety of use is improved; moreover, since the detection electrode is not in direct contact with the liquid in the liquid storage cavity, pollution to the liquid can be avoided.

Another object of the embodiments of the present disclosure is to provide a nebulizer, which solves a problem that the medicine cup assembly falls off and is damaged due to accidental key touching or pressing in an existing nebulizer.

In order to solve the above technical problem, the present disclosure is implemented as follows:
An embodiment of the present disclosure provides a nebulizer, which comprises a main unit and a medicine cup assembly detachably mounted on the main unit. The nebulizer further comprises a guiding and limiting structure and a locking structure, wherein the guiding and limiting structure is used for guiding and limiting the medicine cup assembly when the medicine cup assembly is mounted, and the locking structure is used for releasably locking the medicine cup assembly to the main unit.

Optionally, the main unit comprises a mounting part on an upper part of the main unit, and the medicine cup assembly comprises a mounting cavity for mounting the mounting part.

Optionally, the guiding and limiting structure comprises a retaining rib and a slide channel that are fitted with each other, wherein the retaining rib is arranged on one of the mounting part and the mounting cavity, while the slide channel is arranged on the other of the mounting part and the mounting cavity, and the retaining rib and the slide channel extend in a mounting direction of the medicine cup assembly.

Optionally, the guiding and limiting structure comprises two retaining ribs and two slide channels, wherein the two retaining ribs are respectively arranged on two opposite sides of the mounting part or the mounting cavity, and the two slide channels are respectively arranged on two opposite sides of the mounting cavity or the mounting part.

Optionally, the locking structure is arranged to lock the medicine cup assembly to the main unit when the retaining rib slides to a stroke end of the slide channel.

Optionally, the locking structure comprises a snap-fitting clip and a snap-fitting groove, wherein the snap-fitting clip is arranged on one of the mounting part and the mounting cavity, while the snap-fitting groove is arranged in the other of the mounting part and the mounting cavity; the snap-fitting clip comprises a snap-fitting portion and a pressing portion, wherein the snap-fitting portion is arranged to be snap-fitted in the snap-fitting groove when the medicine cup assembly is mounted in place, and the pressing portion is exposed from the snap-fitting portion, so that the snap-fitting portion can be disengaged from the snap-fitting groove by means of the pressing portion.

Optionally, the mounting cavity has a bottom opening and a side opening for the mounting part to be horizontally mounted therein.

Optionally, the snap-fitting clip is an elastic tab extending obliquely upward from the top of the mounting part in the mounting direction of the medicine cup assembly, the snap-fitting groove is positioned at the top of the mounting cavity and is arranged close to the side opening, an upper edge of the side opening forms an end wall of the snap-fitting groove, and the upper edge can abut against a top surface of the elastic tab to press the elastic tab downward when the medicine cup assembly is mounted.

Optionally, the mounting part comprises a U-shaped cantilever arm extending inward from its outer sidewall and a recess for accommodating the U-shaped cantilever arm, a closed end of the U-shaped cantilever arm is positioned at the inner side of the mounting part, two sidewalls of the U-shaped cantilever arm are arranged one on top of the other, and an upper sidewall forms the snap-fitting clip.

Optionally, the upper sidewall comprises a horizontal portion extending in the horizontal direction and a protruding portion protruding from the middle of a top surface of the horizontal portion, a top surface of the protruding portion is an inclined surface extending obliquely upward in the mounting direction of the medicine cup assembly, the protruding portion is formed as the snap-fitting portion, and an outer end of the horizontal portion is formed as the pressing portion.

In the nebulizer provided by the present disclosure, with the above technical scheme, the guiding and limiting structure can guide and limit the medicine cup assembly in the process of mounting the medicine cup assembly on the main unit, so that the medicine cup assembly can be smoothly mounted and the accuracy of assembly of the medicine cup assembly to the main unit can be ensured; besides, if the locking structure is accidentally released, the guiding and limiting structure can limit the medicine cup assembly on the main unit, and the medicine cup assembly will not fall off and be damaged. Thus, the nebulizer in the present disclosure can not only meet the requirement of the user for replacing and removing the medicine cup assembly by himself/herself, but also improve usability and safety.

Another object of the embodiments of the present disclosure is to provide a device having a nebulization function, which solves a problem that the liquid storage apparatus and the nebulization assembly are prone to rotate in relation to each other and result in liquid leakage from the joint between the liquid storage apparatus and the nebulization assembly in the prior art.

In order to solve the above technical problem, the present disclosure is implemented as follows:
An embodiment of the present disclosure provides a device having a nebulization function, which comprises: a main unit, a liquid storage apparatus and a nebulization assembly, wherein
the liquid storage apparatus is detachably mounted to the main unit in a plug-in manner;
the liquid storage apparatus is provided with a liquid outlet, and the nebulization assembly is in communication with the liquid outlet;
the nebulization assembly is provided with a first limiting structure, and the main unit is provided with a second limiting structure;
when the liquid storage apparatus is assembled to the main unit, the first limiting structure and the second limiting structure are snap-fitted with each other, and the nebulization assembly is fixed in relation with the liquid storage apparatus; and
when the liquid storage apparatus is separated from the main unit, the nebulization assembly and the liquid storage apparatus can be dismounted.

Optionally, the first limiting structure is a limiting rod, and the second limiting structure is a limiting hole;
When the liquid storage apparatus is assembled to the main unit, the limiting rod is at least partially embedded in the limiting hole.

Optionally, the first limiting structure is a limiting hole, and the second limiting structure is a limiting rod;
when the liquid storage apparatus is assembled to the main unit, the limiting rod is at least partially embedded in the limiting hole.

Optionally, conductive probes are arranged in the limiting hole, and are electrically connected to the limiting rod, so that a circuit between the main unit and the nebulization assembly is closed.

Optionally, at least two limiting rods, at least two limiting holes and at least two conductive probes are provided.

Optionally, the liquid storage apparatus is provided with a mounting base, and the nebulization assembly is at least partially embedded in the mounting base and is rotatably connected to the mounting base.

Optionally, the nebulization assembly is provided with a first snap-fitting part, and the mounting base is provided with a second snap-fitting part;
the nebulization assembly and the mounting base can be separated from each other when the nebulization assembly and the mounting base are in a first relative position; and the first snap-fitting part and the second snap-fitting part are snap-fitted with each other when the nebulization assembly and the mounting base are in a second relative position.

Optionally, both the nebulization assembly and the mounting base are provided with a threaded structure, and the nebulization assembly is connected to the mounting base through threads.

Optionally, the bottom of the mounting base is provided with a clearance hole; and
the limiting rod is inserted through the clearance hole and slides in relation to the mounting base in an extension direction of the clearance hole.

Optionally, a slide channel assembly is arranged between the main unit and the liquid storage apparatus; and
the liquid storage apparatus is detachably mounted to the main unit in a plug-in manner via the slide channel assembly.

Optionally, the device having a nebulization function is a nebulization therapeutic device.

In the embodiment of the present disclosure, by arranging a first limiting structure on the nebulization assembly and a second limiting structure on the main unit, when the liquid storage apparatus is assembled to the main unit, the first limiting structure and the second limiting structure are snap-fitted with each other, so that the nebulization assembly and the liquid storage apparatus can be fixed in relation to each other, and both the liquid storage apparatus and the nebulization assembly are in a stationary state relative to the main unit and will not rotate in relation to each other, so that a problem of liquid leakage during the use of the device is avoided, and the risk of a short circuit between the probes on the main unit is reduced; when the liquid storage apparatus is separated from the main unit, the nebulization assembly and the liquid storage apparatus can be dismounted, so that the user can replace the nebulization plate conveniently by himself/herself, and the convenience of use is improved.

Another object of the embodiments of the present disclosure is to provide a safe structure or method to prevent the interface electrodes of another type of power supply from being touched by the human body when one type of power supply is connected, thereby enhance the safety.

**In** order to achieve the above object, in a first aspect, the present disclosure provides a power supply mutual exclusion method for an electronic product that has a battery compartment for mounting a battery, a battery compartment cover for opening and closing a compartment opening of the battery compartment, and an external power supply interface for plugging a power plug, the power supply mutual exclusion method comprising: configuring the battery compartment cover so that the battery compartment cover at least partially shields the external power supply interface when it opens the compartment opening and doesn't shield the external power supply interface when it closes the compartment opening.

According to the power supply mutual exclusion method for an electronic product in the present disclosure, with the above technical scheme, when the battery compartment cover is opened during use, a power plug can't be plugged into the external power supply interface because the battery compartment cover at least partially shields the external power supply interface; in that case, if no battery is mounted, the battery interface electrodes in the battery compartment are accessible, but, since there is no external power supply connected currently, there is no safety risk incurred by touching the battery interface electrodes; if a battery is mounted, only the battery is accessible, and there is no safety risk. A power plug can be plugged into the external power supply interface only when the battery compartment cover is closed; in that state, the battery electrodes and the battery interface electrodes are inaccessible, so there is no safety risk. Therefore, with the power mutual exclusion method in the present disclosure, the battery interface electrodes are inaccessible for the human body when an external power supply is connected; when the battery interface electrodes are accessible, no external power supply can be connected; thus, the safety is enhanced.

Optionally, the power supply mutual exclusion method comprises: configuring the power plug to block the battery compartment cover when the power plug is plugged into the external power supply interface, so that the battery compartment cover can't be opened or can't be fully opened.

In a second aspect, the present disclosure provides a power supply mutual exclusion structure, which comprises a battery compartment, a battery compartment cover and an external power supply interface, wherein the external power supply interface is used for plugging a power plug; the bottom of the battery compartment is provided with battery interface electrodes; the battery compartment has a compartment opening for loading a battery therein; the battery compartment cover is movably arranged at the compartment opening to open and close the compartment opening; the battery compartment cover is configured as follows: the battery compartment cover at least partially shields the external power supply interface when it opens the compartment opening; and the battery compartment cover doesn't shield the external power supply interface when it closes the compartment opening.

According to the power supply mutual exclusion structure in the present disclosure, with the above technical scheme, when the battery compartment cover is opened during use, a power plug can't be plugged into the external power supply interface because the battery compartment cover at least partially shields the external power supply interface; in that case, if no battery is mounted, the battery interface electrodes are accessible, but, since there is no external power supply connected currently, there is no safety risk incurred by touching the battery interface electrodes; if a battery is mounted, only the battery is accessible, and there is no safety risk. A power plug can be plugged into the external power supply interface only when the battery compartment cover is closed; in that state, the battery electrodes and the battery interface electrodes are inaccessible, so there is no safety risk. Therefore, with the power mutual exclusion structure in the present disclosure, the battery interface electrodes are inaccessible for the human body when an external power supply is connected; when the battery interface electrodes are accessible, no external power supply can be connected; thus, the safety is enhanced.

Optionally, the battery compartment cover opens and closes the compartment opening by rotation or sliding.

Optionally, the compartment opening and the external power supply interface are positioned on the same side, the battery compartment cover is rotatably arranged at the compartment opening, with a rotation axis of the battery compartment cover positioned at one side of the compartment opening and the external power supply interface positioned near the rotation axis.

Optionally, the power supply mutual exclusion structure is configured as follows: when the power plug is plugged into the external power supply interface, the power plug can block the battery compartment cover, so that the battery compartment cover can't be opened or can't be fully opened.

Optionally, the battery compartment cover comprises a cover body and a first side wing connected to a side of the cover body, wherein the first side wing is provided with two shaft holes arranged opposite to each other, and two rotating shafts are arranged opposite to each other at a side of the compartment opening, and the two rotating shafts are rotatably inserted into the two shaft holes respectively.

Optionally, the first side wing comprises an arc-shaped portion arranged between the two shaft holes, and the arc-shaped portion is positioned around the periphery of the power plug when the power plug is plugged into the external power supply interface.

Optionally, the power supply mutual exclusion structure further comprises a locking structure for releasably locking the battery compartment cover in its closed position.

Optionally, the battery compartment cover comprises a second side wing connected to an opposite side of the cover body, and the locking structure comprises a first snap-fitting clip arranged at an opposite side of the compartment opening and a second snap-fitting clip arranged on the second side wing and snap-fitted with the first snap-fitting clip.

**In** a third aspect, the present disclosure provides an electronic product, which comprises the power supply mutual exclusion structure described above.

With the power mutual exclusion structure described above, the electronic product in the present disclosure ensures that the battery interface electrodes are inaccessible for the human body when an external power supply is connected; and when the battery interface electrodes are accessible, no external power supply can be connected; thus, the safety of use of the electronic product is effectively enhanced.

Optionally, the electronic product is a nebulizer.

Optionally, the power supply mutual exclusion structure is arranged at the bottom of the nebulizer.

Another object of the embodiments of the present disclosure is to provide a power management system and a nebulization therapeutic device, which can solve a problem of poor safety and poor reliability of the nebulization therapeutic device in a dual power supply mode in the prior art.

In order to solve the above technical problem, the present disclosure is implemented as follows:
In a first aspect, an embodiment of the present disclosure provides a power management system, which comprises a single-chip microcomputer, a battery circuit, a power adapter circuit, a voltage detection unit and a control circuit, wherein
the battery circuit and the power adapter circuit are respectively connected to the single-chip microcomputer;
the voltage detection unit is respectively connected to the battery circuit and a first input pin of the single-chip microcomputer, and the single-chip microcomputer acquires a battery voltage through the voltage detection unit;
the control circuit is respectively connected to the single-chip microcomputer, the battery circuit and the power adapter circuit;
the control circuit is used for controlling the opening and closing of the battery circuit or the power adapter circuit according to a control signal outputted by the single-chip microcomputer;
the control circuit is used to keep the battery circuit in a persistently open state when the single-chip microcomputer is powered through the battery circuit;
the control circuit is used to keep the battery circuit in a closed state when the battery voltage is lower than a first voltage threshold;
and the control circuit is used to keep the battery circuit in the closed state when the single-chip microcomputer is powered through the power adapter circuit.

Optionally, the power management system further comprises an overvoltage protection circuit; wherein
the overvoltage protection circuit is respectively connected to the power adapter circuit and the control circuit; and
the overvoltage protection circuit and the control circuit jointly control the power adapter circuit to be in a closed state when the voltage of the power adapter circuit exceeds a second voltage threshold.

Optionally, the battery circuit comprises: a battery, a first controllable switch, a voltage converter and a second controllable switch, wherein
the battery is connected to the single-chip microcomputer via the first controllable switch, the voltage converter and the second controllable switch;
the single-chip microcomputer comprises a first output pin and a second output pin;
the control circuit comprises a third controllable switch and a fourth controllable switch, wherein the third controllable switch is respectively connected to the first controllable switch and the first output pin, and the fourth controllable switch is respectively connected to the second controllable switch and the second output pin;
the third controllable switch is used for controlling the opening and closing of the first controllable switch according to a control signal outputted via the first output pin; and
the fourth controllable switch is used for controlling the opening and closing of the second controllable switch according to a control signal outputted via the second output pin.

Optionally, the power adapter circuit comprises a power adapter and the second controllable switch, wherein
the power adapter is connected to the single-chip microcomputer via the second controllable switch.

Optionally, the first controllable switch is a first PMOS tube, and the second controllable switch is a second PMOS tube, wherein
the battery is connected to the single-chip microcomputer via a source electrode and a drain electrode of the first PMOS tube, the voltage converter, and a source electrode and a drain electrode of the second PMOS tube;
the third controllable switch is a first transistor, and the fourth controllable switch is a second transistor;
a base electrode of the first transistor is electrically connected to the first output pin, a collector electrode of the first transistor is electrically connected to a grid electrode of the first PMOS tube and the battery, and an emitter electrode of the first transistor is grounded;
a base electrode of the second transistor is electrically connected to the second output pin, a collector electrode of the second transistor is electrically connected to a grid electrode of the second PMOS tube and the power adapter, and an emitter electrode of the second transistor is grounded;
the first transistor is used for controlling the opening and closing of the first PMOS tube according to a control signal outputted via the first output pin;
the second transistor is used for controlling the opening and closing of the second PMOS tube according to a control signal outputted via the second output pin; and
the power adapter is connected to the single-chip microcomputer via the source electrode and the drain electrode of the second PMOS tube.

Optionally, the power adapter circuit further comprises a connection detection circuit; and the single-chip microcomputer further comprises a second input pin, and the connection detection circuit is electrically connected to the second input pin.

Optionally, the overvoltage protection circuit comprises a first diode and a fifth controllable switch;
a cathode of the first diode is electrically connected to an output terminal of the power adapter;
the fifth controllable switch is electrically connected to an anode of the first diode and the second output pin respectively; and
when the output voltage of the power adapter exceeds a second voltage threshold, the first diode is broken down, and the fifth controllable switch and the fourth controllable switch jointly control the second controllable switch to be in an OFF state.

Optionally, the fifth controllable switch is a third transistor;
an anode of the first diode is electrically connected to a base electrode of the third transistor, a collector electrode of the third transistor is electrically connected to the second output pin, and an emitter electrode of the third transistor is grounded; and
when the output voltage of the power adapter exceeds the second voltage threshold, the first diode is broken down, and the third transistor and the second transistor jointly control the second PMOS tube to be in an OFF state.

Optionally, the power management system further comprises a second diode and a third diode, wherein
an anode of the second diode is electrically connected to the output terminal of the voltage converter, and a cathode of the second diode is electrically connected to the second PMOS tube; and an anode of the third diode is electrically connected to the output terminal of the power adapter, and a cathode of the third diode is electrically connected to the second PMOS tube.

Optionally, the power management system further comprises a key switch circuit, wherein the key switch circuit comprises a first resistor, a second resistor, a fourth diode, a fifth diode and a mechanical switch;
one end of the first resistor is electrically connected to the battery and the source electrode of the first PMOS tube, the other end of the first resistor is electrically connected to an anode of the fourth diode and the grid electrode of the first PMOS tube, and a cathode of the fourth diode is connected to the mechanical switch;
one end of the second resistor is electrically connected to the source electrode of the second PMOS tube, the other end of the second resistor is electrically connected to an anode electrode of the fifth diode and the grid electrode of the second PMOS tube, and a cathode of the fifth diode is connected to the mechanical switch; and
the key switch circuit is used for controlling the on/off of the power management system.

In a second aspect, an embodiment of the present disclosure further provides a nebulization therapeutic device, which comprises the power management system described above.

In the embodiment of the present disclosure, after the user controls the single-chip microcomputer to turn on with the mechanical key, the control circuit can keep the battery circuit in a persistently open state when the single-chip microcomputer is powered through the battery circuit; when the single-chip microcomputer detects that the voltage of the battery is lower than a first voltage threshold, the single-chip microcomputer outputs a control signal to switch off the battery circuit through the control circuit, so as to avoid damage to the product caused by over-discharge of the battery and improve the reliability of the product; in the case that the single-chip microcomputer is powered through the power adapter circuit, the single-chip microcomputer outputs a control signal, so as to switch off the battery circuit through the control circuit, thereby the battery stops supplying power, thus the battery is cut off automatically once the power adapter is connected, and the battery and the power adapter will not interfere with each other, thus the safety is improved, and power waste of the battery is effectively avoided.

Other features and advantages of the present disclosure will be further detailed in the following embodiments.

### BRIEF DESCRIPTION OF THE DRAWINGS

Fig. 1 is a flowchart of the method for dismounting a nebulization assembly in an embodiment of the present disclosure;
Fig. 2 is a flowchart of the method for mounting a nebulization assembly in an embodiment of the present disclosure;
Fig. 3 is a schematic structural diagram of the assembly of the nebulization therapeutic device in an embodiment of the present disclosure;
Fig. 4 is an exploded structural view of the nebulization therapeutic device in the embodiment of the present disclosure;
Fig. 5 is a first schematic structural diagram of the assembly of the liquid storage apparatus and the nebulization assembly in an embodiment of the present disclosure;
Fig. 6 is a schematic structural diagram of the main unit in an embodiment of the present disclosure;
Fig. 7 is a second schematic structural diagram of the assembly of the liquid storage apparatus and the nebulization assembly in an embodiment of the present disclosure;
Fig. 8 is a schematic structural diagram of the mesh-type nebulizer in an embodiment of the present disclosure;
Fig. 9 is a schematic structural diagram of the medicine cup assembly in an embodiment of the present disclosure;
Fig. 10 is a schematic structural diagram of the main unit assembly in an embodiment of the present disclosure;
Fig. 11 is an exploded structural view of the medicine cup assembly in an embodiment of the present disclosure;
Fig. 12 is a schematic structural diagram of the medicine cup device in an embodiment of the present disclosure;
Fig. 13 is a schematic structural diagram of the nebulization device in an embodiment of the present disclosure;
Fig. 14 is a schematic structural diagram of the medicine cup body in an embodiment of the present disclosure;
Fig. 15 is a schematic structural diagram of the locking element in an embodiment of the present disclosure;
Fig. 16 is an exploded structural view of the nebulization device in an embodiment of the present disclosure;
Fig. 17 is an exploded structural view of the main unit assembly in an embodiment of the present disclosure;
Fig. 18 is a first schematic structural diagram of the assembly having a nebulization function in an embodiment of the present disclosure;
Fig. 19 is a second schematic structural diagram of the assembly having a nebulization function in an embodiment of the present disclosure;
Fig. 20 is a schematic sectional view A-A of the structure in Fig. 19 in an embodiment of the present disclosure;
Fig. 21 is a schematic sectional view B-B of the structure in Fig. 19 in an embodiment of the present disclosure;
Fig. 22 is an enlarged structural view of a part I in Fig. 20 in an embodiment of the present disclosure;
Fig. 23 is an enlarged structural view of a part II in Fig. 21 in an embodiment of the present disclosure;
Fig. 24 is a schematic structural diagram of the nebulization device in an embodiment of the present disclosure;
Fig. 25 is a perspective view of an embodiment of the nebulizer in the present disclosure;
Fig. 26 is a perspective view of the nebulizer in Fig. 25 from another angle;
Fig. 27 is a schematic diagram of the medicine cup assembly in Fig. 26 before the medicine cup assembly is mounted to the main unit;
Fig. 28 is a sectional view of the nebulizer in Fig. 26;
Fig. 29 is a front view of the main unit in Fig. 27;
Fig. 30 is a perspective view of the main unit in Fig. 29;
Fig. 31 is a perspective view of the medicine cup assembly in Fig. 27;
Fig. 32 is a front view of the medicine cup assembly in Fig. 31;
Fig. 33 is a schematic structural diagram of the device having a nebulization function in an embodiment of the present disclosure;
Fig. 34 is a schematic assembly diagram of the device having a nebulization function in an embodiment of the present disclosure;
Fig. 35 is an exploded view of the liquid storage apparatus and the nebulization assembly in an embodiment of the present disclosure;
Fig. 36 is a schematic assembly diagram of the liquid storage apparatus and the nebulization assembly in an embodiment of the present disclosure;
Fig. 37 is a first schematic structural diagram of the main unit in an embodiment of the present disclosure;
Fig. 38 is a second schematic structural diagram of the main unit in an embodiment of the present disclosure;
Fig. 39 is a first flowchart of mounting of the nebulization assembly and the liquid storage apparatus in an embodiment of the present disclosure;
Fig. 40 is a second flowchart of mounting of the nebulization assembly and the liquid storage apparatus in an embodiment of the present disclosure;
Fig. 41 is a bottom perspective view of an embodiment of the nebulizer in the present disclosure,
with the battery compartment cover in a closed position;
Fig. 42 is an exploded view of the nebulizer in Fig. 41;
Fig. 43 is a schematic structural diagram of the other side of the battery compartment cover in Fig. 42;
Fig. 44 is a front view of the nebulizer in Fig. 41;
Fig. 45 is a partially sectional view of the nebulizer in Fig. 44, mainly showing a positional relationship between the closed battery compartment cover and the power plug;
Fig. 46 is a schematic view of the battery compartment cover of the nebulizer in Fig. 41 in a fully open state;
Fig. 47 is a front view of the nebulizer in Fig. 46;
Fig. 48 is a partially sectional view of the nebulizer in Fig. 46, mainly showing a positional relationship between the fully opened battery compartment cover and the external power supply interface;
Fig. 49 is a schematic view of the nebulizer in Fig. 46 with the battery removed;
Fig. 50 is a front view of the nebulizer in Fig. 49;
Fig. 51 is a technical block diagram of the power management system in an embodiment of the present disclosure;
Fig. 52 is a workflow chart of the power management system in an embodiment of the present disclosure; and
Fig. 53 is a schematic circuit diagram of the power management system in an embodiment of the present disclosure.

### Reference Numerals

10 - main unit; 20 - liquid storage apparatus; 30 - nebulization assembly; 40 - first slide channel; 13 - locking element; 101 - second limiting structure; 102 - snap-fitting clip; 201 - mounting base; 202 - second snap-fitting part; 301 - first limiting structure; 302 - first snap-fitting part; 401 - third limiting structure; 31 - medicine cup assembly; 11 - nebulization device; 111 - first housing; 1112 - protrusion; 1113 - metal contact; 1110 - second sealing element; 113 - nebulization plate; 114 - second housing; 12 - medicine cup device; 1211 - limiting groove; 12111 - first bump; 122 - medicine cup body; 1221 - accommodating cavity; 12211 - boss; 12212 - second recess; 123 - medicine cup cap fastener; 124 - medicine cup cap; 125 - first sealing element; 13 - locking element; 131 - raised rib; 1311 - first recess; 14 - first sliding connection structure; 19 - main unit assembly; 21 - second sliding connection structure; 22 - metal probe; 215 - main unit front housing; 216 - switch button; 203 - aerosol yield adjustment button; 204 - main unit front and rear housings seal ring; 205 - main PCB; 206 - battery compartment cover; 207 - battery contact plate; 208 - battery compartment; 209 - power supply interface board; 210 - power supply interface protection cover; 211 - main unit rear housing; 212 - decorative ring; 213 - probe connection plate; 214 - probe sealant pad; 61 - nebulization module; 51 - detection electrode; 110 - liquid storage cavity; 112 - liquid outlet; 153 - accommodating slot; 16 - mounting part; 130 - snap-fitting portion; 132 - pressing portion; 140 - outer sidewall; 15 - U-shaped cantilever arm; 161 - recess; 220 - mounting cavity; 221 - bottom opening; 23 - side opening; 24 - retaining rib; 25 - snap-fitting groove; 26 - upper edge; 400 - second slide channel; 402 - conductive probe; 50 - slide channel assembly; 224 - clearance hole; 208 - battery compartment; 133 - rotating shaft; 162 - first snap-fitting clip; 206 - battery compartment cover; 231 - cover body; 232 - first side wing; 233 - shaft hole; 234 - arc-shaped portion; 235 - second side wing; 236 - second snap-fitting clip; 3 - external power supply interface; 4 - power plug; 5 - battery interface electrode; 6 - battery; 7 - nebulizer; 71 - housing; 72 - bottom wall; 9 - single-chip microcomputer; 41 - battery circuit; 42 - power adapter circuit; 43 - voltage detection unit; 44 - control circuit; 60 - first resistor; 70 - second resistor; 80 - mechanical switch; 141 - first input pin; 142 - first output pin; 143 - second output pin; 144 - second input pin; 242 - first PMOS tube; 243 - voltage converter; 244 - second PMOS tube; 245 - second diode; 246 - fourth diode; 341 - power adapter; 342 - third diode; 343 - fifth diode; 501 - first transistor; 502 - second transistor; 503 - first diode; 504 - third transistor.

### DETAILED DESCRIPTION

The technical schemes in the embodiments of the present disclosure will be detailed below clearly and completely with reference to the accompanying drawings of the embodiments. Obviously, the embodiments described herein are only some embodiments of the present disclosure, but not all possible embodiments of the present disclosure. Those having ordinary skills in the art can obtain other embodiments on the basis of the embodiments described herein without expending any creative labor; however, all such embodiments shall be deemed as falling in the scope of protection of the present disclosure.

The terms "first" and "second", etc. in the Description and Claims of the present disclosure are intended to distinguish similar objects, rather than to describe a specific order or precedence. It should be understood that the data used in such a manner can be interchanged in appropriate circumstances, so that the embodiments of the present disclosure can be implemented in an order other than the order illustrated or described herein, and the objects distinguished by "first", "second", etc. are usually of the same category, and the specific number of objects is not limited, for example, a first object may be one object or a plurality of objects. In addition, the phrase "and/or" used in the Description and Claims means at least one of the objects connected by the phrase, and the character "/" usually means that the correlated objects are in an "OR" relationship.

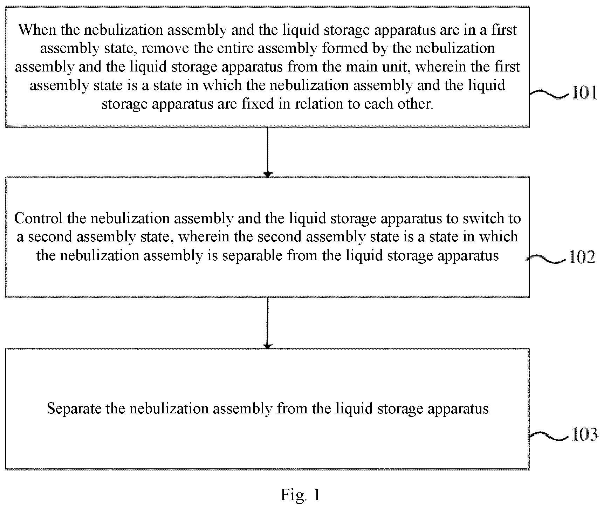

A dismounting and mounting method for a nebulization assembly and a nebulization therapeutic device provided in the embodiments of the present disclosure will be described in detail below in specific embodiments and application scenarios with reference to Figs. 1-7.

The nebulizer comprises a main unit 10, a liquid storage apparatus 20 and a nebulization assembly 30, wherein the liquid storage apparatus 20 is detachably mounted to the main unit 10, and the nebulization assembly 30 is detachably mounted to the liquid storage apparatus 20. The liquid storage apparatus 20 is used to store a liquid medicine to be nebulized, a side of the liquid storage apparatus 20 is provided with a liquid outlet, and the nebulization assembly 30 and the liquid storage apparatus 20 are assembled together in a detachable manner; in addition, the liquid inlet of the nebulization assembly 30 is in communication with the liquid outlet of the liquid storage apparatus 20, so that the liquid storage apparatus 20 can deliver the liquid medicine to the nebulization assembly 30. The nebulization assembly 30 nebulizes the liquid medicine passing through fine pores of the nebulization plate into nebulized particles by means of the high-frequency vibration of the nebulization plate, then the nebulized particles are inhaled into a human body through an inhalation device (a mask or mouthpiece). The main unit 10 is used to supply power to the nebulization assembly 30, and the user can perform corresponding operations on the main unit 10 to realize functions such as start and stop; besides, the main unit 10 can further provide a holding space for the user, so that the user can use the device conveniently.

As shown in Fig. 1 and Fig. 3 to Fig. 6, in a first aspect, an embodiment of the present disclosure provides a method for dismounting a nebulization assembly, which comprises:
Step 101: removing an entire assembly formed by a nebulization assembly 30 and a liquid storage apparatus 20 from a main unit 10 when the nebulization assembly 30 and the liquid storage apparatus 20 are in a first assembly state, wherein the first assembly state is a state in which the nebulization assembly 30 and the liquid storage apparatus 20 are fixed in relation to each other.

The liquid storage apparatus 20 is detachably mounted to the main unit 10 in a plug-in manner. When the nebulization assembly 30 and the liquid storage apparatus 20 are in the first assembly state, the nebulization assembly 30 and the liquid storage apparatus 20 are fixed in relation to each other, which is to say, there is no displacement or rotation between the nebulization assembly 30 and the liquid storage apparatus 20 in the first assembly state, thereby the liquid medicine can be prevented from leaking at a joint between the nebulization assembly 30 and the liquid storage apparatus 20. Compared with directly removing the nebulization assembly 30 from the main unit 10, removing an entire assembly formed by the nebulization assembly 30 and the liquid storage apparatus 20 in the first assembly state from the main unit 10 can avoid leakage of the liquid medicine, thereby a problem that the liquid medicine invades into the main unit 10 and make the main unit 10 short-circuited can be avoided.

Step 102: controlling the nebulization assembly 30 and the liquid storage apparatus 20 to switch to a second assembly state, wherein the second assembly state is a state in which the nebulization assembly 30 and the liquid storage apparatus 20 are separatable.

Specifically, after the entire assembly formed by the nebulization assembly 30 and the liquid storage apparatus 20 is removed from the main unit 10, the liquid medicine in the liquid storage apparatus 20 is separated from the main unit 10, thus a risk of the liquid medicine invading into the main unit 10 is eliminated, i.e., the nebulization assembly 30 and the liquid storage apparatus 20 can be switched to the second assembly state, in which the nebulization assembly 30 and the liquid storage apparatus 20 can be separated. By switching between the first assembly state and the second assembly state, the nebulization assembly 30 and the liquid storage apparatus 20 can be mounted and dismounted. During normal use, the nebulization assembly 30 and the liquid storage apparatus 20 are switched to the first assembly state; when the nebulization assembly 30 is to be dismounted, the nebulization assembly 30 and the liquid storage apparatus 20 are switched to the second assembly state, thereby a problem that the nebulization assembly 30 and the liquid storage apparatus 20 are separated by mistake can be avoided.

Step 103: separating the nebulization assembly 30 from the liquid storage apparatus 20.

Specifically, after the entire assembly formed by the nebulization assembly 30 and the liquid storage apparatus 20 is removed from the main unit 10 and the nebulization assembly 30 and the liquid storage apparatus 20 are switched to the second assembly state, the nebulization assembly 30 and the liquid storage apparatus 20 can be separated. In the process of separation, the nebulization assembly 30 can be pulled out of the liquid storage apparatus 20, or can be taken down by rotation; the specific separation action may be determined according to the assembly method of the nebulization assembly 30 and the liquid storage apparatus 20. In the process of dismounting the nebulization assembly 30, even if the liquid medicine leaks, it will not affect the main unit 10, thereby the safety of use is greatly improved.

In the embodiment of the present disclosure, when performing dismounting of the nebulization assembly 30, an entire assembly formed by the nebulization assembly 30 and a liquid storage apparatus 20 may be removed from a main unit 10 when the nebulization assembly 30 and the liquid storage apparatus 20 are in the first assembly state; because the nebulization assembly 30 and the liquid storage apparatus 20 are fixed in relation to each another in the first assembly state, a problem of liquid medicine leakage is prevented, and a risk of a short circuit between the probes on the main unit 10 is reduced. After the entire assembly formed by the nebulization assembly 30 and the liquid storage apparatus 20 is removed from the main unit 10, the nebulization assembly 30 and the liquid storage apparatus 20 are switched to a second assembly state; at that point, the nebulization assembly 30 and the liquid storage apparatus 20 are in a separable state, and the nebulization assembly 30 may be smoothly taken off of the liquid storage apparatus 20; even if liquid medicine leakage occurs in this process, the main unit 10 is unaffected, thereby the safety of use is greatly improved.

Optionally, in Step 101, removing the entire assembly formed by the nebulization assembly 30 and the liquid storage apparatus 20 from the main unit 10 when the nebulization assembly 30 and the liquid storage apparatus 20 are in the first assembly state comprises:
Step 1011: controlling the liquid storage apparatus 20 and the main unit 10 to switch from a locked state to an unlocked state.

Specifically, the nebulization assembly 30 is assembled to the main unit 10 via the liquid storage apparatus 20, and the liquid storage apparatus 20 may be removed from the main unit 10 in the dismounting process. The liquid storage apparatus 20 and the main unit 10 may be in a locked state or an unlocked state. In the locked state, the liquid storage apparatus 20 is fixed in relation to the main unit 10, and the liquid storage apparatus 20 can't be removed from the main unit 10. The liquid storage apparatus 20 can be removed from the main unit 10 only after the liquid storage apparatus 20 and the main unit 10 are switched to the unlocked state. By defining a locked state and an unlocked state, the liquid storage apparatus 20 and the main unit 10 are switched to the locked state during use, so that any accidental separation of the liquid storage apparatus 20 from the main unit 10 caused by misoperations can be avoided, and the safety factor is higher.

The locked state and the unlocked state of the liquid storage apparatus 20 and the main unit 10 can be realized by means of electromagnets or physical snap-fitting clips 102, etc. For example, the liquid storage apparatus 20 and the main unit 10 are respectively provided with an electromagnet. When the electromagnets are energized, the liquid storage apparatus 20 and the main unit 10 are in the locked state; after the power supply to the electromagnets is cut off, the liquid storage apparatus 20 and the main unit 10 are switched to the unlocked state.

Step 1012: sliding the liquid storage apparatus 20 along a slide channel 40 on the main unit 10, and separating a first limiting structure 301 on the nebulization assembly 30 from a second limiting structure 101 on the main unit 10 at the same time, so as to remove the entire assembly formed by the nebulization assembly 30 and the liquid storage apparatus 20 from the main unit 10;
wherein the first limiting structure 301 is snap-fitted with the second limiting structure 101 when the liquid storage apparatus 20 and the main unit 10 are in the locked state.

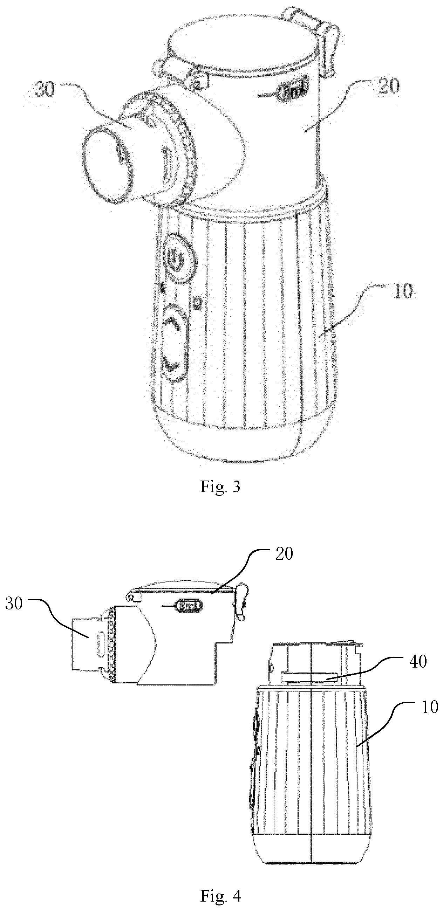

Specifically, as shown in Fig. 4, the main unit 10 is provided with a first slide channel 40 on the sides for assembling with the liquid storage apparatus 20, and the first slide channel 40 are symmetrically arranged on two sides of the main unit 10; the liquid storage apparatus 20 is provided with a structure matching the first slide channel 40 at the bottom of the liquid storage apparatus 20, and the liquid storage apparatus 20 is at least partially embedded in the first slide channel 40 to realize sliding insertion into and sliding removal from the main unit 10. After the liquid storage apparatus 20 and the main unit 10 are switched to the unlocked state, the liquid storage apparatus 20 can slide along the first slide channel 40 on the main unit 10, and finally is disengaged from the first slide channel 40. With the first slide channel 40, the plug-in/plug-out process of the liquid storage apparatus 20 can be guided and limited, and the stability is improved.

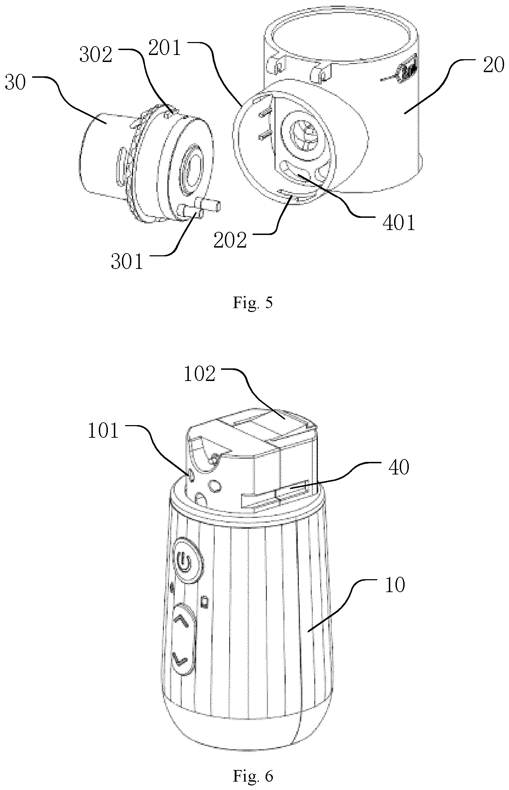

As shown in Fig. 5 and Fig. 6, the nebulization assembly 30 is provided with a first limiting structure 301, the main unit 10 is provided with a second limiting structure 101, and the first limiting structure 301 and the second limiting structure 101 can be snap-fitted with each other. The first limiting structure 301 and the second limiting structure 101 may be a limiting rod and a limiting hole, or may be a limiting baffle and a limiting groove, or may be other structures, as long as they can be snap-fitted together. There is no particular restriction on their specific structures in the embodiment of the present disclosure.

When the liquid storage apparatus 20 is locked to the main unit 10, i.e., after the liquid storage apparatus 20 has been plugged into the main unit 10, the first limiting structure 301 and the second limiting structure 101 are snap-fitted together, and, under the limiting action of the first limiting structure 301 and the second limiting structure 101, the nebulization assembly 30 and the main unit 10 will not be displaced or rotated in relation to each other; besides, since the liquid storage apparatus 20 will not be displaced or rotated in relation to the main unit 10, the nebulization assembly 30 and the liquid storage apparatus 20 are fixed in relation to each other, and the problem of liquid leakage caused by relative rotation of the nebulization assembly 30 and the liquid storage apparatus 20 due to misoperations or other reasons during the use of the device is avoided.

After the liquid storage apparatus 20 and the main unit 10 are switched to the unlocked state, the liquid storage apparatus 20 is pushed or pulled to slide along the first slide channel 40 on the main unit 10, so that it drives the nebulization assembly 30 to move in relation to the main unit 10; at the same time, the first limiting structure 301 on the nebulization assembly 30 is disengaged from the second limiting structure 101 on the main unit 10, so that the entire assembly formed by the nebulization assembly 30 and the liquid storage apparatus 20 is removed from the main unit 10. Then, the nebulization assembly 30 can be dismounted from the liquid storage apparatus 20, without any adverse effect on the main unit 10.

Optionally, controlling the liquid storage apparatus 20 and the main unit 10 to switch from a locked state to an unlocked state in Step 1011 comprises:
Step 1011a: switching at least one snap-fitting clip 102 on the main unit 10 and at least one snap-fitting groove in the liquid storage apparatus 20 from a snap-fitted state to a disengaged state; and/or switching at least one snap-fitting groove in the main unit 10 and at least one snap-fitting clip 102 on the liquid storage apparatus 20 from a snap-fitted state to a disengaged state;
wherein the liquid storage apparatus 20 and the main unit 10 are in the locked state when the snap-fitting clips 102 and the snap-fitting grooves are in the snap-fitted state; and the liquid storage apparatus 20 and the main unit 10 are in the unlocked state when the snap-fitting clips 102 and the snap-fitting grooves are in the disengaged state.

Specifically, the locked state and unlocked state of the liquid storage apparatus 20 and the main unit 10 can be realized by means of the snap-fitting between the snap-fitting groove and the snap-fitting clip 102. In an embodiment, as shown in Fig. 6, a movable snap-fitting clip 102 is arranged at the top of the main unit 10, and a snap-fitting groove is arranged in the liquid storage apparatus 20; when the snap-fitting clip 102 and the snap-fitting grooves are snap-fitted, the liquid storage apparatus 20 and the main unit 10 are in the locked state. The snap-fitting clip 102 and the snap-fitting groove can be switched from the snap-fitted state to the disengaged state by controlling the position of the snap-fitting clip 102, so that the liquid storage apparatus 20 and the main unit 10 are in the unlocked state. For example, the snap-fitting clip 102 and the snap-fitting groove are in the locked state when the snap-fitting clip 102 protrudes from the upper end surface of the main unit 10; the snap-fitting clip 102 and the snap-fitting groove are in the disengaged state when the snap-fitting clip 102 is moved downward so that it is flush with or lower than the upper end face of the main unit 10. The position of the snap-fitting clip 102 may be controlled manually, or may be controlled by a means of a driving mechanism such as a motor or an air cylinder. For example, the snap-fitting clip 102 has an elastic tab extending out of the main unit, and the snap-fitting clip can be disengaged from the snap-fitting groove by pressing the elastic tab to unlock the liquid storage apparatus 20 from the main unit 10.

As a variant, a snap-fitting groove may be provided at the top of the main unit 10, and a movable snap-fitting clip 102 may be provided on the liquid storage apparatus 20.

Optionally, controlling the liquid storage apparatus 20 and the main unit 10 to switch from a locked state to an unlocked state in Step 1011 comprises:
Step 1011b: switching a key on the main unit 10 from a first state to a second state;
wherein the liquid storage apparatus 20 and the main unit 10 are in the locked state when the key is in the first state; and the liquid storage apparatus 20 and the main unit 10 are in the unlocked state when the key is in the second state.

Specifically, the liquid storage apparatus 20 is fastened to the main unit 10 by means of a key, and the working principle of the key is as follows: The end of the key is a semi-arc retraining rib and is connected to a spring, and the spring can be elastically retracted by manual pressing, thereby the key is driven to retract. When the key is pressed, the retaining rib moves backward to release the fastening between the liquid storage apparatus 20 and the main unit 10, so that the liquid storage apparatus 20 falls off.

Optionally, controlling the nebulization assembly 30 and the liquid storage apparatus 20 to switch to the second assembly state in Step 102 comprises:
Step 1021: rotating a locking element 13 on the liquid storage apparatus 20 to switch the locking element 13 and the liquid storage apparatus 20 from a connected state to a separated state;
wherein the locking element 13 is detachably connected to the liquid storage apparatus 20 by rotation, and the nebulization assembly 30 is positioned between the locking element 13 and the liquid storage apparatus 20;
the nebulization assembly 30 and the liquid storage apparatus 20 are in the first assembly state when the locking element 13 and the liquid storage apparatus 20 are in the connected state; and the nebulization assembly 30 and the liquid storage apparatus 20 are in the second assembly state when the locking element 13 and the liquid storage apparatus 20 are in the separated state.

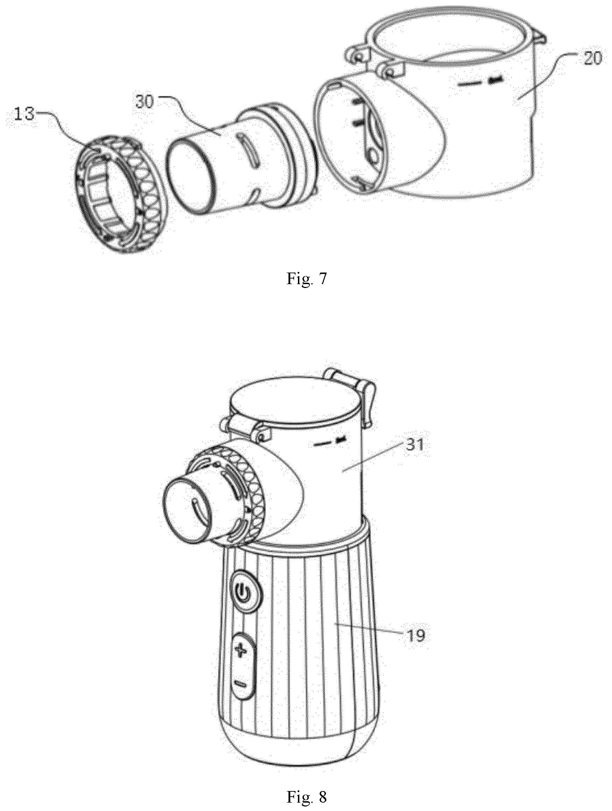

Specifically, as shown in Fig. 7, the liquid storage apparatus 20 is provided with an accommodating cavity in one side, and one end of the nebulization assembly 30 is at least partially inserted into the accommodating cavity. The locking element 13 is fitted around the nebulization assembly 30, and may be of an annular structure, and the nebulization assembly 30 is positioned between the locking element 13 and the liquid storage apparatus 20; when the locking element 13 is connected and fixed to the sidewall of the accommodating cavity, the nebulization assembly 30 can be squeezed and fixed in the accommodating cavity.

The locking element 13 is detachably connected to the sidewall of the accommodating cavity by rotation, specifically, it can be connected by means of a threaded connected or snap-fitted connection, etc. When the locking element 13 is connected to the liquid storage apparatus 20, the nebulization assembly 30 and the liquid storage apparatus 20 are in the first assembly state, and the nebulization assembly 30 can't be dismounted; the locking element 13 is separated from the liquid storage apparatus 20 by rotating the locking element 13; after the restraining effect of the locking element 13 is lost, the nebulization assembly 30 and the liquid storage apparatus 20 are in the second assembly state and can be dismounted.

Optionally, controlling the nebulization assembly 30 and the liquid storage apparatus 20 to switch to the second assembly state in Step 102 comprises:
Step 1022: controlling the nebulization assembly 30 and a mounting base 201 to rotate from a first relative position to a second relative position;
wherein the liquid storage apparatus 20 is provided with the mounting base 201, and the nebulization assembly 30 is at least partially embedded in the mounting base 201 and rotatably connected to the mounting base 201;
wherein the nebulization assembly 30 and the mounting base 201 are fixed in relation to each other when the nebulization assembly 30 and the mounting base 201 are in the first relative position; and the nebulization assembly 30 and the mounting base 201 can be separated from each other when the nebulization assembly 30 and the mounting base 201 are in the second relative position.

Specifically, as shown in Fig. 5 and Fig. 6, the nebulization assembly 30 can be connected to the liquid storage apparatus 20 by rotation. The liquid storage apparatus 20 is provided with the mounting base 201, and the nebulization assembly 30 is at least partially embedded in the mounting base 201, thereby the reliability of the connection between the nebulization assembly 30 and the liquid storage apparatus 20 is ensured. The nebulization assembly 30 and the mounting base 201 are detachably connected by rotation, and the rotation angle and direction can be determined according to the actual design. When the nebulization assembly 30 and the mounting base 201 are in the first relative position, the nebulization assembly 30 and the mounting base 201 are fixed in relation to each other, which is to say, the nebulization assembly 30 and the liquid storage apparatus 20 are in the first assembly state; when the nebulization assembly 30 and the mounting base 201 are rotated to the second relative position, the nebulization assembly 30 and the mounting base 201 can be separated from each other, which is to say, the nebulization assembly 30 and the liquid storage apparatus 20 are in the second assembly state.

Optionally, controlling the nebulization assembly 30 and a mounting base 201 to rotate from a first relative position to a second relative position in Step 1022 comprises:
Step 1022a: fitting a first snap-fitting part 302 on the nebulization assembly 30 with a second snap-fitting part 202 on the mounting base 201 by snap-fitting, so that the nebulization assembly 30 and the mounting base 201 are in the first relative position; and
causing a snap-fitting function of the first snap-fitting part 302 and the second snap-fitting part 202 to fail when the nebulization assembly 30 and the mounting base 201 are controlled to rotate to the second relative position.

Specifically, as shown in Fig. 5 and Fig. 6, the nebulization assembly 30 and the liquid storage apparatus 20 can be assembled by snap-fitting. The nebulization assembly 30 is provided with a first snap-fitting part 302 on a side of the nebulization assembly 30, and the mounting base 201 is provided with a second snap-fitting part 202 on an inner sidewall of the mounting base 201. The first snap-fitting parts 302 are in the same quantity as the second snap-fitting parts 202, and they are in one-to-one correspondence.

The nebulization assembly 30 is at least partially embedded in the mounting base 201, and the side surface of the nebulization assembly 30 is in contact with the inner sidewall of the mounting base 201. When the nebulization assembly 30 and the mounting base 201 are in the first relative position, the first snap-fitting part 302 and the second snap-fitting part 202 are snap-fitted together, thereby the nebulization assembly 30 and the liquid storage apparatus 20 are stably assembled. When the nebulization assembly 30 and the mounting base 201 are rotated to the second relative position, the first snap-fitting part 302 and the second snap-fitting part 202 are also driven to rotate relatively and are in a dislocated and separated state, and the snap-fitting function of the first snap-fitting part 302 and the second snap-fitting part 202 fails, so that the nebulization assembly 30 and the mounting base 201 can be separated from each other.

In another embodiment provided by the present disclosure, when the nebulization assembly 30 and the mounting base 201 are rotated from the first relative position to the second relative position, the rotation range can be limited by means of a third limiting structure 401.

Specifically, the third limiting structure 401 is a clearance slide channel arranged at the bottom of the mounting base 202, the first limiting structure 301 is arranged in the clearance slide channel, and the two ends of the clearance slide channel are respectively a locking end and an unlocking end, wherein the locking end corresponds to the first relative position and the unlocking end corresponds to the second relative position. The length of the clearance slide channel defines the range of rotation between the nebulization assembly 30 and the mounting base 201.

For example, in the case that the first limiting structure 301 is a limiting rod, the third limiting structure 401 is an arc-shaped clearance slide channel, and the limiting rod is inserted in the clearance slide channel. At the first relative position, i.e., the nebulization assembly 30 and the liquid storage apparatus 20 are fixed in relation to each other, the first limiting structure 301 extends through the locking end of the clearance slide channel; when the nebulization assembly 30 is rotated in the mounting base 202, the limiting rod will slide relatively along the clearance slide channel till it reaches the second relative position; at that point, the limiting rod is at the unlocking end of the clearance slide channel.

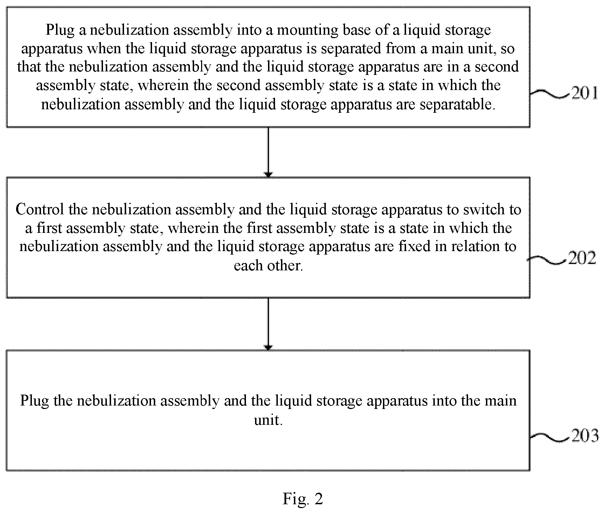

In a second aspect, as shown in Fig. 2, an embodiment of the present disclosure provides a method for mounting a nebulization assembly 30, which comprises:
Step 201: plugging a nebulization assembly 30 into a mounting base 201 of a liquid storage apparatus 20 when the liquid storage apparatus 20 is separated from a main unit 10, so that the nebulization assembly 30 and the liquid storage apparatus 20 are in a second assembly state, wherein the second assembly state is a state in which the nebulization assembly 30 and the liquid storage apparatus 20 are separatable.

Specifically, the liquid storage apparatus 20 is provided with the mounting base 201; when the liquid storage apparatus 20 is separated from the main unit 10, the nebulization assembly 30 is plugged into the mounting base 201 of the liquid storage apparatus 20 first to accomplish preliminary assembly; at that point, the nebulization assembly 30 and the liquid storage apparatus 20 are in a second assembly state. Mounting the nebulization assembly 30 when the liquid storage apparatus 20 is separated from the main unit 10 can avoid a problem that the main unit 10 is short-circuited due to the invasion of the liquid medicine into the main unit 10.

Step 202: controlling the nebulization assembly 30 and the liquid storage apparatus 20 to switch to a first assembly state, wherein the first assembly state is a state in which the nebulization assembly 30 and the liquid storage apparatus 20 are fixed in relation to each other.

Specifically, in order to avoid accidental separation between the nebulization assembly 30 and the liquid storage apparatus 20 due to misoperations, the nebulization assembly 30 is plugged into the mounting base 201 of the liquid storage apparatus 20 first, so that the nebulization assembly 30 and the liquid storage apparatus 20 are in the second assembly state; and then the nebulization assembly 30 and the liquid storage apparatus 20 can be switched to the first assembly state, for example, by rotating the nebulization assembly 30 and the locking element 13. The nebulization assembly 30 and the liquid storage apparatus 20 in the first assembly state greatly reduce the risk of liquid leakage.

Step 203: plugging the entire assembly formed by the nebulization assembly 30 and the liquid storage apparatus 20 into the main unit 10.

Plugging the entire assembly formed by the nebulization assembly 30 and the liquid storage apparatus 20 in the first assembly state into the main unit 10 can avoid liquid medicine leakage, so as to avoid problem that the main unit 10 is short-circuited caused by the invasion of the liquid medicine into the main unit 10.

In a third aspect, as shown in Fig. 3 to Fig. 7, an embodiment of the present disclosure provides a nebulization therapeutic device, which comprises: a main unit 10, a liquid storage apparatus 20 and a nebulization assembly 30, wherein the liquid storage apparatus 20 is detachably mounted to the main unit 10 in a plug-in manner; the nebulization assembly 30 is connected to the liquid storage apparatus 20; the liquid storage apparatus 20 and the nebulization assembly 30 are fixed in relation to each other when an entire assembly formed by the liquid storage apparatus 20 and the nebulization assembly 30 is assembled to the main unit 10; and the liquid storage apparatus 20 and the nebulization assembly 30 are dismountable when the entire assembly formed by the liquid storage apparatus 20 and the nebulization assembly 30 is separated from the main unit 10.

Specifically, the nebulization therapeutic device comprises a main unit 10, a liquid storage apparatus 20 and a nebulization assembly 30. The liquid storage apparatus 20 is detachably mounted to the main unit 10, and the nebulization assembly 30 is detachably mounted to the liquid storage apparatus 20. The liquid storage apparatus 20 is used to store a liquid medicine to be nebulized, a side of the liquid storage apparatus 20 is provided with a liquid outlet, and the nebulization assembly 30 and the liquid storage apparatus 20 are assembled together in a detachable manner; in addition, the liquid inlet of the nebulization assembly 30 is in communication with the liquid outlet of the liquid storage apparatus 20, so that the liquid storage apparatus 20 can deliver the liquid medicine to the nebulization assembly 30. In order to deliver the liquid medicine and reduce the residual liquid in the liquid storage apparatus 20 better, the bottom surface of the liquid storage apparatus 20 may be configured as an inclined surface inclined to the side of the nebulization assembly 30. Besides, the inner wall and/or bottom surface of the liquid storage apparatus 20 may be configure as rough surfaces to reduce the generation of bubbles in the liquid medicine.

The main unit 10 comprises a housing, and a battery and a circuit board arranged in the housing, etc. The main unit 10 is further provided with electrode contacts for supplying power to the nebulization assembly 30, and a user can control the functions of the device, such as start and stop, by performing corresponding operations on the main unit 10; besides, the main unit 10 can further provide a holding space for the user, so that the user can use the device conveniently.

The main unit 10 is provided with a connecting structure at the top of the main unit 10, and the liquid storage apparatus 20 is detachably mounted to the main unit 10 in a plug-in manner via the connecting structure, so that the user can mount and dismount it conveniently, thereby the flexibility of use of the device is improved. The liquid storage apparatus 20 can be assembled to the main unit 10 by snap-fitting or bolting, etc. When the liquid storage apparatus 20 is assembled to the main unit 10, the liquid storage apparatus 20 will not be displaced or rotated in relation to the main unit 10. The nebulization assembly 30 and the liquid storage apparatus 20 may be assembled together by snap-fitting or threaded connection, etc.

When the liquid storage apparatus 20 is assembled to the main unit 10, i.e., when the liquid storage apparatus 20 has been plugged into the main unit 10, there will be no relative displacement or rotation between the nebulization assembly 30 and the main unit 10, and the nebulization assembly 30 and the liquid storage apparatus 20 will be fixed in relation to each other because the liquid storage apparatus 20 will not be displaced or rotated in relation to the main unit 10; thus, the problem of liquid leakage caused by relative rotation of the nebulization assembly 30 and the liquid storage apparatus 20 owing to misoperations during the use of the device is avoided, and the risk of a short circuit between the probes on the main unit 10 is reduced.

When the entire assembly formed by the liquid storage apparatus 20 and the nebulization assembly 30 is separated from the main unit 10, the liquid medicine in the liquid storage apparatus 20 is separated from the main unit 10; thus, the risk that the liquid medicine invades into the main unit 10 is eliminated; at that point, the liquid storage apparatus 20 and the nebulization assembly 30 are separatable.

In a fourth aspect, an embodiment of the present disclosure provides a nebulization therapeutic device, which comprises a nebulization assembly 30, wherein the nebulization assembly 30 can be dismounted with the above-mentioned dismounting method.

In the embodiment of the present disclosure, with the above dismounting method for the nebulization assembly of the nebulization therapeutic device, when performing dismounting of the nebulization assembly 30, an entire assembly formed by the nebulization assembly 30 and a liquid storage apparatus 20 may be removed from a main unit 10 when the nebulization assembly 30 and the liquid storage apparatus 20 are in the first assembly state; because the nebulization assembly 30 and the liquid storage apparatus 20 are fixed in relation to each another in the first assembly state, a problem of liquid medicine leakage is prevented, and a risk of a short circuit between the probes of the main unit 10 is reduced. After the entire assembly formed by the nebulization assembly 30 and the liquid storage apparatus 20 is removed from the main unit 10, the nebulization assembly 30 and the liquid storage apparatus 20 are switched to a second assembly state; at that point, the nebulization assembly 30 and the liquid storage apparatus 20 are in a separable state, and the nebulization assembly 30 may be smoothly taken off of the liquid storage apparatus 20; even if liquid medicine leakage occurs in this process, the main unit 10 is unaffected, thereby the safety of use is greatly improved.

Presently, aerosol inhalation therapy has become an important means to treat human respiratory diseases. Existing nebulizers are mainly categorized into mesh-type nebulizers, compressed air nebulizers and ultrasonic nebulizers. A mesh-type nebulizer nebulizes the liquid medicine passing through fine pores of a nebulization plate into nebulized particles by means of the high-frequency vibration of the nebulization plate, then the nebulized particles are inhaled into a human body through an inhalation device (a mask or mouthpiece). In the prior art, once the nebulization plate fails, the entire mesh-type nebulizer has to be replaced or the nebulization plate has to be replaced by a specialized person. However, it is necessary to dismount the nebulizer to replace the nebulizer. The nebulizer dismounting operation will drive the nebulizer to sway in the dismounting process, resulting in leakage of the liquid medicine in the nebulizer.

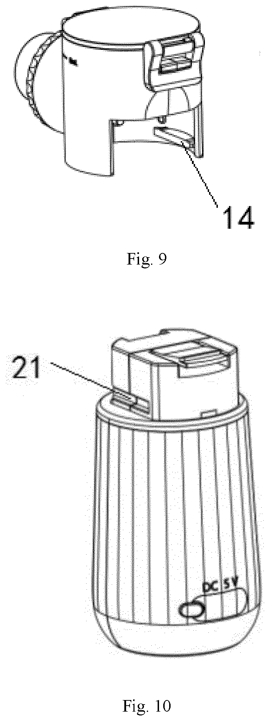

In order to solve the problem that the defective connection between the nebulization device and the medicine cup device in the prior art affects the sealing performance of the medicine cup assembly, the present disclosure provides a medicine cup assembly and a mesh-type nebulizer. The medicine cup assembly and the mesh-type nebulizer provided in the embodiments of the present disclosure will be described below in detail in specific embodiments and application scenarios with reference to Figs. 8-17.

As shown in Fig. 8 to Fig. 17, an embodiment of the present disclosure provides a medicine cup assembly 31, which comprises a nebulization device 11, a medicine cup device 12 and a locking element 13, wherein the nebulization device 11 and the medicine cup device 12 are detachably connected together, and the nebulization device 11 is at least partially positioned inside the medicine cup device 12;
the locking element 13 is detachably connected to the medicine cup device 12 by rotation, and the nebulization device 11 is positioned between the locking element 13 and the medicine cup device 12; when the locking element 13 is connected to the medicine cup device 12, the locking element 13 is at least partially positioned inside the medicine cup device 12, and the locking element 13 is used for locking the nebulization device 11 to the medicine cup device 12.

In the embodiment of the present disclosure, the medicine cup device 12 is configured for storing a liquid medicine, the nebulization device 11 is configured for nebulizing the liquid medicine in the medicine cup into nebulized particles, and the nebulization device 11 is further used for connecting an external inhalation device, so that a user can inhale the nebulized particles through the inhalation device. The nebulization device 11 is at least partially positioned inside the medicine cup device 12, and the nebulization device 11 is detachably connected to the medicine cup device 12; when the locking element 13 is connected to the medicine cup device 12, the nebulization device 11 is positioned between the locking element and the medicine cup device 12; when the nebulization device 11 doesn't take effect anymore, the user may separate the locking element 13 from the medicine cup device 12, so that the nebulization device 11 can be smoothly pulled out of the medicine cup device 12, and then a new nebulization device 11 can be smoothly plugged into the medicine cup device 12. The locking element 13 is configured to lock the nebulization device 11 to the medicine cup device 12, and the locking element 13 is at least partially positioned inside the medicine cup device 12; when the nebulization device 11 is connected to the medicine cup device 12, the locking element 13 is detachably connected to the medicine cup device 12 by rotation; under the locking action of the locking element 13, the nebulization device 11 will not rotate or get loose unless it is dismounted intentionally, thereby the sealing performance of the connection between the nebulization device 11 and the medicine cup device 12 is enhanced, and a beneficial effect of preventing the liquid medicine in the medicine cup device 12 from leaking due to poor sealing performance is achieved.

It may be noted that, in addition, the plug-in mounting/dismounting of the nebulization device 11 to/from the medicine cup device 12 improves the convenience and stability in replacement of the nebulization device 11, and the locking element 13 is connected to the medicine cup device 12 by rotation, so that the locking element 13 can be quickly and conveniently unscrewed from the medicine cup device 12, thereby the operability of replacement of the nebulization device 11 by the user is improved.

Optionally, in an embodiment of the present disclosure, the locking element 13 and the nebulization assembly 11 are integrally arranged, and the nebulization device 11 abuts against the medicine cup device 10 when the locking element 13 is connected to the medicine cup device 10.

In the embodiment of the present disclosure, the locking element 13 is configured to lock the nebulization device 11 to the medicine cup device 12; the locking element 13 is fitted around the periphery of the nebulization device 11 and at least partially positioned in the medicine cup device 12; when the nebulization device 11 is connected to the medicine cup device 12, the locking element 13 is detachably connected to the medicine cup device 12 by rotation; under the locking action of the locking element 13, the nebulization device 11 abuts against the medicine cup device 10; thus, the nebulization device 11 will not rotate or get loose unless it is dismounted intentionally; and the integrally formed locking element 13 and nebulization device 11 further enhance the sealing between the locking element 13 and nebulization device 11, achieving a beneficial effect of preventing the liquid medicine from leaking owing to poor sealing performance between the locking element 13 and nebulization device 11 during use.

Optionally, in an embodiment of the present disclosure, the locking element 13 and the nebulization device 11 are separate structures, and the locking element 13 is fitted around the periphery of the nebulization device 11.

In the embodiment of the present disclosure, the locking element 13 and the nebulization device 11 are two separate parts. When the locking element 13 and the nebulization device 11 are separated from the medicine cup device 10 respectively, the locking element 13 and the nebulization device 11 are also separated from each other. The locking element 13 is configured to lock the nebulization device 11 to the medicine cup device 12, and the locking element 13 is fitted around the periphery of the nebulization device 11 and at least partially positioned inside the medicine cup device 12; when the nebulization device 11 is connected to the medicine cup device 12, the locking element 13 is detachably connected to the medicine cup device 12 by rotation; under the locking action of the locking element 13, the nebulization device 11 will not rotate or get loose unless it is dismounted intentionally, thereby the sealing performance of the connection between the nebulization device 11 and the medicine cup device 12 is enhanced, and a beneficial effect of preventing the liquid medicine in the medicine cup device 12 from leaking due to poor sealing performance is achieved.

It may be noted that when either one of the locking element 13 and the nebulization device 11 separated from each other is injured or damaged, the injured or damaged part can be replaced with a replacement part, and it is unnecessary to replace the locking element 13 and the nebulization device 13 together; thus, the ongoing replacement cost of the locking element 13 and the nebulization device 11 can be reduced.

Optionally, in an embodiment of the present disclosure, the locking element 13 is of an annular structure, the locking element 13 is provided with a raised rib 131 on a side connected to the medicine cup device 12, and the medicine cup device 12 is provided with a second slide channel 400 at a position where the medicine cup device 12 is connected to the locking element 13; the rib 131 is fitted with the second slide channel 400 so that the medicine cup device 12 is rotatably connected to the locking element 13.

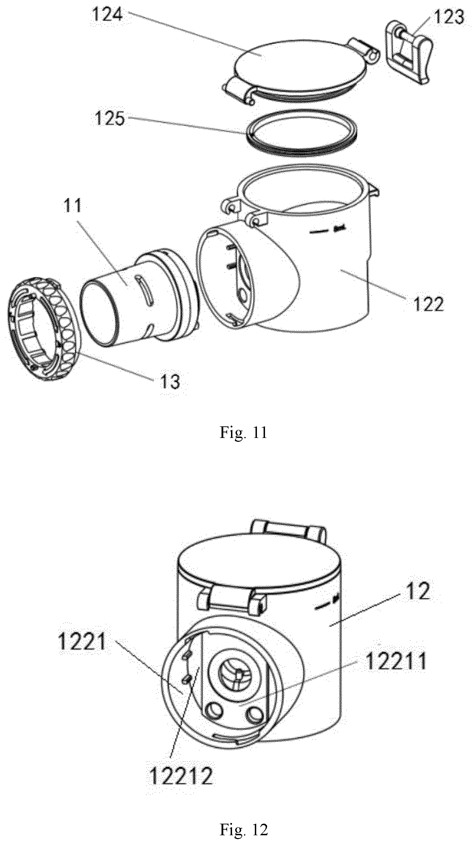

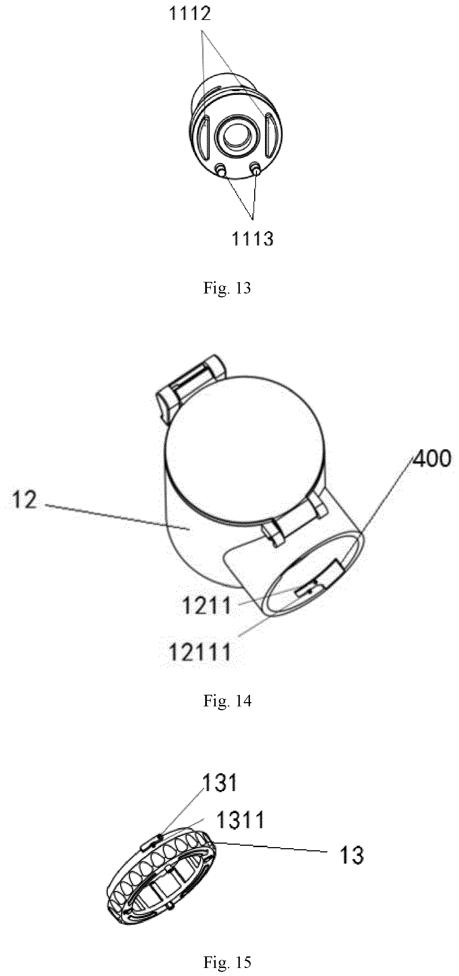

In the embodiment of the present disclosure, as shown in Fig. 11 and Fig. 15, the locking element 13 having an annular structure is fitted around the periphery of the medicine cup device 11, and the locking element 13 is provided with a raised rib 131 on a side connected to the medicine cup device 12; the medicine cup device 12 is provided with a second slide channel 400 at a position where the medicine cup device 12 is connected to the locking element 13; the raised rib 131 and the second slide channel 400 are fitted with each other to realize a connection between the locking element 13 and the medicine cup device 12. In the process that the locking element 13 is connected to the medicine cup device 12, the raised rib 131 enters the second slide channel 400 and the connection between the locking element 13 and the medicine cup device 12 can be realized by rotating in the second slide channel 400. In the process that the locking element 13 is separated from the medicine cup device 12, the raised rib 131 rotates in the second slide channel 400 and is disengaged from the second slide channel 400, thus the locking element 13 is separated from the medicine cup device 12. In the embodiment of the present disclosure, the rotatable connection between the locking element 13 and the medicine cup device 12 is realized by means of the fitting between the raised rib 131 and the second slide channel 400. Since the locking element 13 is fitted around the periphery of the nebulization device 11, the rotatable connection between the locking element 13 and the medicine cup device 12 has beneficial effects of preventing the nebulization device 11 from rotating unless it is dismounted intentionally and enhancing the tightness of the connection between the nebulization device 11 and the medicine cup device 12. In the dismounting process, the rotatably connected locking element 13 can be rotated out of the medicine cup device 12 quickly and conveniently, achieving a beneficial effect of improving the operability of the replacement of the nebulization device 11.

It may be noted that the raised rib 131 may be arranged on the locking element 13 or on the medicine cup device 12, and accordingly, the second slide channel 400 may be arranged on the medicine cup device 12 or on the locking element 13. There is no particular restriction on the specific arrangement in this embodiment.

It may be further noted that the side surfaces of the locking element 13 are provided with concave or anti-slip patterns to facilitate the user to apply force in the rotating operation and prevent slippage during force application.

Optionally, in an embodiment of the present disclosure, the second slide channel 400 comprises a limiting groove 1211, which matches the raised rib 131 and is used to limit the position of the raised rib 131.

In the embodiment of the present disclosure, as shown in Fig. 14 and Fig. 15, the limiting groove 1211 matches the raised rib 131, and is configured to realize the connection between the raised rib 131 and the second slide channel 400 after the raised rib 131 enters the second slide channel 400 and is rotated; the limiting groove 1211 attains an effect of limiting the position of the raised rib 131 after the raised rib 131 enters the second slide channel 400 unless it is rotated intentionally. This embodiment of the present disclosure achieves a beneficial effect of preventing the raised rib 131 from being disengaged from the second slide channel 400, thereby preventing the locking element 13 from being disengaged from the medicine cup device 12 unless it is rotated intentionally.

Optionally, in an embodiment of the present disclosure, either of the raised rib 131 and the limiting groove 1211 is provided with a first recess 1311, while the other of the raised rib 131 and the limiting groove 1211 is provided with a first bump 12111, and the first recess 1311 is snap-fitted with the first bump 12111.

In the embodiment of the present disclosure, the first recess 1311 and the first convex point 12111 match each other, and the first recess 1311 and the first convex point 12111 are configured to realize snap-fitting between the raised rib 131 and the second slide channel 400; when the raised rib 131 is connected to the second slide channel 400, the raised rib 131 is moved along the second slide channel 400; when the raised rib 131 enters the limiting groove 1211 and is rotated to a preset position, the first bump 12111 and the first recess 1311 are snap-fitted with each other, and the locking element 13 and the medicine cup device 12 are locked together; at that point, the nebulization device 11 is locked to the medicine cup device 12 by means of the locking element 13, and the nebulization device will not rotate or get loose since the first recess 1311 and the first bump 12111 are snap-fitted together. Thus, this embodiment of the present disclosure achieves a beneficial effect of enhancing the tightness of the connection between the nebulization device 11 and the medicine cup device 12.

It may be noted that the preset position may be determined according to the specific situation in the actual application, and there is no particular restriction on it in this embodiment.

It may be further noted that the first recess 1311 may be arranged in the raised rib 131 or in the limiting groove 1211, and accordingly, the first bump 12111 may be arranged on the limiting groove 1211 or on the raised rib 131.

Optionally, in an embodiment of the present disclosure, the medicine cup device 12 comprises a medicine cup body 122, and one side of the medicine cup body 122 is provided with an accommodating cavity 1221 for at least partially accommodating the nebulization device 11.

In the embodiment of the present disclosure, the medicine cup body 122 is configured to store the liquid medicine, and the accommodating cavity 1221 is configured to at least partially accommodate the nebulization device 11, so as to realize the connection between the nebulization device 11 and the medicine cup device 12. Thus, this embodiment achieves a beneficial effect of enhancing the sealing performance of the connection between the accommodating cavity 1221 and the nebulization device 11.

Optionally, in an embodiment of the present disclosure, a boss 12211 is arranged in the accommodating cavity 1221, and the sidewall of the boss 12211 and the inner wall of the accommodating cavity 1221 enclose to form a second recess 12212.

In the embodiment of the present disclosure, the sidewall of the boss 12211 and the inner wall of the accommodating cavity 1221 enclose to form a second recess 12212, which is configured to be connected to the nebulization device 11, and the second recess 12212 arranged in the medicine cup device 12 can be snap-fitted with the nebulization device 11 so that the nebulization device 11 and the medicine cup device 12 can be detachably connected in a plug-in manner, and the plug-in dismounting and mounting improve the convenience and stability in replacement of the nebulization device 11, improve the operability of replacement of the nebulization device 11, and achieve a beneficial effect of preventing leakage of the liquid medicine in the dismounting and mounting process.

Optionally, in an embodiment of the present disclosure, the medicine cup device further comprises a medicine cup cap fastener 123 and a medicine cup cap 124, and the medicine cup cap fastener 123 is movably connected to the medicine cup cap 124 and the medicine cup body 122 respectively.

In the embodiment of the present disclosure, as shown in Fig. 11, the medicine cup cap fastener 123 is configured to connect the medicine cup cap 124 with the medicine cup body 122; the medicine cup cap 124 is configured to close a non-nebulization outlet of the medicine cup body 122; the medicine cup cap 124 is mounted on the medicine cup and can be turned over, and the medicine cup cap 124 is press-fitted with the medicine cup body 122 via the medicine cup cap fastener 123; the medicine cup body 122 is configured to store the liquid medicine and be connected to the nebulization device 11 and the locking element 13, achieving beneficial effects of preventing the moisture or dust in the air from invading into the medicine cup device and preventing the liquid medicine in the medicine cup body 122 from spilling.

It may be further noted that an air inlet is arranged in the wall of the medicine cup cap 124 in order to balance the air pressure inside and outside the medicine cup assembly 31 and facilitate the nebulization of the liquid medicine.

Optionally, in an embodiment of the present disclosure, the medicine cup device further comprises a first sealing element 125, which is arranged between the medicine cup cap 124 and the medicine cup body 122.

In the embodiment of the present disclosure, the medicine cup body 122 is configured to store the liquid medicine and be connected to the nebulization device 11 and the locking element 13, and the first sealing element 125 is arranged between the medicine cup cap 124 and the medicine cup body 122, and is configured to seal the medicine cup body 122 and achieve beneficial effects of further preventing the moisture or dust in the air from invading into the medicine cup device, enhancing the sealing between the medicine cup cap 124 and the medicine cup body 122, and preventing the liquid medicine in the medicine cup body 122 from spilling.

It may be noted that the first sealing element 125 may be a seal ring, or may be a sealing gasket arranged along the edge of the medicine cup cap 124; the first sealing element 125 may be made of Buna-N rubber, polyurethane or silicone. There is no particular restriction on the specific material of the first sealing element 125 in this embodiment.

Optionally, in an embodiment of the present disclosure, the nebulization device 11 comprises a first housing 111, a second sealing element 1110, a nebulization plate 113 and a second housing 114 fixedly connected to the first housing 111, which are arranged sequentially, wherein the first housing 111 is detachably connected to the medicine cup device 12, and the second sealing element 1110 and the nebulization plate 113 are sandwiched between the first housing 111 and the second housing 114.

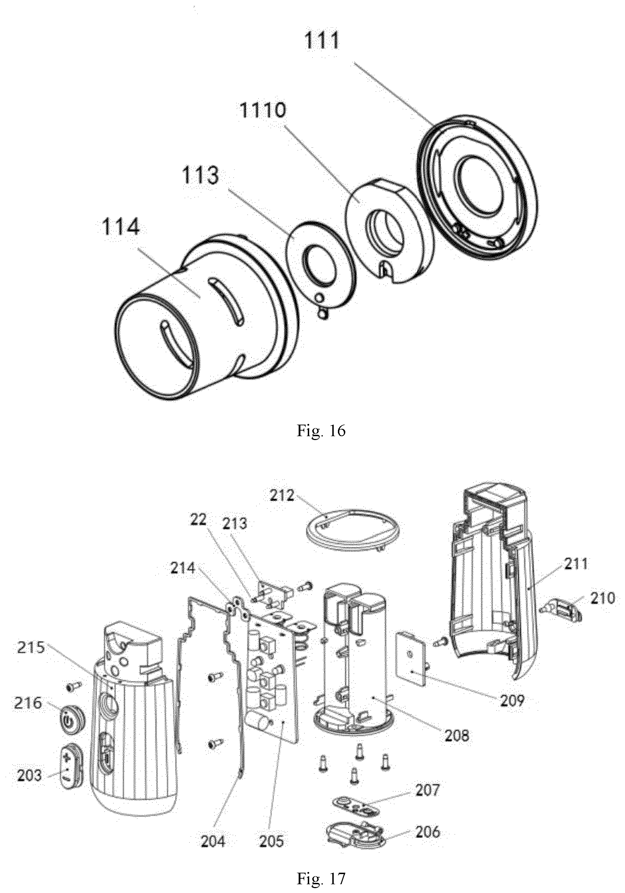

In the embodiment of the present disclosure, as shown in Fig. 16, the first housing 111 and the second housing 114 are configured to form a space for accommodating the nebulization plate 113 and the second sealing element 1110, the nebulization plate 113 is configured to nebulize the liquid medicine, the second sealing element 1110 is wrapped around the nebulization plate 113, and the first housing 111 and the second housing 114 are welded together through an ultrasonic welding process and squeeze the second sealing element 1110 therein to achieve a sealing effect. In the embodiment of the present disclosure, a second sealing element 1110 is provided and the first housing 111 and the second housing 114 squeeze the sealing element to seal the nebulization plate 113, thereby a beneficial effect of preventing the moisture or dust in the air from invading into the nebulization device 11 and preventing the nebulization plate 113 from being polluted is achieved.

It may be noted that the first housing 111 and the second housing 114 may be fixedly connected together by means of screws, welding or adhesive. There is no particular restriction on the specific connection method in this embodiment.

It may also be noted that the outer diameter of the second sealing element 1110 is greater than or equal to the outer diameter of the nebulization plate 113.

Optionally, in an embodiment of the present disclosure, the first housing 111 is provided with a protrusion 1112, which matches the second recess 12212; the protrusion 1112 is used to fit with the second recess 12212 to realize a detachable connection between the medicine cup device 12 and the nebulization device 11 in a plug-in manner; and
the first housing 111 is further provided with metal contacts 1113, which are used for receiving electric energy and electrical signals.

In the embodiment of the present disclosure, as shown in Fig. 12 and Fig. 13, the protrusion 1112 matches the second recess 12212, and the protrusion 1112 is configured to be fitted with the second recess 12212 to realize the connection between the medicine cup device 12 and the nebulization device 11. During the dismounting and mounting of the medicine cup device 12 and the nebulization device 11, the connection between the medicine cup device 12 and the nebulization device 11 can be achieved in a plug-in manner by means of the fitting between the protrusion 1112 and the second recess 12212. The plug-in dismounting and mounting improves the convenience and stability in the replacement of the nebulization device 11, improves the operability of replacement of the nebulization device 11, and achieves a beneficial effect of preventing the liquid medicine from spilling during dismounting and mounting. The metal contacts 1113 are configured to receive electric energy and electrical signals, to enable the nebulization plate 113 to vibrate at high frequencies, so as to nebulize the liquid medicine in the medicine cup.

It may be noted that the front end of the first housing 111 is an aerosol nozzle, to which an inhalation device can be mounted. When the inhalation device is mounted or removed by rotation, the protrusion 1112 provided on the first housing 111 achieves a beneficial effect of preventing the nebulization device from rotating.

Optionally, an embodiment of the present disclosure provides a mesh-type nebulizer, which comprises the medicine cup assembly 31 described above; the mesh-type nebulizer further comprises a main unit assembly 19, the medicine cup assembly 31 is provided with a first sliding connection structure 14, and the main unit assembly 19 is provided with a second sliding connection structure 21; the first sliding connection structure 14 and the second sliding connection structure 21 are fitted together for detachably connecting the medicine cup assembly 31 to the main unit assembly 19.

In the embodiment of the present disclosure, the mesh-type nebulizer comprises the medicine cup assembly 31 described above and a main unit assembly 19; the main unit assembly 19 and the medicine cup assembly 31 are connected together via a connecting structure; the first sliding connecting structure 14 is arranged on the medicine cup assembly 31, and the second sliding connecting structure 21 is arranged on the main unit assembly 19; the first sliding connecting structure 14 and the second sliding connecting structure 21 are fitted together to realize a detachable connection between the medicine cup assembly 31 and the main unit assembly 19.

It may be noted that two sides of the lower end of the medicine cup assembly 31 are respectively provided with sliding ribs, which are fitted with slide channels provided on two sides of the upper end of the main unit assembly 19; after the medicine cup assembly 31 slides backward into the main unit assembly 19, an elastic arm at the top end of the main unit assembly 19 catches the medicine cup assembly 31, thus realizing the connection between the medicine cup assembly 31 and the main unit assembly 19.

Optionally, in an embodiment of the present disclosure, the main unit assembly 19 is provided with metal probes 22, and the metal probes 22 abut against the metal contacts 1113 to realize an electrical connection between the main unit assembly 19 and the medicine cup assembly 31, and the main unit assembly 19 is configured to supply electric energy and transmit electrical signals to the medicine cup assembly 31.

In the embodiment of the present disclosure, the metal probes 22 are configured to be in contact with the metal contacts 1113; when the medicine cup assembly 31 is connected to the main unit assembly 19, the metal probes 22 abut against the metal contacts 1113 to realize the power supply and control of the medicine cup assembly 31 by the main unit assembly 19.

It may be noted that, as shown in Fig. 17, the main unit assembly 19 further comprises: a main unit front housing 215, a switch button 216, an aerosol yield adjustment button 203, a main unit front and rear housings seal ring 204, a main PCB 205, a battery compartment cover 206, battery contact plates 207, a battery compartment 208, a power supply interface board 209, a power supply interface protection cover 210, a main unit rear housing 211, a decorative ring 212, a probe connection board 213 and a probe sealant pad 214, wherein the switch button 216 and the aerosol yield adjustment button 203 are mounted in corresponding holes in the main unit front housing 215; the probe connecting plate 213 is fixed to the main unit front housing 215by screws, and the probe sealant pad 214 is arranged between them for sealing; the probe connecting plate 213 is provided with metal probes 22, and the metal probes 22 abuts against the metal contacts 1113 to realize the power supply and control of the medicine cup assembly 31 by the main unit assembly 19; the main PCB 205 and the power supply interface board 209 are fixed to the battery compartment 208 by screws, the battery contact plates 207 are thermally fused on the battery compartment cover 206, and the battery compartment cover 206 is mounted at the bottom of the battery compartment 208 and can be turned over; the power supply interface protection cover 210 is arranged on the main unit rear housing 211; the main unit front housing 215 and the main unit rear housing 211are fitted by means of snap-fitting clips and locked by means of a screw, and the main unit front and rear housings seal ring 204 is arranged between the main unit front housing 215 and the main unit rear housing 211 for sealing; the battery compartment 208 is locked to the main unit front housing 215 and the main unit rear housing 211 by screws at the bottom; the decorative ring 212 is fitted with the main unit front housing 215 and the main unit rear housing 211 by means of snap-fitting clips.

In the embodiment of the present disclosure, the medicine cup assembly 31 as described above is connected to the main unit assembly 19; when the medicine cup assembly 31 is connected to the main unit assembly 19, the nebulization device 11 and the medicine cup device 12 that are detachably connected in a plug-in manner and the locking element 13 that locks the nebulization device 11 to the medicine cup device 12 prevent the nebulization device 11 from rotating with an inhalation device when the user mounts the inhalation device by rotation; besides, the metal contacts 1113 arranged on the nebulization device 11 are always in contact with the metal probes 22 on the main unit assembly 19, so that beneficial effects of ensuring the electrical connection between the medicine cup assembly 31 and the main unit assembly 19 and reducing the risk of damage of the metal probes 22 are achieved.

The nebulization technology is widely applied in daily life, such as humidifiers, aromatherapy devices and nebulization therapeutic devices, etc. Such devices can utilize a nebulization plate to nebulize the liquid contained in a container, so as to realize its nebulization function. During the operation of the nebulization plate, when the liquid is depleted, the nebulization plate will continue vibrating without any liquid, resulting in damages to the nebulization plate. Therefore, it is necessary to provide a liquid level detection device to prevent the nebulization plate from vibrating without any liquid. At present, the existing liquid level detection device is usually mounted in a container containing liquid, and the liquid in the container is electrically communicated with the liquid level detection device; consequently, the liquid in the container become an accessible charged body, and may bring a risk of electric shock in some special circumstances.

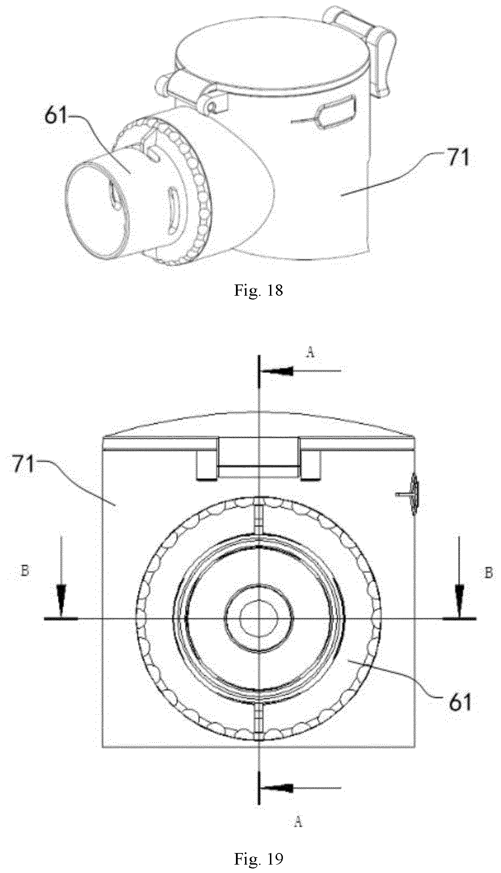

In order to solve the problem that the liquid stored in the nebulization device becomes an accessible charged body and brings an electric shock risk in some special circumstances because the liquid is electrically communicated with the liquid level detection device in the prior art, the present disclosure provides an assembly having a nebulization function and a nebulization device. The assembly having a nebulization function and the nebulization device provided in the embodiments of the present disclosure will be described below in detail in specific embodiments and application scenarios with reference to Figs. 18-24.

As shown in Fig. 18 to Fig. 21, an embodiment of the present disclosure provides an assembly having a nebulization function, which comprises: a housing 71, a nebulization module 61 and a detection electrode 51, wherein the housing 71 is provided with a liquid storage cavity 110 and a liquid outlet 112 in communication with the liquid storage cavity 110; the nebulization module 61 is detachably connected to the housing 71, and the nebulization module 61 is in communication with the liquid outlet 112; the detection electrode 51 is arranged on an outer wall of the housing 71 at the bottom of the housing 71, and the outer wall of the housing 71 separates the detection electrode 51 from the liquid storage cavity 110; and the detection electrode 51 is used for detecting whether the liquid in the liquid storage cavity 110 is in shortage.

Specifically, as shown in Fig. 18 to Fig. 21, a liquid storage cavity 110 is arranged in the housing 71, and the liquid storage cavity 110 is used for storing a liquid to be nebulized; the liquid in the liquid storage cavity 110 may be water, an aromatherapy liquid, or a liquid medicine, etc., depending on the specific device and the application requirements in the specific scenario. The housing 71 may be made of plastics or organic glass, etc. The housing 71 is further provided with a liquid outlet 112 in communication with the liquid storage cavity 110, and the nebulization module 61 is in communication with the liquid outlet 112, so that the liquid led out from the liquid outlet 112 can be nebulized. The nebulization module 61 is further hermetically connected to the housing 71 around the liquid outlet 112 to seal the periphery of the liquid outlet 112, so that the liquid in the liquid storage cavity 110 can only flow into the nebulization module 61 through the liquid outlet 112, and liquid waste caused by liquid leakage can be avoided. The nebulization module 61 is detachably connected to the housing 71; specifically, it may be assembled by snap-fitting or threaded connection, etc. With the detachable connection, the nebulization module 61 can be mounted to and dismounted from the housing 71 conveniently.

The detection electrode 51 is arranged on the outer wall of the housing 71 at the bottom of the housing 71, and the liquid storage cavity 110 and the detection electrode 51 are separated by the housing 71 and separate from each other. In the case that there is no liquid between the detection electrode 51 and the nebulization module 61 or the amount of liquid is very small, the sample value of the detection electrode 51 (capacitance value around the detection electrode 51) will change, and whether the liquid storage cavity 110 is in a liquid shortage state can be judged according to the sample value, then measures can be taken, for example, cutting off the power supply to the nebulization module 61. When judging whether the liquid storage cavity 110 is in a liquid shortage state, the actual state of the liquid in the liquid storage cavity 110 can be quickly ascertained according to a corresponding relationship between the sample value of the detection electrode 51 and the remaining liquid in the liquid storage cavity 110.

The detection electrode 51 may be a metal piece, or may be a combination of metal and any connecting device that can feed back the sample value, for example, a combination of metal and spring. The detection electrode 51 may be in a cylindrical shape, or a cubic shape, a prism shape or other shape. There is no particular restriction on the specific shape of the detection electrode 51 in the present disclosure.

In the embodiment of the present disclosure, the assembly having a nebulization function comprises: a housing 71, a nebulization module 61 and a detection electrode 51, wherein the housing 71 is provided with a liquid storage cavity 110 and a liquid outlet 112 in communication with the liquid storage cavity 110; the nebulization module 61 is detachably connected to the housing 71 and is in communication with the liquid outlet 112; the detection electrode 51 is arranged on an outer wall of the housing 71 at the bottom of the housing 71, and the outer wall of the housing 71 separates the detection electrode 51 from the liquid storage cavity 110; the detection electrode 51 is used for detecting whether the liquid in the liquid storage cavity 110 is in shortage. By arranging the detection electrode 51 on the outer wall of the housing 71, a liquid shortage detection function can also be realized under a condition that the detection electrode 51 is not in direct contact with the liquid in the liquid storage cavity 110, so that the liquid is prevented from becoming an accessible charged body, a risk of electric shock is reduced, and the safety of use is improved; moreover, since the detection electrode 51 is not in direct contact with the liquid in the liquid storage cavity 110, pollution to the liquid can be avoided.

Optionally, the assembly further comprises: a detection circuit and a control module, wherein the detection circuit is electrically connected to the detection electrode 51 and the control module respectively, and is used for acquiring a potential signal of the detection electrode 51 and transmitting the potential signal to the control module; and the control module is used for judging whether the liquid storage cavity 110 is short of liquid according to the potential signal.

Specifically, the detection circuit is composed of resistors, capacitors, diodes and other elements connected in series or in parallel. The detection circuit is electrically connected to the detection electrode 51 and the control module respectively, and can acquire potential signals from the detection electrode 51 and transmit the potential signals to the control module. Each potential signal corresponds to the remaining liquid level in the liquid storage cavity 110, and the control module can judge whether there is a lack of liquid in the liquid storage cavity 110 according to the potential signal, so as to control the nebulization module 61 to continue the operation or stop the operation, thereby achieve a purpose of protecting the nebulization module 61.

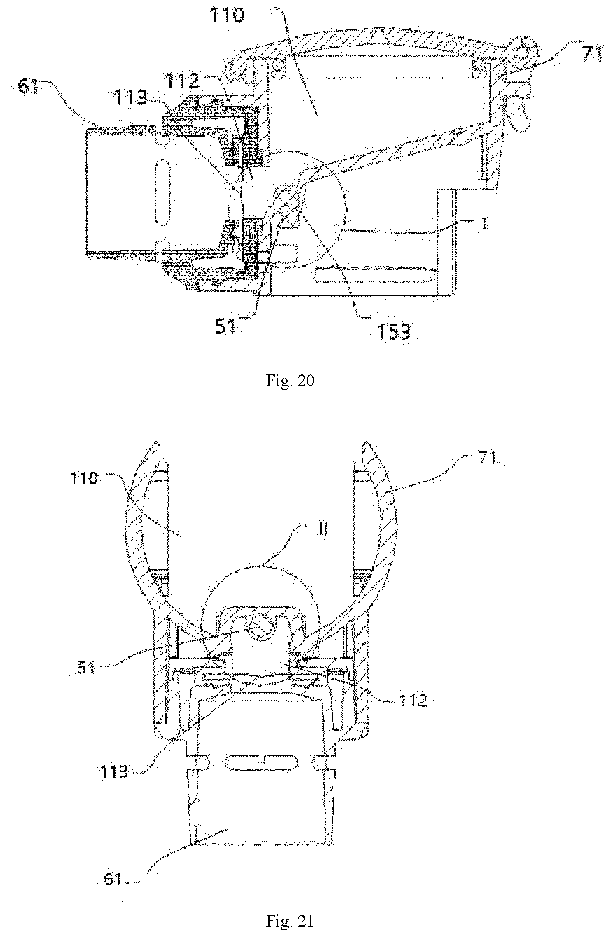

Optionally, as shown in Fig. 20 to Fig. 22, the outer wall of the housing 71 is provided with an accommodating slot 153 at the bottom of the housing 71, and the detection electrode 51 is at least partially embedded in the accommodating slot 153.

Specifically, as shown in Fig. 20 to Fig. 22, the outer wall of the bottom of the housing 71 is provided with an accommodating slot 153, which may be directly formed on the outer wall of the housing 71 by integral die-opening, or may be formed on the outer wall of the housing 71 by assembling. The detection electrode 51 is at least partially embedded in the accommodating slot 153, so that the detection electrode 51 can be fixed. In order to ensure the stability of mounting of the detection electrode 51, the detection electrode 51 and the accommodating slot 153 may be assembled together in an interference-fit manner. In the embodiment of the present disclosure, the accommodating slot 153 is integrally formed with the housing 71, and the housing 71 is bent toward the liquid storage cavity 110 to form the accommodating slot 153. The accommodating slot 153 may be in a cylindrical shape or prism shape, etc., which matches the shape of the detection electrode 51.

Optionally, as shown in Fig 20 to Fig. 22, the accommodating slot 153 is provided with a first snap-fitting part 302 therein, the detection electrode 51 is provided with a second snap-fitting part 202, and the first snap-fitting part 302 is snap-fitted with the second snap-fitting part 202.

Specifically, as shown in Fig. 20 to Fig. 22, in order to ensure the stability of mounting of the detection electrode 51 and prevent the detection electrode 51 from falling out of the accommodating slot 153, the accommodating slot 153 is provided with a first snap-fitting part 302 therein, and the detection electrode 51 is provided with a second snap-fitting part 202, so that the detection electrode 51 can be stably assembled with the accommodating slot 153 by means of the snap-fitting between the first snap-fitting part 302 and the second snap-fitting part 202. The first snap-fitting part 302 may be a boss, and correspondingly, the second snap-fitting part 202 may be a recess; similarly, the first snap-fitting part 302 may be a recess, and correspondingly, the second snap-fitting part 202 may be a boss. Alternatively, the first snap-fitting part 302 and the second snap-fitting part 202 may be hooks, threads or other structures, as long as a stable connection between the accommodating slot 153 and the detection electrode 51 can be realized by means of them. In an embodiment of the present disclosure, a boss is arranged on the wall of the accommodating slot 153, and a recess is arranged in the side surface of the detection electrode 51. By means of the fitting between the boss and the recess, the detection electrode 51 can be fitted in the accommodating slot 153, thereby the detection electrode 51 is prevented from falling out of the accommodating slot 153.

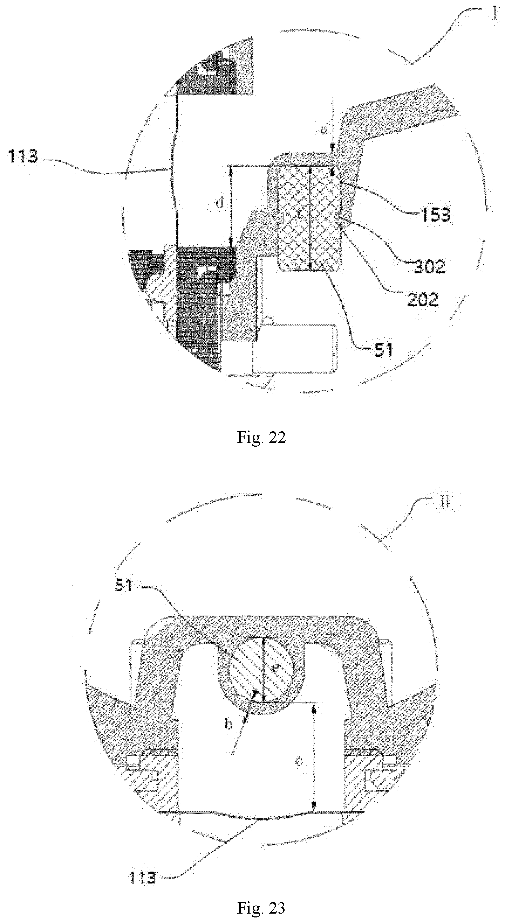

Optionally, as shown in Fig. 22 to Fig. 23, the thickness of the bottom of the accommodating slot 153 is 0.1 mm - 3 mm. The wall thickness of the accommodating slot 153 near the nebulization module 61 is 0.1 mm - 3 mm. The nebulization module 61 comprises a nebulization plate 113, which is arranged opposite to the liquid outlet 112; and the distance between the nebulization plate 113 and the detection electrode 51 is 0.1 mm - 20 mm in the thickness direction of the nebulization plate 113. The distance between an upper edge of the detection electrode 51 and a lower edge of the nebulization plate 113 is 0 mm - 20 mm in the height direction of the nebulization plate 113. The dimension of the detection electrode 51 is 1 mm - 10 mm in the thickness direction of the nebulization plate 113; the dimension of the detection electrode 51 is 1 mm - 10 mm in the height direction of the nebulization plate 113.

Specifically, as shown in Fig. 22 to Fig. 23, the nebulization module 61 comprises a nebulization plate 113, which may be of an ultrasonic nebulization plate 113, or a mesh-type nebulization plate 113, etc. The nebulization plate 113 is arranged opposite to the liquid outlet 112, and the liquid passing through fine pores in the nebulization plate 113 is nebulized by the nebulization plate 113 vibrating at high frequencies into nebulized particles. The nebulization plate 113 and the detection electrode 51 may be arranged in parallel or in other directions, for example, the detection electrode 51 may be perpendicular to the nebulization plate 113 or at a certain angle with respect to the nebulization plate 113.

After the detection electrode 51 is separated from the liquid by the housing 71, there is little difference in the sample value between the case that there is liquid in the liquid storage cavity 110 and the case that there is no liquid in the liquid storage cavity 110, which also brings a challenge to the accuracy of liquid shortage detection. An expected detection result can be achieved by controlling the area/height of the detection electrode 51 corresponding to the liquid level, the wall thickness between the liquid level and the detection electrode 51, and the relative position between the detection electrode 51 and the nebulization plate 113.

As shown in Fig. 22 and Fig. 23, the thickness a of the bottom of the accommodating slot 153 is 0.1 mm - 3 mm; the wall thickness b of the accommodating slot 153 near the nebulization module 61 is 0.1 mm - 3 mm; the distance c between the nebulization plate 113 and the detection electrode 51 is 0.1 mm - 20 mm in the thickness direction of the nebulization plate 113; the distance d between an upper edge of the detection electrode 51 and a lower edge of the nebulization plate 113 is 0 mm - 20 mm in the height direction of the nebulization plate 113; the dimension e of the detection electrode 51 is 1 mm - 10 mm in the thickness direction of the nebulization plate 113; the dimension f of the detection electrode 51 is 1 mm - 10 mm in the height direction of the nebulization plate 113. With the above dimension definitions, the accuracy of liquid shortage detection can be ensured.

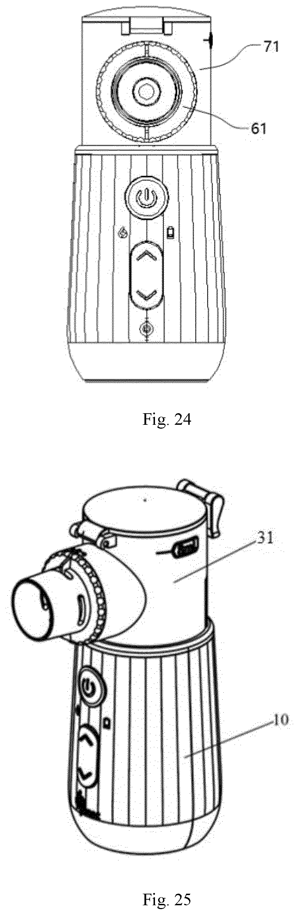

As shown in Fig. 24, an embodiment of the present disclosure further provides a nebulization device, which comprises the above-mentioned assembly having a nebulization function.

Specifically, as shown in Fig. 24, the nebulization device may be a humidifier, an aromatherapy device, or a nebulization therapeutic device, etc. The nebulization device comprises a main unit and the above-mentioned assembly having a nebulization function. The main unit comprises a housing, a battery, and a circuit board arranged in the housing, etc. The main unit is further provided with electrode contacts for supplying power to a nebulization module 61, and a user can control the functions of the nebulization device, such as start and stop, by performing corresponding operations on the main unit; besides, the main unit can further provide a holding space for the user, so that the user can use the device conveniently.

The main unit is provided with a connecting structure at the top of the main unit, the housing 71 is mounted to the main unit in a plug-in manner via the connecting structure, and the housing 71 can be assembled to the main unit by snap-fitting or bolting, etc. Liquid is stored in the liquid storage cavity 110 of the housing 71, the sidewall of the housing 71 is provided with a liquid outlet 112 for leading out the liquid, and the nebulization module 61 is in communication with the liquid outlet 112, so that the liquid led out from the liquid outlet 112 can be nebulized.

In the embodiment of the present disclosure, the nebulization device employs the above-mentioned assembly having a nebulization function, which comprises a housing 71, a nebulization module 61 and a detection electrode 51; the housing 71 is provided with a liquid storage cavity 110 and a liquid outlet 112 in communication with the liquid storage cavity 110; the nebulization module 61 is detachably connected to the housing 71 and is in communication with the liquid outlet 112; the detection electrode 51 is arranged on an outer wall of the housing 71 at the bottom of the housing 71, and the outer wall of the housing 71 separates the detection electrode 51 from the liquid storage cavity 110; the detection electrode 51 is used for detecting whether the liquid in the liquid storage cavity 110 is in shortage. By arranging the detection electrode 51 on the outer wall of the housing 71, a liquid shortage detection function can also be realized under a condition that the detection electrode 51 is not in direct contact with the liquid in the liquid storage cavity 110, so that the liquid is prevented from becoming an accessible charged body, a risk of electric shock is reduced, and the safety of use is improved; moreover, since the detection electrode 51 is not in direct contact with the liquid in the liquid storage cavity 110, pollution to the liquid can be avoided.

Presently, aerosol inhalation therapy has become an important means to treat diseases in the respiratory system. Existing nebulizers are mainly categorized into mesh-type nebulizers, compressed air nebulizers and ultrasonic nebulizers. A mesh-type nebulizer nebulizes the liquid medicine passing through fine pores of a nebulization plate into nebulized particles by means of the high-frequency vibration of the nebulization plate, then the nebulized particles are inhaled into a human body through an inhalation device (a mask or mouthpiece). In most of the existing mesh-type nebulizers available in the market, the nebulization plate is integrated with the medicine cup into an integral structure, and the medicine cup assembly is fastened to the main unit of the nebulizer by means of a key clip. The working principle of the key clip is as follows: The end of the key is a semi-arc retraining rib and is connected to a spring, and the spring can be elastically retracted by manual pressing, thereby the key is driven to retract. When the key is pressed, the retaining rib is moved backward to release the fastening between the medicine cup assembly and the main unit, so that the medicine cup falls off. However, the above nebulizer has the following problems: the key clip has a complex structure and is difficult to assemble, resulting in severe waste of assembly and material costs; if the key is touched or pressed by mistake, the medicine cup assembly may fall off easily and be damaged.

In order to solve the problem that the medicine cup assembly falls off and is damaged owing to accidental touching or pressing of the key in existing nebulizers, the present disclosure provides a nebulizer. The nebulizer provided in the embodiments of the present disclosure will be described below in detail in specific embodiments and application scenarios with reference to Fig. 25 to Fig. 32.

The present disclosure provides a nebulizer, which comprises a main unit 10 and a medicine cup assembly 31 detachably mounted on the main unit 10. The nebulizer further comprises a guiding and limiting structure and a locking structure, wherein the guiding and limiting structure is used for guiding and limiting the medicine cup assembly 31 when the medicine cup assembly 31 is mounted, and the locking structure is used for releasably locking the medicine cup assembly 31 to the main unit 10.

In the nebulizer provided by the present disclosure, with the above technical scheme, the guiding and limiting structure can guide and limit the medicine cup assembly 31 in the process of mounting the medicine cup assembly 31 on the main unit 10, so that the medicine cup assembly 31 can be smoothly mounted and the accuracy of assembly of the medicine cup assembly 31 to the main unit 10 can be ensured; besides, if the locking structure is accidentally released (e.g., touched or squeezed by mistake), the guiding and limiting structure can limit the medicine cup assembly 31 on the main unit 10, and the medicine cup assembly 31 will not fall off and be damaged. Thus, the nebulizer in the present disclosure can not only meet the requirement of the user for replacing and removing the medicine cup assembly by himself/herself, but also improve usability and safety.

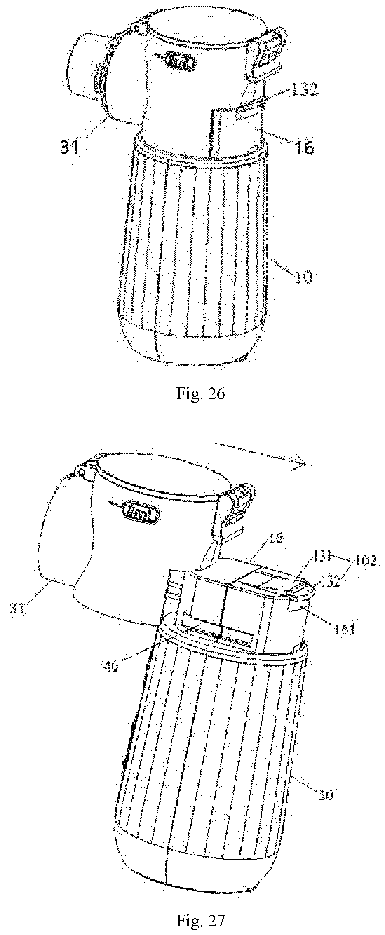

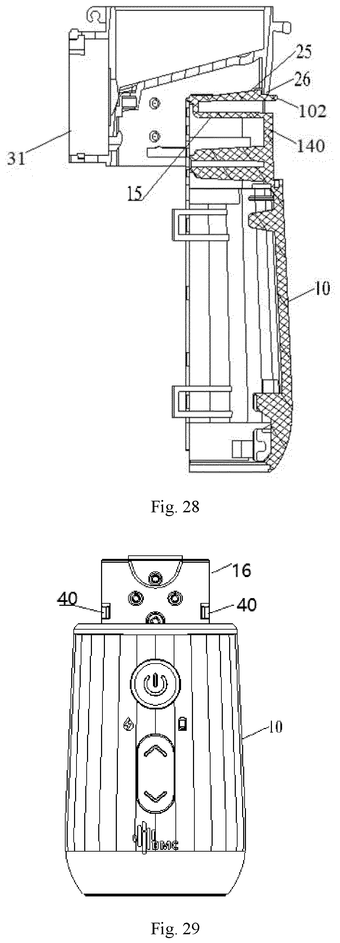

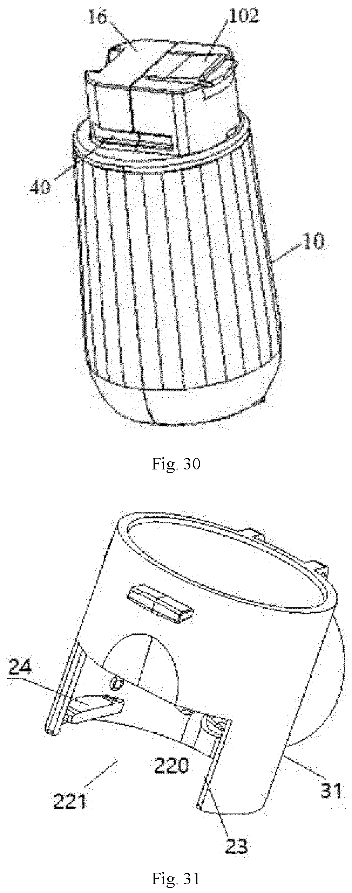

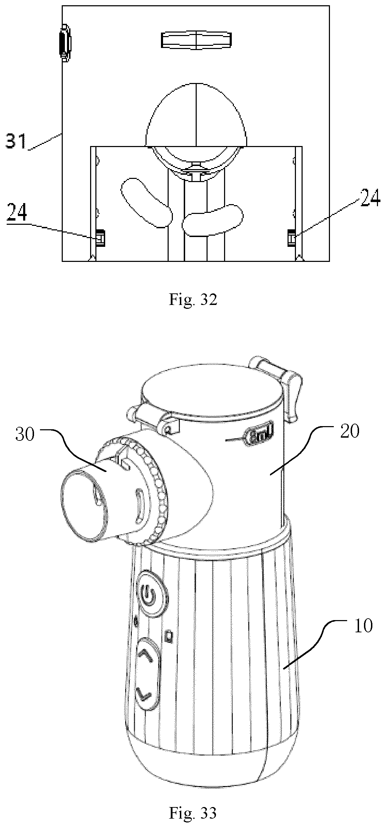

Specifically, as shown in Figs. 25 and Fig. 26, the medicine cup assembly 31 is detachably mounted on the top of the main unit 10. As shown in Figs. 29 and Fig. 30, the main unit 10 may comprise a mounting part 16 positioned at the upper part thereof, and the mounting part 16 is generally cylindrical. As shown in Figs. 31 and Fig. 32, the medicine cup assembly 31 may comprise a mounting cavity 220 for mounting the mounting part 16. It may be noted that the mounting part 16 and the mounting cavity 220 may have other shapes and structures in other embodiments. The medicine cup assembly 31 may be mounted to the main unit 10 in a vertical direction, and, in that case, the mounting cavity 220 may have a bottom opening into which the mounting part 16 can be mounted vertically. Alternatively, the medicine cup assembly 31 may be mounted on the main unit 10 in the horizontal direction, and, in that case, the mounting cavity 220 may have a bottom opening 221 and a side opening 23 (see Fig. 27 and Fig. 31) into which the mounting part 16 can be mounted horizontally.

In the present disclosure, the guiding and limiting structure may comprise a retaining rib 24 and a first slide channel 40 that are fitted with each other, wherein the retaining rib 24 is arranged on one of the mounting part 16 and the mounting cavity 220, while the first slide channel 40 is arranged on the other of the mounting part 16 and the mounting cavity 220, and the retaining rib 24 and the first slide channel 40 extend in the mounting direction of the medicine cup assembly 31 (see the direction indicated by the arrow in Fig. 27). The fitting between the retaining rib 24 and the first slide channel 40 can not only guide and limit the medicine cup assembly 31, but also has a simple structure, is easy to manufacture and has a low production cost.

The retaining rib 24 and the first slide channel 40 may be respectively arranged on the mounting part 16 and the mounting cavity 220 at any suitable position, for example, on the top or a side of the mounting part 16 and the mounting cavity 220. A plurality of retaining ribs 24 and a plurality of first slide channels 40 may be provided. In order to improve the stability and the guiding and limiting performance, the guiding and limiting structure preferably comprises two retaining ribs 24 and two first slide channels 40, wherein the two retaining ribs 24 are respectively arranged on two opposite sides of the mounting part 16 or the mounting cavity 220, and the two first slide channels 40 are respectively arranged on two opposite sides of the mounting cavity 220 or the mounting part 16. Specifically, for example, as shown in Fig. 31 and Fig. 32, two retaining ribs 24 are respectively arranged on two opposite sides of the mounting cavity 220. As shown in Fig. 29 and Fig. 30, two first slide channels 40 are respectively arranged on two opposite sides of the mounting part 16. In that case, the sidewalls of the two opposite sides of the mounting part 16 may be curved surfaces or flat surfaces. In order to reduce the occupied space and facilitate the sliding of the medicine cup assembly, the sidewalls of the two opposite sides of the mounting part 16 are preferably flat surfaces, as shown in Fig. 30.

In the present disclosure, the locking structure may be implemented as appropriate, as long as the medicine cup assembly 31 can be releasably locked to the main unit 10. Moreover, the locking action of the locking structure can be independently performed, i.e., the locking structure can be locked by means of an additional operation after the medicine cup assembly 31 is mounted in place; alternatively, the locking action may be associated with the guiding and limiting action of the guiding and limiting structure, for example, in the sliding process of the medicine cup assembly 31, the locking structure can be locked by using a sliding force.

In the present disclosure, preferably the locking structure is configured to lock the medicine cup assembly 31 to the main unit 10 when the retaining rib 24 slides to a stroke end of the first slide channel 40, thereby the compactness of the structure and the accuracy of fitting can be improved. According to an embodiment of the present disclosure, the locking structure may comprise a snap-fitting clip 102 and a snap-fitting groove 25, wherein the snap-fitting clip 102 is arranged on one of the mounting part 16 and the mounting cavity 220, while the snap-fitting groove 25 is arranged on the other of the mounting part 16 and the mounting cavity 220, and the snap-fitting clip 102 comprises a snap-fitting portion 131 and a pressing portion 132, the snap-fitting portion 131 is configured to be snap-fitted in the snap-fitting groove 25 when the medicine cup assembly 31 is mounted in place, and the pressing portion 132 is exposed to the snap-fitting portion 131 so that the snap-fitting portion 131 can be disengaged from the snap-fitting groove 25 by pressing the pressing portion 132. To dismount the medicine cup assembly 31, the pressing portion 132 may be pressed so that the snap-fitting portion 131 is disengaged from the snap-fitting groove 25, thereby the locking is released; then the medicine cup assembly 31 may be moved in a direction reverse to the mounting direction of the medicine cup assembly 31; when the retaining rib 24 is disengaged from the first slide channel 40, the medicine cup assembly 31 is disengaged from the main unit 10.

Specifically, in the embodiment shown in Fig. 27 and Fig. 28, the snap-fitting clip 102 is an elastic tab extending obliquely upward from the top of the mounting part 16 in the mounting direction of the medicine cup assembly 31. The snap-fitting groove 25 is positioned at the top of the mounting cavity 220 and is arranged close to the side opening 23; the upper edge 26 of the side opening 23 forms an end wall of the snap-fitting groove 25, and the upper edge 26 can abut against the top surface of the elastic tab to press the elastic tab downward when the medicine cup assembly 31 is mounted.

As shown in Fig. 27, to mount the medicine cup assembly 31, first, the retaining rib 24 is aligned to the first slide channel 40, and the medicine cup assembly 31 can be pushed or pulled to slide to the right along the first slide channel 40; in the sliding process, the upper edge 26 of the medicine cup assembly 31 continuously presses down the elastic tab; when the medicine cup assembly 31 slides to the stroke end of the first slide channel 40, the elastic tab is positioned in the snap-fitting groove 25, and, at that moment, the elastic tab snaps into the snap-fitting groove 25 to achieve locking. With the above arrangement, no additional part is needed, the structure is simple, and the loss of assembly cost and material cost can be reduced.

As shown in Fig. 27 and Fig. 28, the mounting part 16 may comprise a U-shaped cantilever arm 15 extending inward (i.e., to the right in Fig. 28) from its outer sidewall 140 and a recess 161 for accommodating the U-shaped cantilever arm 15, wherein an closed end of the U-shaped cantilever arm 15 (i.e., the left end of the U-shaped cantilever arm 15 in Fig. 28) is positioned inside the mounting part 16, and the two sidewalls of the U-shaped cantilever arm 15 are arranged one on top of the other, and the upper sidewall forms a snap-fitting clip 102. By providing a U-shaped cantilever arm 15 and configuring the upper sidewall of the U-shaped cantilever arm 15 into a snap-fitting clip 102 (e.g., an elastic tab), the thin upper sidewall can realize certain elasticity of the elastic tab, and the elastic tab can be deformed in the vertical direction, so that it can be pressed downward and recovered.

In the above embodiment, the U-shaped cantilever arm 15 may be made of a plastic material, and may be integrally injection-molded with the housing (including the outer sidewall 140) of the main unit 10 (see Fig. 28), thereby the structure is further simplified, parts are reduced, and the cost is reduced.

Of course, in other embodiments, the snap-fitting clip 102 may be separate from the main unit, and the snap-fitting clip 102 may be made of an elastic material.

In order to ensure the structural stability of the U-shaped cantilever arm 15 and prolong its service life, the closed end of the U-shaped cantilever arm 15 may be fixed to the mounting part 16.

As shown in Fig. 28, the upper sidewall of the U-shaped cantilever arm 15 comprises a horizontal portion extending in the horizontal direction and a protruding portion protruding from the middle of a top surface of the horizontal portion, a top surface of the protruding portion is an inclined surface extending obliquely upward in the mounting direction of the medicine cup assembly 31, the protruding portion is formed as the snap-fitting portion 131, and an outer end of the horizontal portion (e.g., the left end shown in Fig. 28) is formed as the pressing portion 132. To dismount the medicine cup assembly 31, the pressing portion 132 may be pressed downward so that the snap-fitting portion 131 is disengaged from the snap-fitting groove 25, thereby the locking is released.

By using the above-mentioned guiding and limiting structure and the locking structure, the nebulizer provided by the present disclosure not only has a simple structure, but also has high stability, thereby avoids the risks of the problems caused by the fitting between the parts. It not only solves the problem that the medicine cup assembly falls off and is damaged owing to pressing or squeezing by the user by mistake, but also reduces the material cost and the assembly labor cost, and greatly improves the user experience.

The nebulization technology is widely applied in daily life, such as humidifiers, aromatherapy devices and nebulization therapeutic devices, etc. Such devices can utilize a nebulization plate to nebulize the liquid contained in a container, so as to realize its nebulization function. At present, a nebulization device generally comprises a main unit, a liquid storage apparatus and a nebulization assembly. The liquid in the liquid storage apparatus is introduced into the nebulization assembly through a liquid outlet and then is driven to pass through a nebulization plate to form nebulized particles. During the use of the nebulization device, the liquid storage apparatus and the nebulization assembly are prone to rotate in relation to each other, which may lead to leakage of the liquid from the joint between the liquid storage apparatus and nebulization assembly, and result in waste. The leaking liquid may even cause a short circuit between the probes on the main unit, and lead to safety accidents.

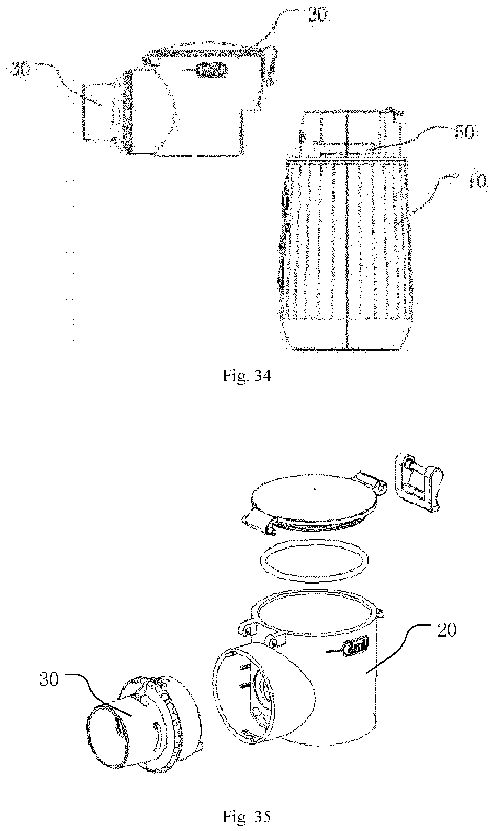

In order to solve a problem that the liquid storage apparatus and the nebulization assembly are prone to rotate in relation to each other and result in liquid leakage from the joint between the liquid storage apparatus and the nebulization assembly in the prior art, the present disclosure provides a device having a nebulization function. The device having a nebulization function provided in the embodiments of the present disclosure will be described below in detail in specific embodiments and application scenarios with reference to Fig. 33 to Fig. 40.

As shown in Fig. 33 to Fig. 38, an embodiment of the present disclosure provides a device having a nebulization function, which comprises: a main unit 10, a liquid storage apparatus 20 and a nebulization assembly 30, wherein the liquid storage apparatus 20 is detachably mounted to the main unit 10 in a plug-in manner; the liquid storage apparatus 20 is provided with a liquid outlet 112, and the nebulization assembly 30 is in communication with the liquid outlet 112; the nebulization assembly 30 is provided with a first limiting structure 301, and the main unit 10 is provided with a second limiting structure 101; when the liquid storage apparatus 20 is assembled to the main unit 10, the first limiting structure 301 and the second limiting structure 101 are snap-fitted with each other, and the nebulization assembly 30 is fixed in relation with the liquid storage apparatus 20; and when the liquid storage apparatus 20 is separated from the main unit 10, the nebulization assembly 30 and the liquid storage apparatus 20 can be dismounted.

Specifically, as shown in Fig. 33 to Fig. 38, the device having a nebulization function comprises a main unit 10, a liquid storage apparatus 20 and a nebulization assembly 30. The main unit 10 comprises a housing, and a battery and a circuit board arranged in the housing, etc. The main unit 10 is further provided with electrode contacts for supplying power to the nebulization assembly 30, and a user can control the functions of the device, such as start and stop, by performing corresponding operations on the main unit 10; besides, the main unit 10 can further provide a holding space for the user, so that the user can use the device conveniently.

The main unit 10 is provided with a connecting structure at the top of the main unit 10, and the liquid storage apparatus 20 is detachably mounted to the main unit 10 in a plug-in manner via the connecting structure, so that the user can mount and dismount it conveniently, thereby the flexibility of use of the device is improved. The liquid storage apparatus 20 can be assembled to the main unit 10 by snap-fitting or bolting, etc. When the liquid storage apparatus 20 is assembled to the main unit 10, the liquid storage apparatus 20 will not be displaced or rotated in relation to the main unit 10. The liquid storage apparatus 20 is used to store a liquid to be nebulized, and the liquid in the liquid storage apparatus 20 may be water, aromatherapy liquid, or medicinal liquid, depending on the specific device and the specific scenario. In an embodiment of the present disclosure, the liquid storage apparatus 20 is provided with a medicine cup containing a liquid medicine, the sidewall of the liquid storage apparatus 20 is provided with a liquid outlet 112 for leading out the liquid medicine, and the nebulization assembly 30 is in communication with the liquid outlet 112, so that the liquid medicine led out from the liquid outlet 112 can be nebulized. Nebulized aerosol inhalation therapy is an important adjuvant therapy, and is the first choice for asthma and chronic obstructive pulmonary diseases. The medicine can be administrated into tracheae and bronchi through mouth or nose to inhibit the inflammatory response of the respiratory tract and relieve clinical symptoms such as bronchospasm, and is of great significance to the improvement of the respiratory tract function of the patient.

The nebulization assembly 30 is further hermetically connected to the housing 71 around the liquid outlet 102 to seal the periphery of the liquid outlet 102, so that the liquid in the liquid storage apparatus 20 can flow into the nebulization assembly 30 only through the liquid outlet 102, and liquid waste caused by liquid leakage can be avoided.

The nebulization assembly 30 comprises a nebulization plate, and it nebulizes the liquid medicine passing through fine pores of the nebulization plate into nebulized particles by means of the high-frequency vibration of the nebulization plate, then the nebulized particles are inhaled into a human body through an inhalation device (a mask or mouthpiece). The nebulization assembly 30 and the liquid storage apparatus 20 may be assembled together by snap-fitting or threaded connection, etc. To assemble the nebulization assembly 30 with the liquid storage apparatus 20, it is necessary to rotate the nebulization assembly 30. During the use of the device having a nebulization function, the nebulization assembly 30 and the liquid storage apparatus 20 may rotate with respect to each other and get loose owing to misoperations and other reasons, and a phenomenon of liquid leakage may easily occur. Therefore, in an embodiment of the present disclosure, to avoid the phenomenon of liquid leakage, the nebulization assembly 30 is provided with a first limiting structure 301, the main unit 10 is provided with a second limiting structure 101, and the first limiting structure 301 and the second limiting structure 101 can be snap-fitted with each other. The first limiting structure 301 and the second limiting structure 101 may be a limiting rod and a limiting hole, or may be a limiting baffle and a limiting groove, or may be other structures, as long as they can be snap-fitted together. There is no particular restriction on their specific structures in the embodiment of the present disclosure.

When the liquid storage apparatus 20 is assembled to the main unit 10, i.e., after the liquid storage apparatus 20 has been plugged into the main unit 10, the first limiting structure 301 and the second limiting structure 101 are snap-fitted together, and, under the limiting action of the first limiting structure 301 and the second limiting structure 101, the nebulization assembly 30 and the main unit 10 will not be displaced or rotated in relation to each other; besides, since the liquid storage apparatus 20 will not be displaced or rotated in relation to the main unit 10, the nebulization assembly 30 and the liquid storage apparatus 20 are fixed in relation to each other, and the problem of liquid leakage caused by relative rotation of the nebulization assembly 30 and the liquid storage apparatus 20 due to misoperations or other reasons during the use of the device is avoided.

In the embodiment of the present disclosure, by arranging a first limiting structure 301 on the nebulization assembly 30 and a second limiting structure 101 on the main unit 10, when the liquid storage apparatus 20 is assembled to the main unit 10, the first limiting structure 301 and the second limiting structure 101 are snap-fitted with each other, so that the nebulization assembly 30 and the liquid storage apparatus 20 can be fixed in relation to each other, and both the liquid storage apparatus 20 and the nebulization assembly 30 are in a stationary state relative to the main unit 10 and will not rotate in relation to each other, so that a problem of liquid leakage during the use of the device is avoided, and the risk of a short circuit between the probes on the main unit 10 is reduced; when the liquid storage apparatus 20 is separated from the main unit 10, the nebulization assembly 30 and the liquid storage apparatus 20 can be dismounted, so that the user can replace the nebulization plate conveniently by himself/herself, and the convenience of use is improved.

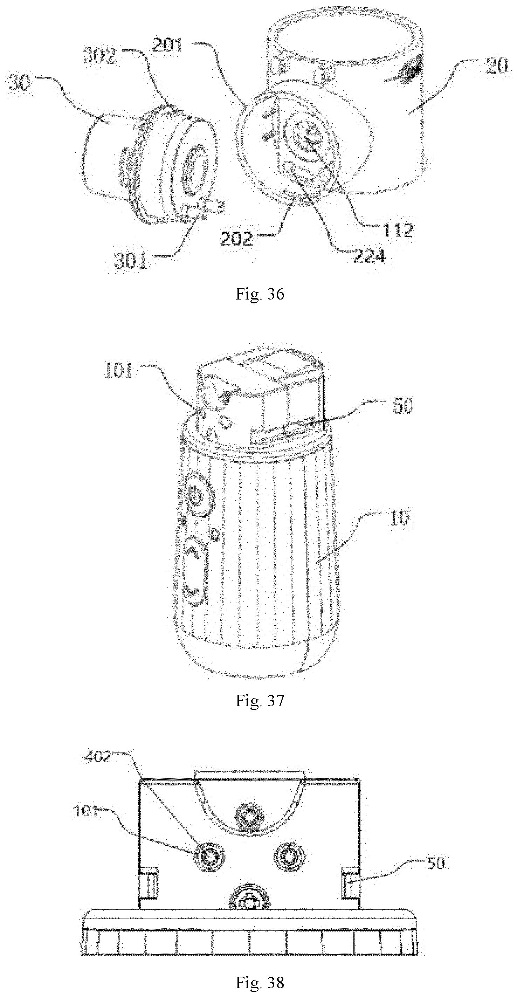

Optionally, as shown in Fig. 36, the first limiting structure 301 is a limiting rod, and the second limiting structure 101 is a limiting hole; when the liquid storage apparatus 20 is assembled to the main unit 10, the limiting rod is at least partially embedded in the limiting hole.

Specifically, as shown in Fig. 36, under the limiting action of the first limiting structure 301 and the second limiting structure 101, there will be no relative displacement or rotation between the nebulization assembly 30 and the main unit 10. The nebulization assembly 30 may be provided with a limiting rod, and accordingly, the main unit 10 may be provided with a limiting hole, and the shape of the limiting rod matches the shape of the limiting hole. When the liquid storage apparatus 20 is assembled to the main unit 10, the limiting rod is at least partially embedded in the limiting hole, so that the nebulization assembly 30 and the main unit 10 are fixed in relation to each other, and the nebulization assembly 30 and the liquid storage apparatus 20 are also fixed in relation to each other, thereby the problem of liquid leakage due to the relative rotation of the nebulization assembly 30 and the liquid storage apparatus 20 is avoided.

Optionally, the first limiting structure 301 is a limiting hole, and the second limiting structure 101 is a limiting rod; when the liquid storage apparatus 20 is assembled to the main unit 10, the limiting rod is at least partially embedded in the limiting hole.

Specifically, in an embodiment of the present disclosure, the nebulization assembly 30 can be provided with a limiting hole, and accordingly, the main unit 10 can be provided with a limiting rod, and the shape of the limiting rod matches the shape of the limiting hole. When the liquid storage apparatus 20 is assembled to the main unit 10, the limiting rod is at least partially embedded in the limiting hole, so that the nebulization assembly 30 and the main unit 10 are fixed in relation to each other, and the nebulization assembly 30 and the liquid storage apparatus 20 are also fixed in relation to each other, thereby the problem of liquid leakage due to the relative rotation of the nebulization assembly 30 and the liquid storage apparatus 20 is avoided.

Optionally, as shown in Fig. 38, conductive probes 402 are arranged in the limiting hole, and are electrically connected to the limiting rod, so that a circuit between the main unit 10 and the nebulization assembly 30 is closed.

Specifically, as shown in Fig. 38, the limiting rod and the limiting hole can not only limit the main unit 10 and the nebulization assembly 30, but also realize circuit closing. Conductive probes 402 are arranged in the limiting hole; when the limiting rod is embedded in the limiting hole, the conductive probes 402 are electrically connected to the limiting rod, so that a circuit between the main unit 10 and the nebulization assembly 30 is closed. With the fitting between the limiting rod and the limiting hole, a limiting function is realized for the main unit 10 and the nebulization assembly 30, and the circuit between the main unit 10 and the nebulization assembly 30 is closed; thus, the number of parts is reduced and the production cost is reduced.

Optionally, as shown in Fig. 33 to Fig. 38, at least two limiting rods, at least two limiting holes and at least two conductive probes 402 are provided.

Specifically, as shown in Fig. 33 to Fig. 38, in an embodiment of the present disclosure, at least two limiting rods, at least two limiting holes and at least two conductive probes 402 are provided. For example, in the embodiment of the present disclosure, two groups of limiting rods, limiting holes and conductive probes 402 are used to form positive and negative circuits respectively; besides, the stability of limiting the main unit 10 and the nebulization assembly 30 is also improved.

Optionally, as shown in Fig. 33 to Fig. 38, the liquid storage apparatus 20 is provided with a mounting base 201, and the nebulization assembly 30 is at least partially embedded in the mounting base 201 and rotatably connected to the mounting base 201.

Specifically, as shown in Fig. 33 and Fig. 38, the nebulization assembly 30 can be connected to the liquid storage apparatus 20 by rotation. The liquid storage apparatus 20 is provided with the mounting base 201, and the nebulization assembly 30 is at least partially embedded in the mounting base 201, thereby the reliability of the connection between the nebulization assembly 30 and the liquid storage apparatus 20 is ensured.

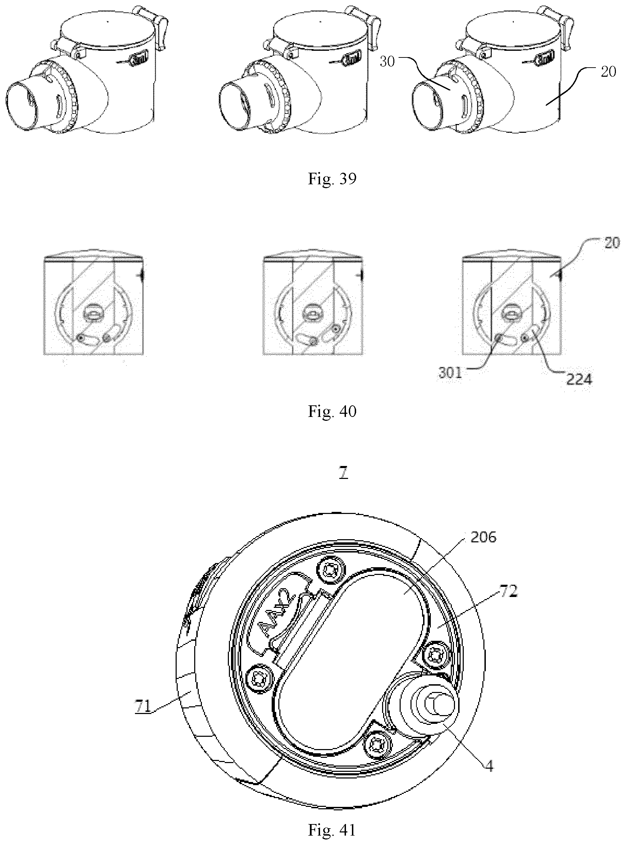

Optionally, as shown in Fig. 36 and Fig. 39, the nebulization assembly 30 is provided with a first snap-fitting part 302, and the mounting base 201 is provided with a second snap-fitting part 202; the nebulization assembly 30 and the mounting base 201 can be separated from each other when the nebulization assembly 30 and the mounting base 201 are in a first relative position; the first snap-fitting part 302 and the second snap-fitting part 202 are snap-fitted with each other when the nebulization assembly 30 and the mounting base 201 are in a second relative position.

Specifically, as shown in Fig. 36 and Fig. 39, the nebulization assembly 30 and the liquid storage apparatus 20 can be assembled by snap-fitting. The nebulization assembly 30 is provided with a first snap-fitting part 302, and the mounting base 201 is provided with a second snap-fitting part 202. The nebulization assembly 30 and the mounting base 201 can be separated from each other when the nebulization assembly 30 and the mounting base 201 are in a first relative position; thus, the user can replace the nebulization plate and replenish the liquid conveniently. The nebulization assembly 30 and the liquid storage apparatus 20 can rotate in relation to each other. When the nebulization assembly 30 and the mounting base 201 rotate to a second relative position, the first snap-fitting part 302 is snap-fitted with the second snap-fitting part 202, and the nebulization assembly 30 and the liquid storage apparatus 20 are stably assembled. At that point, the liquid storage apparatus 20 can be plugged into the main unit 10, and, under the limiting action of the first limiting structure 301 and the second limiting structure 101, the nebulization assembly 30, the liquid storage apparatus 20 and the main unit 10 are stationary in relation to each other, so that the problem of liquid leakage will not occur.

Fig. 39 shows a flowchart of mounting of the nebulization assembly 30 and the liquid storage apparatus 20 in an embodiment of the present disclosure. After the liquid storage apparatus 20 is removed from the main unit 10, the nebulization assembly 30 and the liquid storage apparatus 20 are set in the first relative position by rotating the nebulization assembly 30, so that the used nebulization assembly 30 can be removed and replaced with a new nebulization assembly 30; then, the nebulization assembly 30 is rotated to set the nebulization assembly 30 and the liquid storage apparatus 20 in the second relative position, thus they are mounted and fixed.

Optionally, both the nebulization assembly 30 and the mounting base 201 are provided with a threaded structure, and the nebulization assembly 30 is connected to the mounting base 201 through threads.

Specifically, alternatively, the nebulization assembly 30 and the liquid storage apparatus 20 may be assembled together by means of a threaded connection. Both the nebulization assembly 30 and the mounting base 201 are provided with a threaded structure, and the nebulization assembly 30 and the liquid storage apparatus 20 are fixed by screwing.

Optionally, as shown in Fig. 4 and Fig. 40, the bottom of the mounting base 201 is provided with a clearance hole 224; the limiting rod is inserted through the clearance hole 224 and slides in relation to the mounting base 201 in the extension direction of the clearance hole 224.

Specifically, as shown in Fig. 36 and Fig. 40, in order to reduce the mounting space, the limiting rod may be inserted into the mounting base 201. Since relative rotation between the nebulization assembly 30 and the mounting base 201 is required, an clearance hole 224 can be arranged at the bottom of the mounting base 201 in order to avoid the limitation caused by the limiting rod when the nebulization assembly 30 is assembled with the mounting base 201, and the limiting rod is inserted through the clearance hole 224, so that when the nebulization assembly 30 is assembled with the mounting base 201 by rotation, the limiting rod also slides in relation to the mounting base 201 in the extension direction of the clearance hole 224, and the extension direction of the clearance hole 224 matches the sliding path of the limiting rod, to avoid blocking the limiting rod. The clearance hole 224 may be an oblong hole or an arc-shaped hole, etc.

Fig. 40 shows a flowchart of mounting of the nebulization assembly 30 and the liquid storage apparatus 20 in an embodiment of the present disclosure. After the liquid storage apparatus 20 is removed from the main unit 10, the nebulization assembly 30 and the liquid storage apparatus 20 are set in the first relative position by rotating the nebulization assembly 30, so that the used nebulization assembly 30 can be removed; at that point, the limiting rod slides from the left side of the clearance hole 224 to the right side of the clearance hole 224; after the nebulization assembly 30 is replaced with a new one, the nebulization assembly 30 is rotated so that the nebulization assembly 30 and the liquid storage apparatus 20 are set in the second relative position; at that point, the limiting rod slides from the right side of the clearance hole 224 to the left side of the clearance hole 224, thus, mounting and fixation are realized.

Optionally, as shown in Fig. 37 to Fig. 38, a slide channel assembly 50 is arranged between the main unit 10 and the liquid storage apparatus 20; the liquid storage apparatus 20 is detachably mounted to the main unit 10 in a plug-in manner via the slide channel assembly 50.

Specifically, as shown in Fig. 37 to Fig. 38, the slide channel assembly 50 may be a combination of a slider and a slide channel. For example, the slider is arranged on the liquid storage apparatus 20, while the slide channel is arranged on the main unit 10, and the liquid storage apparatus 20 can be smoothly plugged into the main unit 10 by means of the fitting between the slider and the slide channel; similarly, the liquid storage apparatus 20 may be provided with a slide channel, while the main unit 10 may be provided with a slider. The slider and the slide channel may be respectively arranged on the two sides of an entire assembly formed by the main unit 10 and the liquid storage apparatus 20, and may be arranged symmetrically; thus, the stability of the assembling of the liquid storage apparatus 20 and the main unit 10 can be improved.

Optionally, the device having a nebulization function is a nebulization therapeutic device.

Specifically, a nebulization therapeutic device is an important adjunctive therapeutic appliance. Nebulized aerosol inhalation therapy is an important adjuvant therapy, and is the first choice for asthma and chronic obstructive pulmonary diseases. The medicine can be administrated into tracheae and bronchi through mouth or nose to inhibit the inflammatory response of the respiratory tract and relieve clinical symptoms such as bronchospasm, and is of great significance to the improvement of the respiratory tract function of the patient.

In the embodiment of the present disclosure, by arranging a first limiting structure 301 on the nebulization assembly 30 and a second limiting structure 101 on the main unit 10, when the liquid storage apparatus 20 is assembled to the main unit 10, the first limiting structure 301 and the second limiting structure 101 are snap-fitted with each other, so that the nebulization assembly 30 and the liquid storage apparatus 20 can be fixed in relation to each other, and both the liquid storage apparatus 20 and the nebulization assembly 30 are in a stationary state relative to the main unit 10 and will not rotate in relation to each other, so that a problem of liquid medicine leakage during the use of the device is avoided, and the risk of a short circuit between the probes on the main unit 10 is reduced; when the liquid storage apparatus 20 is separated from the main unit 10, the nebulization assembly 30 and the liquid storage apparatus 20 can be dismounted, so that the user can replace the nebulization plate conveniently by himself/herself, and the convenience of use is improved.

Presently, aerosol inhalation therapy has become an important means to treat diseases in the respiratory system. Existing nebulizers are mainly categorized into mesh-type nebulizers, compressed air nebulizers and ultrasonic nebulizers. A mesh-type nebulizer nebulizes the liquid medicine passing through fine pores of a nebulization plate into nebulized particles by means of the high-frequency vibration of the nebulization plate, then the nebulized particles are inhaled into a human body through an inhalation device (a mask or mouthpiece). Mesh-type nebulizer usually has two power supply means, one of which is direct power supply from an external DC power source, and the other of which is power supply from a dry battery. Consequently, when one power supply is connected, the interface electrodes of the other power supply are accessible, and may result in a leakage current out of specification and bring a safety risk. Therefore, in order to improve safety, it is necessary to design a power supply mutual exclusion structure.

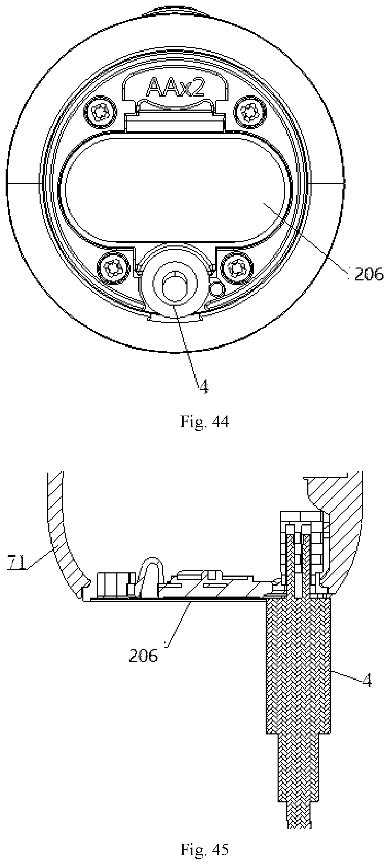

In order to prevent the interface electrodes of another power supply from being touched by a human body when one power supply is connected, thereby enhance the safety, the present disclosure provides a power supply mutual exclusion structure, a power supply mutual exclusion method and an electronic product. The power supply mutual exclusion structure, the power mutually exclusive method and the electronic product provided in the embodiments of the present disclosure will be described in detail below in specific embodiments and application scenarios with reference to Fig. 41 to Fig. 50.

In a first aspect, the present disclosure provides a power supply mutual exclusion method for an electronic product that has a battery compartment 208 for mounting a battery 6, a battery compartment cover 206 for opening and closing a compartment opening of the battery compartment 208, and an external power supply interface 3 for plugging a power plug 4, the power supply mutual exclusion method comprising: configuring the battery compartment cover 206 so that the battery compartment cover 206 at least partially shields the external power supply interface 3 when it opens the compartment opening and doesn't shield the external power supply interface 3 when it closes the compartment opening.

It may be noted that the above-mentioned "configuring the battery compartment cover 206 ... when it opens the compartment opening" means that the battery compartment cover 206 opens a part of the compartment opening or opens the compartment opening fully (i.e., the battery compartment cover 206 doesn't shield the compartment opening, and the compartment opening is fully exposed).

In the case that the battery interface electrodes are arranged at the bottom of the battery compartment 208 and the compartment opening is arranged at the top of the battery compartment 208, the battery interface electrodes are accessible only when the compartment opening is fully opened. Therefore, the safety can be ensured as long as the battery compartment cover 206 can at least partially shield the external power supply interface 3 when it opens the compartment opening fully.

In addition, it may be noted that the position of the compartment opening when the battery compartment cover 206 opens the compartment opening fully is not necessarily the maximum open position of the battery compartment cover 206.

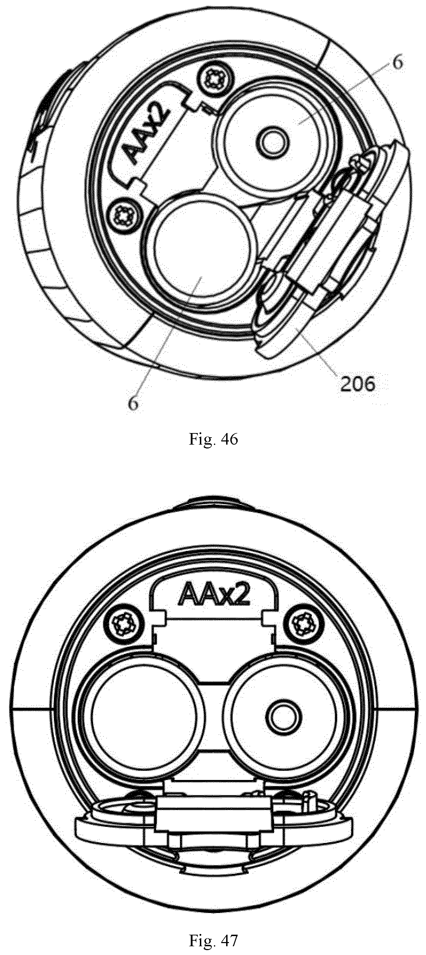

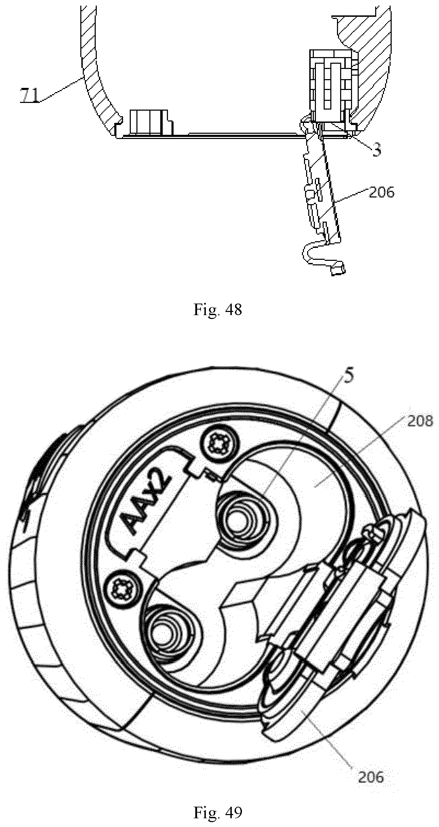

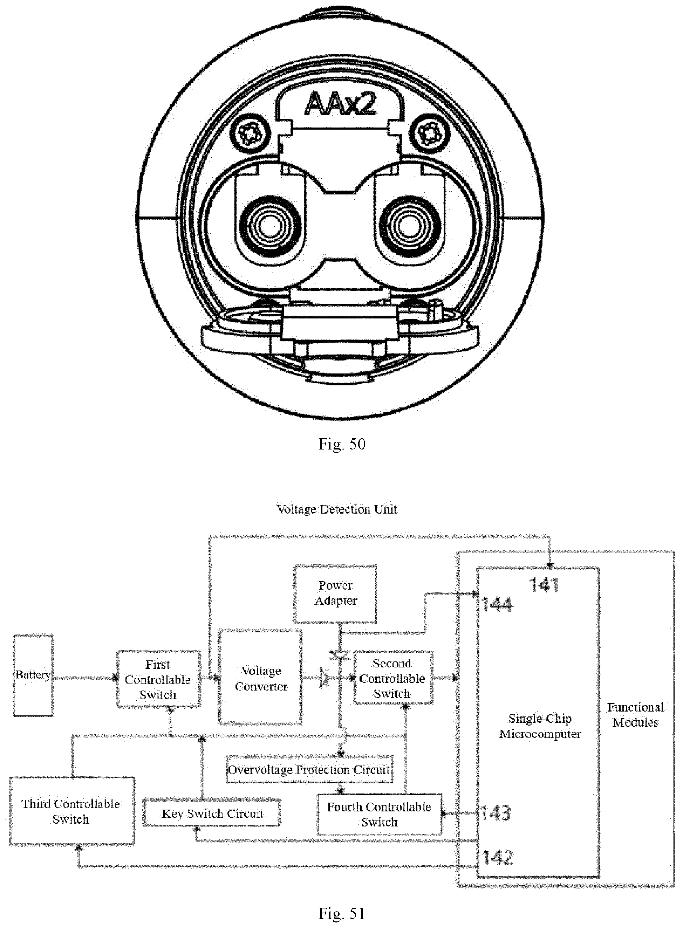

According to the power supply mutual exclusion method for an electronic product in the present disclosure, with the above technical scheme, when the battery compartment cover 206 is opened during use (see Fig. 46 to Fig. 50), a power plug 4 can't be plugged into the external power supply interface 3 because the battery compartment cover 206 at least partially shields the external power supply interface 3; in that case, if no battery 6 is mounted (see Fig. 49 and Fig. 50), the battery interface electrodes 5 are accessible, but, since there is no external power supply connected currently, there is no safety risk incurred by touching the battery interface electrodes 5; if a battery 6 is mounted (see Fig. 46 to Fig. 48), only the battery 6 is accessible, and there is no safety risk. A power plug 4 can be plugged into the external power supply interface 3 only when the battery compartment cover 206 is closed (see Fig. 41 to Fig. 45); in that state, the electrodes of the battery 6 and the battery interface electrodes 5 are inaccessible, so there is no safety risk. Therefore, with the power mutual exclusion structure in the present disclosure, the battery interface electrodes are inaccessible for the human body when an external power supply is connected; when the battery interface electrodes are accessible, no external power supply can be connected; thus, the safety is enhanced.

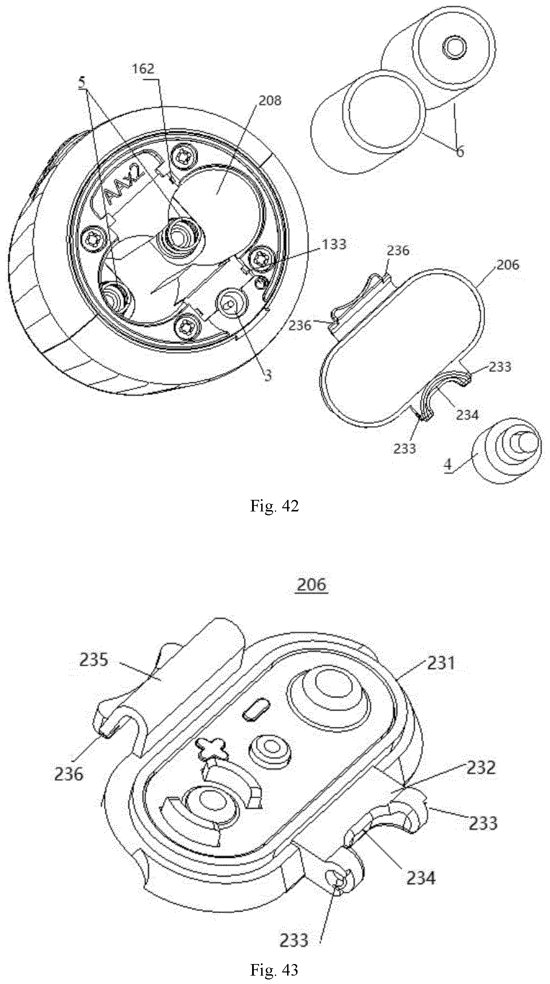

For example, the battery compartment cover 206 may open and close the compartment opening by rotation. As shown in Fig. 41 and Fig. 42, the compartment opening and the external power supply interface 3 may be positioned on the same side (i.e., on the same side of the electronic product), and the battery compartment cover 206 can be rotatably arranged at the compartment opening, with the rotation axis of the battery compartment cover 206 positioned at one side of the compartment opening (see the right side of the compartment opening shown in Fig. 42) and the external power supply interface 3 positioned near the rotation axis of the battery compartment cover 206. Thus, when the battery compartment cover 206 opens the compartment opening, the battery compartment cover 206 rotates around the rotation axis. Since the external power supply interface 3 is positioned near the rotation axis of the battery compartment cover 206, the battery compartment cover 206 can partially or fully cover the external power supply interface 3.

Furthermore, the power supply mutual exclusion method may further comprise: configuring the power plug 4 to block the battery compartment cover 206 so that the battery compartment cover 206 can't be opened or can't be opened fully when the power plug 4 is plugged in the external power supply interface 3. Thus, the battery compartment cover 206 can't be fully opened to an extent that the battery interface electrodes are accessible, and there is no safety risk.

In a second aspect, the present disclosure provides a power supply mutual exclusion structure, which comprises a battery compartment 208, a battery compartment cover 206 and an external power supply interface 3, wherein the external power supply interface 3 is used for plugging a power plug 4; the bottom of the battery compartment 208 is provided with battery interface electrodes 5; the battery compartment 208 has a compartment opening for loading a battery 6 therein; the battery compartment cover 206 is movably arranged at the compartment opening to open and close the compartment opening; the battery compartment cover 206 is configured as follows: the battery compartment cover 206 at least partially shields the external power supply interface 3 when it opens the compartment opening; the battery compartment cover 206 doesn't shield the external power supply interface 3 when it closes the compartment opening.

According to the power supply mutual exclusion structure in the present disclosure, with the above technical scheme, when the battery compartment cover 206 is opened during use (see Fig. 46 to Fig. 50), a power plug 4 can't be plugged into the external power supply interface 3 because the battery compartment cover 206 at least partially shields the external power supply interface 3; in that case, if no battery 6 is mounted (see Fig. 49 and Fig. 50), the battery interface electrodes 5 are accessible, but, since there is no external power supply connected currently, there is no safety risk incurred by touching the battery interface electrodes 5; if a battery 6 is mounted (see Fig. 46 to Fig. 48), only the battery 6 is accessible, and there is no safety risk. A power plug 4 can be plugged into the external power supply interface 3 only when the battery compartment cover 206 is closed (see Fig. 41 to Fig. 45); in that state, the electrodes of the battery 6 and the battery interface electrodes 5 are inaccessible, so there is no safety risk. Therefore, with the power mutual exclusion structure in the present disclosure, the battery interface electrodes are inaccessible for the human body when an external power supply is connected; when the battery interface electrodes are accessible, no external power supply can be connected; thus, the safety is enhanced.

In the present disclosure, the battery compartment cover 206 may open and close the compartment opening in any suitable way, as long as the above-mentioned cooperation with the external power supply interface 3 can be ensured. For example, in some embodiments, the battery compartment cover 206 can open and close the compartment opening by rotation or sliding.

In an embodiment where the battery compartment cover 206 opens and closes the compartment opening by sliding, the compartment opening and the external power supply interface 3 may be positioned on the same side (i.e., on the same side of the electronic product), and the battery compartment cover 206 can slide between the compartment opening and the external power supply interface 3; when the battery compartment cover 206 slides to a position above the compartment opening and closes the compartment opening, the external power supply interface 3 is fully exposed, so that a power plug 4 can be plugged into the external power supply interface 3; when the battery compartment cover 206 slides to a position above the external power supply interface 3 and fully open the compartment opening, the battery compartment cover 206 can partially or fully block the external power supply interface 3 and prevent the power plug 4 from being plugged into the external power supply interface 3.

In the case that the battery compartment cover 206 opens and closes the compartment opening by rotation, for example, as shown in Fig. 41 and Fig. 42, the compartment opening and the external power supply interface 3 may be positioned on the same side (i.e., on the same side of the electronic product), and the battery compartment cover 206 can be rotatably arranged at the compartment opening, with the rotation axis of the battery compartment cover 206 positioned at one side of the compartment opening (see the right side of the compartment opening shown in Fig. 42) and the external power supply interface 3 positioned near the rotation axis of the battery compartment cover 206.

The external power supply interface 3 may be positioned on the rotation axis of the battery compartment cover 206, so that the battery compartment cover 206 can partially or fully shield the external power supply interface 3 by rotating over a small angle (i.e., partially opening the compartment opening). Of course, there may be a certain distance between the external power supply interface 3 and the rotation axis of the battery compartment cover 206. For example, as indicated by the orientation shown in Fig. 42, the external power supply interface 3 is positioned at the right side of the rotation axis of the battery compartment cover 206; thus, the battery compartment cover 206 can partially shield the external power supply interface 3 (see Fig. 48) or fully shield the external power supply interface 3 when the battery compartment cover 206 fully opens the compartment opening. That is to say, the opening angle required for the battery compartment cover 206 to shield the external power supply interface 3 is determined by the distance between the external power supply interface 3 and the rotation axis of the battery compartment cover 206.

The battery compartment cover 206 may be rotatably arranged at the compartment opening in any suitable way. For example, in some embodiments, as shown in Fig. 42 and Fig. 43, the battery compartment cover 206 may comprise a cover body 231 and a first side wing 232 connected to one side of the cover body 231, wherein the first side wing 232 has two shaft holes 233 arranged opposite to each other, two rotating shafts 133 opposite to each other are arranged at one side of the compartment opening, and the two rotating shafts 133 are rotatably inserted into the two shaft holes 233.

Specifically, as shown in Fig. 42, the battery compartment 208 may be a dual-cavity battery compartment, which can hold two batteries 6; the two rotating shafts 133 are arranged parallel to a line connecting the centers of circles of two circular compartment openings at the right side of the compartment opening; the cover body 231 of the battery compartment cover 206 is oval so that the battery compartment cover 206 can fully cover the two circular compartment openings, and the first side wing 232 is connected to a longer side of the cover body 231.

In the present disclosure, in order to further improve the safety, the power supply mutual exclusion structure may be further configured as follows: the power plug 4 is configured to be capable of blocking the battery compartment cover 206 so that the battery compartment cover 206 can't be opened or can't be opened fully when the power plug 4 is plugged in the external power supply interface 3. Thus, the battery compartment cover 206 can't be fully opened to an extent that the battery interface electrodes are accessible, and there is no safety risk. For example, in some embodiments, when the power plug 4 is plugged into the external power supply interface 3, the power plug 4 can form structural interference with the battery compartment cover 206 to prevent the battery compartment cover 206 from being opened.

The shape of the power plug 4 matches the shape of the power supply interface 3, and the specific shape may be determined according to the design requirement. For example, the power supply interface 3 may be designed as a circular power supply interface as shown in Fig. 42 or a square power supply interface, such as a USB interface.

Specifically, for example, in the embodiment shown in Fig. 42 to Fig. 45, the first side wing 232 of the battery compartment cover 206 comprises an arc-shaped portion 234 positioned between the two shaft holes 233, and the arc-shaped portion 234 is positioned around the periphery of the power plug 4 when the power plug 4 is plugged into the external power supply interface 3. When the power plug 4 is plugged into the external power supply interface 3, the battery compartment cover 206 is structurally locked by the power plug 4 and can't be fully opened to an extent that the battery interface electrodes are accessible, so there is no safety risk.

In the present disclosure, the power supply mutual exclusion structure may further comprise a locking structure for releasably locking the battery compartment cover 206 in its closed position. The locking structure may be any suitable structure, as long as it can prevent the battery compartment cover 206 from being opened accidentally.

Specifically, for example, in the embodiment shown in Fig. 42 and Fig. 43, the battery compartment cover 206 may further comprise a second side wing 235 connected to the opposite side of the cover 231 (i.e., the second side wing 235 and the first side wing 232 are positioned on two opposite sides of the cover 231 respectively), and the locking structure comprises a first snap-fitting clip 162 arranged at the opposite side of the compartment opening (i.e., the first snap-fitting clip 162 and the external power supply interface 3 (and the rotating shafts 133) are positioned at two opposite sides of the battery compartment opening respectively) and a second snap-fitting clip 236 arranged on the second side wing 235 and fitted with the first snap-fitting clip 162.

The first snap-fitting clip 162 and the second snap-fitting clip 236 may have any suitable structural form, as long as they can be snap-fitted with each other or form structural interference with each other (e.g., as shown in Figs 42 and Fig. 43).

In another aspect, the present disclosure provides an electronic product, which comprises the power supply mutual exclusion structure described above. Of course, the electronic product may further comprise a battery 6, a power plug 4 and the like.

The electronic product may be any product that uses two power supply means, namely, an external power source and a battery.

For example, the electronic product may be a nebulizer 7. As shown in Fig. 41, the power supply mutual exclusion structure may be arranged at the bottom of the nebulizer 7. The nebulizer 7 may comprise a housing 71, and the battery compartment 208 is positioned in the housing 71. All of the compartment opening of the battery compartment 208, the battery compartment cover 206, the external power supply interface 3, the rotating shafts 133 and the first snap-fitting clip 162 are arranged on the bottom wall 72 of the housing 71.

Nebulized aerosol inhalation therapy is an important adjuvant therapy, and is the first choice for asthma and chronic obstructive pulmonary diseases. The medicine can be administrated into tracheae and bronchi through mouth or nose to inhibit the inflammatory response of the respiratory tract and relieve clinical symptoms such as bronchospasm, and is of great significance to the improvement of the respiratory tract function of the patient. At present, patients are usually treated by nebulized aerosol inhalation with a nebulization therapeutic device. The nebulization therapeutic device usually employs a dual power supply mode, namely, a battery and a power adapter. In the dual power supply mode, there are problems of poor safety and poor reliability.

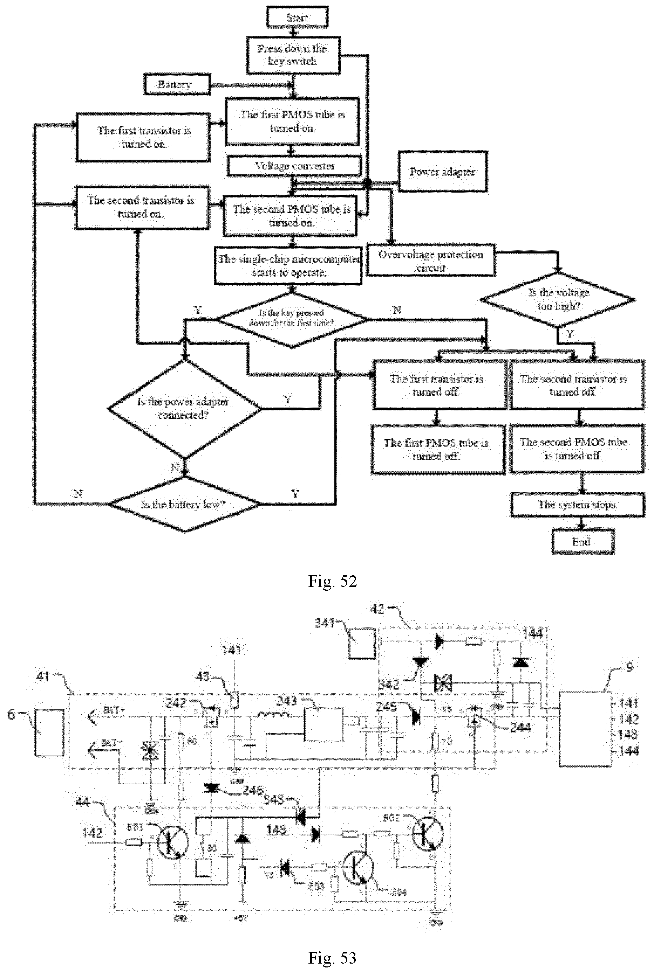

In order to solve the problems of poor safety and poor reliability of a nebulization therapeutic device in the dual power supply mode in the prior art, the present disclosure provides a power management system and a nebulization therapeutic device. The power management system and the nebulization therapeutic device provided in the embodiments of the present disclosure will be described in detail below in specific embodiments and application scenarios with reference to Fig. 51 to Fig. 53.

As shown in Fig. 51 to Fig. 53, an embodiment of the present disclosure provides a power management system, which comprises a single-chip microcomputer 9, a battery circuit 41, a power adapter circuit 42, a voltage detection unit 43 and a control circuit 44, wherein the battery circuit 41 and the power adapter circuit 42 are respectively connected to the single-chip microcomputer 9; the voltage detection unit 43 is respectively connected to the battery circuit 41 and a first input pin 141 of the single-chip microcomputer 9, and the single-chip microcomputer 9 acquires the voltage of the battery 6 through the voltage detection unit 43; the control circuit 44 is respectively connected to the single-chip microcomputer 9, the battery circuit 41 and the power adapter circuit 42; the control circuit 44 is used for controlling the opening and closing of the battery circuit 41 or the power adapter circuit 42 according to a control signal outputted by the single-chip microcomputer 9; the control circuit 44 is used to keep the battery circuit 41 in a persistently closed state when the single-chip microcomputer 9 is powered through the battery circuit 41; the control circuit 44 is used to keep the battery circuit 41 in an open state when the voltage of the battery 6 is lower than a first voltage threshold; and the control circuit 44 is used to keep the battery circuit 41 in the open state when the single-chip microcomputer 9 is powered through the power adapter circuit 42.

Specifically, the single-chip microcomputer (also referred to as a microcontroller unit, MCU) 9 mainly plays a role of controlling the circuit connection in the nebulization therapeutic device. The battery circuit 41 and the power adapter circuit 42 are respectively connected to the single-chip microcomputer 9, the battery 6 supplies power to the single-chip microcomputer 9 through the battery circuit 41, and the power adapter 341 supplies power to the single-chip microcomputer 9 through the power adapter circuit 42. The output voltage of the battery 6 and the output voltage of the power adapter 341 may be selected according to the exact model of the nebulizer.

The single-chip microcomputer 9 has a first input pin 141, which can receive analog signals from peripheral circuits and convert the analog signals into digital signals for the single-chip microcomputer 9 to identify. The voltage detection unit 43 is respectively connected to the battery circuit 41 and a first input pin 141 of the single-chip microcomputer 9, and the single-chip microcomputer 9 acquires the voltage of the battery 6 through the voltage detection unit 43.

The control circuit 44 is respectively connected to the single-chip microcomputer 9, the battery circuit 41 and the power adapter circuit 42; specifically, the control circuit 44 may be connected to a signal output pin of the single-chip microcomputer 9, and the opening or closing of the battery circuit 41 or the power adapter circuit 42 is controlled according to control signals outputted by the single-chip microcomputer 9, thereby the switching between the battery circuit 41 and the power adapter circuit 42 is realized.

The nebulization therapeutic device is provided with a key switch, and the nebulization therapeutic device can be turned on or off by pressing down the key switch for a certain time. When the nebulization therapeutic device is in an OFF state, and the key switch is pressed down for a certain time, the battery circuit 41 or the power adapter circuit 42 will be turned on, and the single-chip microcomputer 9 will be powered on. In the case that the single-chip microcomputer 9 is powered only through the battery circuit 41, the single-chip microcomputer 9 outputs a control signal to the control circuit 44, to keep the battery circuit 41 in a persistent closed state via the control circuit 44. Thus, even if the key switch is released, the nebulization therapeutic apparatus can still be in the normal operation state. When the nebulization therapeutic device is in an ON state, the single-chip microcomputer 9 may also be instructed to output a control signal to the control circuit 44 by pressing the key switch for a certain time, to switch off the battery circuit 41 and the power adapter circuit 42 via the control circuit 44, so that the shutdown function of the nebulization therapeutic device is realized.

When the single-chip microcomputer 9 is powered through the battery circuit 41, the single-chip microcomputer 9 acquires the voltage of the battery 6 through the voltage detection unit 43. In the case that the voltage of the battery 6 is lower than a first voltage threshold, the single-chip microcomputer 9 outputs a control signal to the control circuit 44 to switch off the battery circuit 41 via the control circuit 44, so as to prevent the battery 6 from being damaged due to over-discharge. The first voltage threshold may be the normal voltage when the battery 6 supplies power, or may be lower than the normal voltage when the battery 6 supplies power.

In the case that the single-chip microcomputer 9 is powered through the power adapter circuit 42, the single-chip microcomputer 9 outputs a control signal, so as to switch off the battery circuit 41 through the control circuit 44, thereby the battery 6 stops supplying power, thus the battery 6 is cut off automatically once the power adapter 341 is connected, and the battery 6 and the power adapter 341 will not interfere with each other, thus the safety is improved, and power waste of the battery 6 is effectively avoided.

Optionally, as shown in Fig. 51 to Fig. 53, the power management system further comprises an overvoltage protection circuit; wherein the overvoltage protection circuit is respectively connected to the power adapter circuit 42 and the control circuit 44; and the overvoltage protection circuit and the control circuit 44 jointly control the power adapter circuit 42 to be in an open state when the voltage of the power adapter circuit 42 exceeds a second voltage threshold.

Specifically, when the single-chip microcomputer 9 is powered through the power adapter circuit 42, in order to avoid damages to the components caused by an excessive output voltage of the power adapter 341 or an unexpected power adapter 341 connected to the device, an overvoltage protection circuit is further provided. The overvoltage protection circuit is respectively connected to the power adapter circuit 42 and the control circuit 44. When the voltage of the power adapter circuit 42 exceeds a second voltage threshold, the overvoltage protection circuit and the control circuit 44 can jointly control to switch off the power adapter circuit 42, thus the safety of use of the nebulization therapeutic device is improved.

Optionally, as shown in Fig. 51 to Fig. 53, the battery circuit comprises: a battery, a first controllable switch, a voltage converter and a second controllable switch, wherein the battery is connected to the single-chip microcomputer via the first controllable switch, the voltage converter and the second controllable switch; the single-chip microcomputer comprises a first output pin and a second output pin; the control circuit comprises a third controllable switch and a fourth controllable switch, wherein the third controllable switch is respectively connected to the first controllable switch and the first output pin, and the fourth controllable switch is respectively connected to the second controllable switch and the second output pin; the third controllable switch is used for controlling the opening and closing of the first controllable switch according to a control signal outputted via the first output pin; and the fourth controllable switch is used for controlling the opening and closing of the second controllable switch according to a control signal outputted via the second output pin.

Specifically, the battery circuit 41 comprises a battery 6, a first controllable switch, a voltage converter 243, and a second controllable switch. There is no particular restriction on the model and type of the battery 6, for example, the battery 5 may be an AA alkaline dry battery, an AAA alkaline dry battery, or a lithium battery, etc. The voltage converter 243 can convert the voltage from the battery 6 into a voltage required by the single-chip microcomputer 9. There is not particular restriction on the package, model, output accuracy, etc. of the voltage converter 243. For example, the voltage converter 243 may be of model PW5100-50, with an input voltage range of 0.95 V - 5 V, an output voltage of 5 V, and accuracy of ±2.5%.

The control circuit comprises a third controllable switch and a fourth controllable switch, and the first controllable switch, the second controllable switch, the third controllable switch and the fourth controllable switch may be one or a combination of controllable devices such as PMOS tubes, NMOS tubes and transistors, as long as they can be used to realize circuit opening and closing under the control of the single-chip microcomputer. There is not particular restriction on them in the embodiments of the present disclosure.

When the single-chip microcomputer 9 is powered only by the battery 6, the first controllable switch and the second controllable switch are turned on when the key switch is pressed, and the voltage of the battery 6 is boosted by the voltage converter 243 and then is supplied to the single-chip microcomputer 9; the single-chip microcomputer 9 operates and outputs a control signal to the third controllable switch via the first output pin 142, and outputs a control signal to the fourth controllable switch via the second output pin 143, so that the third controllable switch and the fourth controllable switch are turned on, thereby the first controllable switch and the second controllable switch are in an ON state persistently, and electric power is supplied to the system stably. During the operation, the voltage of the battery 6 is fed to the first input pin 141 of the single-chip microcomputer 9 through the voltage detection unit 43, and the single-chip microcomputer 9 detects the voltage of the battery 6 in real time by means of a program; when the voltage of the battery 6 is lower than the first voltage threshold, the single-chip microcomputer 9 outputs a control signal to the third controllable switch via the first output pin 142, and outputs a control signal to the fourth controllable switch via the second output pin 143, so that the third controllable switch and the fourth controllable switch are turned off, thereby the first controllable switch and the second controllable switch are turned off, and the system stops, so as to avoid damages to the battery 6 and the product caused by over-discharge of the battery 6.

The above-mentioned control signals may be high-level, low-level or other types of signals, and may be selected specifically according to the types of the controllable switches and ON/OFF conditions. There is no particular restriction on them in the embodiments of the present disclosure.

Optionally, as shown in Fig. 51 to Fig. 53, the power adapter circuit comprises a power adapter and the second controllable switch, wherein the power adapter is connected to the single-chip microcomputer via the second controllable switch.

Specifically, the power adapter circuit 42 and the battery circuit 41 share a second controllable switch. There is no particular restriction on the type, interface type, specification and model of the power adapter 341. For example, the power adapter 341 may be an AC-DC power adapter 341, with a rated input voltage of 100 Vac - 240 Vac, a rated input frequency of 50 Hz - 60 Hz, and an output voltage of 5 V ± 0.25 V. The power adapter 341 is connected to the single-chip microcomputer 9 via the second controllable switch. When the power adapter 341 is connected, the first controllable switch and the second controllable switch are turned on the key switch is pressed; the single-chip microcomputer 9 detects that the power adapter 341 is connected, then outputs a control signal to the third controllable switch via the first output pin 142, and outputs a control signal to the fourth controllable switch via the second output pin 143, so that the third controllable switch is turned off and the fourth controllable switch is turned on. Thus, the second controllable switch is turned on, thereby the system is stably powered through the power adapter 341. Since the third controllable switch is turned off and the first controllable switch is turned off accordingly, the battery 6 stops supplying power, thus, the power supply from the battery 6 is automatically cut off once the power adapter 341 is connected, and power waste of the battery 6 is effectively avoided.

Alternatively, the switching between the battery 6 and the power adapter 341 may be implemented by means of a mechanical structure, for example, a DC003A-1.3 power supply interface. The above scheme of the present disclosure can also be realized by replacing the second controllable switch with a self-locking mechanical key and omitting the fourth controllable switch.

Optionally, as shown in Fig. 51 to Fig. 53, the first controllable switch is a first PMOS tube 242, and the second controllable switch is a second PMOS tube 244, wherein the battery 6 is connected to the single-chip microcomputer 9 via a source electrode S and a drain electrode D of the first PMOS tube 242, the voltage converter 243, and a source electrode S and a drain electrode D of the second PMOS tube 244; a base electrode B of the first transistor 501 is electrically connected to the first output pin 142 , a collector electrode C of the first transistor 501 is electrically connected to a grid electrode G of the first PMOS tube 242 and the battery 6, and an emitter electrode E of the first transistor 501 is grounded; a base electrode B of the second transistor 502 is electrically connected to the second output pin 143, a collector electrode C of the second transistor 502 is electrically connected to a grid electrode G of the second PMOS tube 244 and the power adapter 341, and an emitter electrode E of the second transistor 502 is grounded; the first transistor 501 is used for controlling the opening and closing of the first PMOS tube 242 according to a control signal outputted via the first output pin 142; the second transistor 502 is used for controlling the opening and closing of the second PMOS tube 244 according to a control signal outputted via the second output pin 143; and the power adapter 341 is connected to the single-chip microcomputer 9 via the source electrode and the drain electrode of the second PMOS tube 244.

Specifically, the first PMOS tube 242 and the second PMOS tube 244 function as controllable switches in the battery circuit 41, and can control the opening or closing of the battery circuit 41. There is not particular restriction on the package, model, and parameters (e.g., withstand voltage, gate-on threshold, etc.) of the first PMOS transistor 242 and the second PMOS transistor 244. For example, they may be of a model YJL3401A or AO3401A, with V_{DS}max = -30 V, V_{GS}max = ±12 V, and V_{GS(th)} = -0.9 V.

The control circuit 44 comprises a first transistor 501 and a second transistor 502. There is no particular restriction on the package, model and parameters (e.g., withstand voltage) of the first transistor 501 and the second transistor 502. For example, they may be of model S8050, with V_{CBO}max = 40 V, V_{CEO}max = 25 V, V_{EBO}max = 5 V and I_{C} = 500 mA.

The battery 6 is connected to the single-chip microcomputer 9 via the source electrode S and the drain electrode D of the first PMOS tube 242, the voltage converter 243, and the source electrode S and the drain electrode D of the second PMOS tube 244. The single-chip microcomputer 9 comprises a first output pin 142 and a second output pin 143. The base electrode B of the first transistor 501 is electrically connected to the first output pin 142, the collector electrode C of the first transistor 501 is electrically connected to the grid electrode G of the first PMOS tube 242 and the battery 6, and the emitter electrode E of the first transistor 501 is grounded. The base electrode B of the second transistor 502 is electrically connected to the second output pin 143, the collector electrode C of the second transistor 502 is electrically connected to the grid electrode G of the second PMOS tube 244 and the power adapter 341, and the emitter electrode E of the second transistor 502 is grounded.

When the single-chip microcomputer 9 is powered only by the battery 6, the first PMOS tube 242 and the second PMOS tube 244 are turned on when the key switch is pressed, and the voltage of the battery 6 is boosted by the voltage converter 243 and then is supplied to the single-chip microcomputer 9; the single-chip microcomputer 9 operates and outputs a high-level signal to the first transistor 501 via the first output pin 142, and outputs a high-level signal to the second transistor 502 via the second output pin 143, so that the first transistor 501 and the second transistor 502 are turned on, thereby the first PMOS tube 242 and the second PMOS tube 244 are in an ON state persistently, and electric power is supplied to the system stably. During the operation, the voltage of the battery 6 is fed to the first input pin 141 of the single-chip microcomputer 9 through the voltage detection unit 43, and the single-chip microcomputer 9 detects the voltage of the battery 6 in real time by means of a program; when the voltage of the battery 6 is lower than the first voltage threshold, the single-chip microcomputer 9 outputs a low-level signal to the first transistor 501 via the first output pin 142, and outputs a low-level signal to the second transistor 502 via the second output pin 143, so that the first transistor 501 and the second transistor 502 are turned off, thereby the first PMOS tube 242 and the second PMOS tube 244 are turned off, and the system stops, so as to avoid damages to the battery 6 and the product caused by over-discharge of the battery 6.

The power adapter 341 is connected to the single-chip microcomputer 9 via the source electrode S and the drain electrode D of the second PMOS tube 244. When the power adapter 341 is connected, the first PMOS tube 242 and the second PMOS tube 244 are turned on the key switch is pressed; the single-chip microcomputer 9 detects that the power adapter 341 is connected, and then outputs a low-level signal to the first transistor 501 via the first output pin 142 and outputs a high-level signal to the second transistor 502 via the second output pin 143, so that the first transistor 501 is turned off and the second transistor 502 is turned on. Thus, the second PMOS tube 244 is turned on, thereby the system is stably powered through the power adapter 341. Since the first transistor is turned off and the first PMOS tube 242 is turned off accordingly, the battery 6 stops supplying power, thus, the power supply from the battery 6 is automatically cut off once the power adapter 341 is connected, and power waste of the battery 6 is effectively avoided.

Optionally, as shown in Fig. 51 to Fig. 53, the power adapter circuit 42 further comprises a connection detection circuit; the single-chip microcomputer 9 further comprises a second input pin 144, and the connection detection circuit is electrically connected to the second input pin 144.

Specifically, the voltage outputted by the power adapter 341 is fed to the second input pin 144 of the single-chip microcomputer 9 after being divided by a resistor, so that the single-chip microcomputer 9 can timely recognize that the power adapter 341 is connected, and then output a low-level signal to the first transistor 501 via the first output pin 142 and output a high-level signal to the second transistor 502 via the second output pin 143, thereby the first transistor 501 is turned off and the second transistor 502 is turned on. Thus, the second PMOS tube 244 is turned on, thereby the system is stably powered through the power adapter 341.

Optionally, as shown in Fig. 51 to Fig. 53, the overvoltage protection circuit comprises a first diode and a fifth controllable switch; a cathode of the first diode is electrically connected to an output terminal of the power adapter; the fifth controllable switch is electrically connected to an anode of the first diode and the second output pin respectively; and when the output voltage of the power adapter exceeds a second voltage threshold, the first diode is broken down, and the fifth controllable switch and the fourth controllable switch jointly control the second controllable switch to be in an OFF state.

Specifically, the first diode 503 is a voltage-regulator diode, and there is no particular restriction on the package, model and parameters (e.g., accuracy) on the first diode 503. For example, first diode 503 may be of model BZT52C6V2, with V_{Ztyp} = 6.2 V, V_{Zmax} = 6.6 V, and V_{Zmin} = 5.8V. The fifth controllable switch may be one or a combination of controllable devices such as PMOS tubes, NMOS tubes and transistors, as long as it can be used to turn on or off the circuit under the control of the single-chip microcomputer. There is no particular restriction on it in the embodiments of the present disclosure.

If an unexpected power adapter 341 is connected or the output voltage of the power adapter 341 is too high, the first diode 503 is broken down, consequently the fifth controllable switch is turned on, and the fourth controllable switch is turned off, thereby the second controllable switch is turned off, and the power supply to the system is cut off, thus, the back-end circuits are protected and the safety of the system is improved.

Optionally, as shown in Fig. 51 to Fig. 53, the fifth controllable switch is a third transistor 504; an anode of the first diode 503 is electrically connected to a base electrode B of the third transistor 504, a collector electrode C of the third transistor 504 is electrically connected to the second output pin 143, and an emitter electrode E of the third transistor 504 is grounded; and when the output voltage of the power adapter 341 exceeds the second voltage threshold, the first diode 503 is broken down, and the third transistor 504 and the second transistor 502 jointly control the second PMOS tube 244 to be in an OFF state.

Specifically, if an unexpected power adapter 341 is connected or the output voltage of the power adapter 341 is too high, the first diode 503 is broken down, consequently the third transistor 504 is turned on, and the second transistor 502 is turned off, thereby the second PMOS tube 244 is turned off, and the power supply to the system is cut off, thus, the back-end circuits are protected and the safety of the system is improved.

Optionally, as shown in Fig. 51 to Fig. 53, the power management system further comprises a second diode 245 and a third diode 342, wherein an anode of the second diode 245 is electrically connected to the output terminal of the voltage converter 243, and an cathode of the second diode 245 is electrically connected to the second PMOS tube 244; and an anode of the third diode 342 is electrically connected to the output terminal of the power adapter 341, and a cathode of the third diode 342 is electrically connected to the second PMOS 244 tube.

Specifically, the second diode 245 and the third diode 342 can isolate reverse electric power, so as to avoid the mutual influence between the battery 6 and the power regulator, and improve the stability and safety of the system.

Optionally, as shown in Fig. 51 to Fig. 53, the power management system further comprises a key switch circuit; wherein the key switch circuit comprises a first resistor 60, a second resistor 70, a fourth diode 246, a fifth diode 343 and a mechanical switch 80; one end of the first resistor 60 is electrically connected to the battery 6 and the source electrode S of the first PMOS tube 242, the other end of the first resistor 60 is electrically connected to an anode of the fourth diode 246 and the grid electrode G of the first PMOS tube 242, and a cathode of the fourth diode 246 is connected to the mechanical switch 80; one end of the second resistor 70 is electrically connected to the source electrode S of the second PMOS tube 244, the other end of the second resistor 70 is electrically connected to an anode electrode of the fifth diode 343 and the grid electrode G of the second PMOS tube 244, and a cathode of the fifth diode 343 is connected to the mechanical switch 80; and the key switch circuit is used for controlling the on/off of the power management system.

Specifically, when the nebulization therapeutic device is used, the nebulization therapeutic device can be turned on or off by pressing down the mechanical switch 80 for a certain time. When the nebulization therapeutic device is in an OFF state, the mechanical switch 80 can be pressed down for a certain time, so that the first PMOS tube 242 and the second PMOS tube 244 are turned on, and the single-chip microcomputer 9 is powered on. When the single-chip microcomputer 9 is powered only by the battery 6, the single-chip microcomputer 9 operates and outputs a high-level signal to the first transistor 501 via the first output pin 142 and outputs a high-level signal to the second transistor 502 via the second output pin 143, so that the first transistor 501 and the second transistor 502 are turned on, and then the first PMOS tube 242 and the second PMOS tube 244 are in an ON state persistently, and electric power is supplied to the system stably.

When the nebulization therapeutic device is in the ON state, the single-chip microcomputer 9 can also output a low-level signal to the first transistor 501 via the first output pin 142 and output a low-level signal to the second transistor 502 via the second output pin 143 by pressing down the mechanical switch 80 for a certain time, so that the first transistor 501 and the second transistor 502 are turned off, then the first PMOS tube 242 and the second PMOS tube 244 are turned off, and the system stops.

In addition, the user can also click and double-click the mechanical switch 80 to adjust the power and temperature of the nebulization therapeutic device, etc. In a standby mode, the first PMOS tube 242 and the second PMOS tube 244 can be turned on and the system can operate only when the mechanical switch 80 is pressed down. Therefore, in the standby mode, the electric power of the battery 6 will not be wasted owing to static power consumption of the components, such as the chips.

The fourth diode 246 and the fifth diode 343 can isolate reverse electric power, so as to avoid the mutual influence between the battery 6 and the power regulator, and improve the stability and safety of the system.

An embodiment of the present disclosure further provides a nebulization therapeutic device, which comprises the power management system described above.

In the embodiment of the present disclosure, the nebulization therapeutic device employs the power management system described above; after the user controls the single-chip microcomputer 9 to turn on with the mechanical key, the control circuit 44 can keep the battery circuit 41 in a persistently closed state when the single-chip microcomputer 9 is powered through the battery circuit 41; when the single-chip microcomputer 9 detects that the voltage of the battery 6 is lower than a first voltage threshold, the single-chip microcomputer 9 outputs a control signal to switch off the battery circuit 41 through the control circuit 44, so as to avoid damage to the nebulization therapeutic device caused by over-discharge of the battery 6 and improve the reliability of the nebulization therapeutic device; in the case that the single-chip microcomputer 9 is powered through the power adapter circuit 42, the single-chip microcomputer 9 outputs a control signal, so as to switch off the battery circuit 41 through the control circuit 44, thereby the battery 6 stops supplying power, thus the battery 6 is cut off automatically once the power adapter 341 is connected, and the battery 6 and the power adapter 341 will not interfere with each other, thus the safety of the nebulization therapeutic device is improved, power waste of the battery 6 is effectively avoided, and the durability of the nebulization therapeutic device is improved.

It may be noted that the terms "comprise", "include" or any other variant thereof as used herein are intended to encompass non-exclusive inclusion, such that a process, method, article or device comprising a plurality of elements includes not only those elements but also other elements not listed explicitly herein, or further includes elements that are inherent to such a process, method, article or device. An element defined by the phrase "comprising a ..." does not exclude the presence of additional identical elements in the process, method, article or device including the element, provided that there is no more confinement. In addition, it may be pointed out that the scope of the method and device in the embodiments of the present disclosure is not limited to performing functions in the order as shown or discussed, but further encompasses performing functions in a substantially simultaneous manner or in a reversed order according to the functions involved. For example, the described method may be performed in an order different from the described order, and various steps may be added, omitted, or combined. Moreover, features described with reference to some examples may be combined in other examples.

While some embodiments of the present disclosure are described above with reference to the accompanying drawings, the present disclosure is not limited to those embodiments. The embodiments described above are only illustrative rather than limiting. Various modifications and alternations may be made by those having ordinary skills in the art inspired by the present disclosure without departing from the spirit of the present disclosure and the scope of protection defined by the claims. However, all of such modifications and alternations shall be deemed as falling in the scope of protection of the present disclosure.

## Claims

1. A method for dismounting a nebulization assembly, comprising:
removing an entire assembly formed by a nebulization assembly and a liquid storage apparatus from a main unit when the nebulization assembly and the liquid storage apparatus are in a first assembly state, wherein the first assembly state is a state in which the nebulization assembly and the liquid storage apparatus are fixed in relation to each other;
controlling the nebulization assembly and the liquid storage apparatus to switch to a second assembly state, wherein the second assembly state is a state in which the nebulization assembly and the liquid storage apparatus are separatable; and
separating the nebulization assembly from the liquid storage apparatus.

2. The method for dismounting a nebulization assembly of claim 1, wherein removing the entire assembly formed by the nebulization assembly and the liquid storage apparatus from the main unit when the nebulization assembly and the liquid storage apparatus are in the first assembly state comprises:
controlling the liquid storage apparatus and the main unit to switch from a locked state to an unlocked state; and
sliding the liquid storage apparatus along a slide channel on the main unit, and separating a first limiting structure on the nebulization assembly from a second limiting structure on the main unit at the same time, so as to remove the entire assembly formed by the nebulization assembly and the liquid storage apparatus from the main unit;
wherein the first limiting structure is snap-fitted with the second limiting structure when the liquid storage apparatus and the main unit are in the locked state.

3. The method for dismounting a nebulization assembly of claim 2, wherein controlling the liquid storage apparatus and the main unit to switch from the locked state to the unlocked state comprises:
switching at least one snap-fitting clip on the main unit and at least one snap-fitting groove in the liquid storage apparatus from a snap-fitted state to a disengaged state; and/or switching at least one snap-fitting groove in the main unit and at least one snap-fitting clip on the liquid storage apparatus from a snap-fitted state to a disengaged state;
wherein the liquid storage apparatus and the main unit are in the locked state when the snap-fitting clips and the snap-fitting grooves are in the snap-fitted state; and the liquid storage apparatus and the main unit are in the unlocked state when the snap-fitting clips and the snap-fitting grooves are in the disengaged state.

4. The method for dismounting a nebulization assembly of claim 2, wherein controlling the liquid storage apparatus and the main unit to switch from the locked state to the unlocked state comprises:
switching a key on the main unit from a first state to a second state;
wherein the liquid storage apparatus and the main unit are in the locked state when the key is in the first state; and the liquid storage apparatus and the main unit are in the unlocked state when the key is in the second state.

5. The method for dismounting a nebulization assembly of claim 1, wherein controlling the nebulization assembly and the liquid storage apparatus to switch to the second assembly state comprises:
rotating a locking element on the liquid storage apparatus to switch the locking element and the liquid storage apparatus from a connected state to a separated state;
wherein the locking element is detachably connected to the liquid storage apparatus by rotation, and the nebulization assembly is positioned between the locking element and the liquid storage apparatus;
the nebulization assembly and the liquid storage apparatus are in the first assembly state when the locking element and the liquid storage apparatus are in the connected state; and the nebulization assembly and the liquid storage apparatus are in the second assembly state when the locking element and the liquid storage apparatus are in the separated state.

6. The method for dismounting a nebulization assembly of claim 1, wherein controlling the nebulization assembly and the liquid storage apparatus to switch to the second assembly state comprises:
controlling the nebulization assembly and a mounting base to rotate from a first relative position to a second relative position;
wherein the liquid storage apparatus is provided with the mounting base, and the nebulization assembly is at least partially embedded in the mounting base and rotatably connected to the mounting base;
wherein the nebulization assembly and the mounting base are fixed in relation to each other when the nebulization assembly and the mounting base are in the first relative position; and the nebulization assembly and the mounting base can be separated from each other when the nebulization assembly and the mounting base are in the second relative position.

7. The method for dismounting a nebulization assembly of claim 6, wherein controlling the nebulization assembly and the mounting base to rotate from the first relative position to the second relative position comprises:
fitting a first snap-fitting part on the nebulization assembly with a second snap-fitting part on the mounting base by snap-fitting, so that the nebulization assembly and the mounting base are in the first relative position; and
causing a snap-fitting function of the first snap-fitting part and the second snap-fitting part to fail when the nebulization assembly and the mounting base are controlled to rotate to the second relative position.

8. The method for dismounting a nebulization assembly of claim 6, wherein controlling the nebulization assembly and the mounting base to rotate from the first relative position to the second relative position comprises:
controlling the nebulization assembly and the mounting base to rotate in relation to each other, so as to drive the first limiting structure to slide from a locking end of a third limiting structure to an unlocking end of the third limiting structure;
wherein the first limiting structure is arranged on the nebulization assembly, the third limiting structure is arranged at the bottom of the mounting base, and the third limiting structure is used for limiting a rotation range of the nebulization assembly and the mounting base;
the nebulization assembly and the mounting base are in the first relative position when the first limiting structure is at the locking end; and the nebulization assembly and the mounting base are in the second relative position when the first limiting structure is at the unlocking end.

9. A method for mounting a nebulization assembly, comprising:
plugging a nebulization assembly into a mounting base of a liquid storage apparatus when the liquid storage apparatus is separated from a main unit, so that the nebulization assembly and the liquid storage apparatus are in a second assembly state, wherein the second assembly state is a state in which the nebulization assembly and the liquid storage apparatus are separatable;
controlling the nebulization assembly and the liquid storage apparatus to switch to a first assembly state, wherein the first assembly state is a state in which the nebulization assembly and the liquid storage apparatus are fixed in relation to each other; and
plugging an entire assembly formed by the nebulization assembly and the liquid storage apparatus into the main unit.

10. A nebulization therapeutic device, comprising: a main unit, a liquid storage apparatus and a nebulization assembly, wherein
the liquid storage apparatus is detachably mounted to the main unit in a plug-in manner;
the nebulization assembly is connected to the liquid storage apparatus;
the liquid storage apparatus and the nebulization assembly are fixed in relation to each other when an entire assembly formed by the liquid storage apparatus and the nebulization assembly is assembled to the main unit; and
the liquid storage apparatus and the nebulization assembly are dismountable when the entire assembly formed by the liquid storage apparatus and the nebulization assembly is separated from the main unit.

11. A nebulization therapeutic device, comprising a nebulization assembly, wherein the nebulization assembly can be dismounted with the method for dismounting a nebulization assembly of any of claims 1-8.
